# EUROPÄISCHE PATENTANMELDUNG

(11) **EP 2 940 144 A1**
(43) Veröffentlichungstag der Anmeldung: **04.11.2015**
(21) Anmeldenummer: 14166666.9
(22) Anmeldetag: 30.04.2014
(51) Int. Cl.: C12P 13/08, C12N 9/10, C12N 15/63

(54) **Verfahren zur Produktion von L-Lysin unter Verwendung eines alkaliphilen Bakteriums**

(71) Anmelder: Evonik Degussa GmbH, 45128 Essen (DE)
(72) Erfinder: Ochrombel, Ines, 33729 Bielefeld (DE); Bathe, Brigitte, 33154 Salzkotten (DE); Hasselmeyer, Marleen, 33649 Bielefeld (DE)

(57) **Zusammenfassung**

Überraschenderweise wurde gefunden, dass durch Selektion von alkaliphilen Bakterien in einem Medium, das S-2-Aminoethyl-L-Cystein und L-Threonin enthält, alkaliphile Bakterien erhalten werden können, die L-Lysin überproduzieren.

SEQUENCE LISTING

<110>  Evonik Industries AG

<120>  Verfahren zur Produktion von L-Lysin unter Verwendung eines
       alkaliphilen Bakteriums

<130>  201400113

<160>  36

<170>  PatentIn version 3.5

<210>  1
<211>  1509
<212>  DNA
<213>  Corynebacterium humireducens


<220>
<221>  CDS
<222>  (301)..(1209)
<223>  gene dapA

<400>  1
ggcaggccgc ccgcgcggtg ctgccgaacg ccacggagtc acgcatcgtc gtcaccggca      60

actaccgggc gtggcgccac ttcatcggcg cccgggccac cgagcacgcg gacgtcgaga     120

tccgggcact ggcggtggcg tgcctccgcc tcctcgcgca gcagtccccg gtgctcttcg     180

ccgatttcca gatctccact ctctccgacg gcacagagat ggccgtcagt ccctacgcca     240

ccgacttctg accagcgagg gattccccag cggaaaggta aacaggtaac cttgaacacc     300

atg agt aca ggt atg act tcg aag acc gga gtc gag gac ttc ggt acc       348
Met Ser Thr Gly Met Thr Ser Lys Thr Gly Val Glu Asp Phe Gly Thr
1               5                   10                  15

gtc tgc gtc gcc atg gtc acg ccc ttc gac cgg gac ggc gcc ctg gac       396
Val Cys Val Ala Met Val Thr Pro Phe Asp Arg Asp Gly Ala Leu Asp
            20                  25                  30

ctc gag gcc ggt cgc cgc atc gcg gcc cac ctc gtc gac aac ggt ctg       444
Leu Glu Ala Gly Arg Arg Ile Ala Ala His Leu Val Asp Asn Gly Leu
        35                  40                  45

gac gcc ctc gtg ctc gca ggc acg acc ggt gag tcc ccg acc acc acg       492
Asp Ala Leu Val Leu Ala Gly Thr Thr Gly Glu Ser Pro Thr Thr Thr
    50                  55                  60

ctc gac gag aag atc agc ctg ctc aag gcg gtg aag gag gag gtc ggc       540
Leu Asp Glu Lys Ile Ser Leu Leu Lys Ala Val Lys Glu Glu Val Gly
65                  70                  75                  80

gac cgc tgc aag ctc atc gcg ggt gcc ggc acc aac gac acc gcc tcc       588
Asp Arg Cys Lys Leu Ile Ala Gly Ala Gly Thr Asn Asp Thr Ala Ser
                85                  90                  95

tcc atc gag ctc gcc cgc gcc tcc gcc gag gcc ggc gcc gac gcc ctg       636
Ser Ile Glu Leu Ala Arg Ala Ser Ala Glu Ala Gly Ala Asp Ala Leu
            100                 105                 110

ctc gtg gtc acc ccc tac tac tcg aag ccg agc cag gag ggt gtc tac 684
Leu Val Val Thr Pro Tyr Tyr Ser Lys Pro Ser Gln Glu Gly Val Tyr
        115                 120                 125

cag cac ttc aag gcc gtc gcc ggg gcc acc gac ctg ccc atc atg ctc 732
Gln His Phe Lys Ala Val Ala Gly Ala Thr Asp Leu Pro Ile Met Leu
    130                 135                 140

tac gac atc ccg ccg cgc tcc tcg atc ccg atc gag ggg gac acc atc 780
Tyr Asp Ile Pro Pro Arg Ser Ser Ile Pro Ile Glu Gly Asp Thr Ile
145                 150                 155                 160

cgc cga ctc gcg gag ctg ccc acc att cgt gcg gtg aag gat gca aag 828
Arg Arg Leu Ala Glu Leu Pro Thr Ile Arg Ala Val Lys Asp Ala Lys
                165                 170                 175

ggt aac atg gct gag tct gca ccg ctc atg cat gag acc ggt ctc gcc 876
Gly Asn Met Ala Glu Ser Ala Pro Leu Met His Glu Thr Gly Leu Ala
            180                 185                 190

tgg tac tcg ggc gat gat ccg ctc aac gtc ccg tgg ctg tcg ctc ggc 924
Trp Tyr Ser Gly Asp Asp Pro Leu Asn Val Pro Trp Leu Ser Leu Gly
        195                 200                 205

gcc tcg gga ttc gtt tcc gtg gtc ggc cat gcc gca ccc cag gca ctg 972
Ala Ser Gly Phe Val Ser Val Val Gly His Ala Ala Pro Gln Ala Leu
    210                 215                 220

cgt gag ctg tac aca agt ttc gag gaa ggc gac ctc gcc cgt gcg cgg 1020
Arg Glu Leu Tyr Thr Ser Phe Glu Glu Gly Asp Leu Ala Arg Ala Arg
225                 230                 235                 240

gaa atc aac gcc act gtt ctt tca ccg ctg atc gcc gct cag ggt cgc 1068
Glu Ile Asn Ala Thr Val Leu Ser Pro Leu Ile Ala Ala Gln Gly Arg
                245                 250                 255

ctg ggt ggt gtc agc atg gcc aag gct gcc ctg cgt ctg cag ggc att 1116
Leu Gly Gly Val Ser Met Ala Lys Ala Ala Leu Arg Leu Gln Gly Ile
            260                 265                 270

gag gtg ggg gac ccg cgt ctg cct gtc gtc gct ccc gac gag cag gag 1164
Glu Val Gly Asp Pro Arg Leu Pro Val Val Ala Pro Asp Glu Gln Glu
        275                 280                 285

atc gag gcc ctc cgc cgt gac atg gaa aag gct gga gtc ctt taa 1209
Ile Glu Ala Leu Arg Arg Asp Met Glu Lys Ala Gly Val Leu
    290                 295                 300

atatgactga atcccgtagt cgtgcccgga aggtcacccg caaggcgggc ccgccggaga 1269

ccgagagcac tggtgcatcc ccggttttcc aggcaccgac cccggaaacg aacggaaacg 1329

gcgcatccgc cgagaagaag accaccgaaa ggaagtccgc ggacaccgac aacgccccgc 1389

ggggcggcgg caaccgctcc cggaacggtg gcggccgcgc ccgcggcggc cgtggtggcc 1449

gcggcggtca gggtggccag ggccagggcg gtcagggtgg tcagcagggg ggacggggac 1509

<210> 2
<211> 302
<212> PRT
<213> Corynebacterium humireducens

<400> 2

Met Ser Thr Gly Met Thr Ser Lys Thr Gly Val Glu Asp Phe Gly Thr
1               5                   10                  15

Val Cys Val Ala Met Val Thr Pro Phe Asp Arg Asp Gly Ala Leu Asp
            20                  25                  30

Leu Glu Ala Gly Arg Arg Ile Ala Ala His Leu Val Asp Asn Gly Leu
        35                  40                  45

Asp Ala Leu Val Leu Ala Gly Thr Thr Gly Glu Ser Pro Thr Thr Thr
    50                  55                  60

Leu Asp Glu Lys Ile Ser Leu Leu Lys Ala Val Lys Glu Glu Val Gly
65                  70                  75                  80

Asp Arg Cys Lys Leu Ile Ala Gly Ala Gly Thr Asn Asp Thr Ala Ser
                85                  90                  95

Ser Ile Glu Leu Ala Arg Ala Ser Ala Glu Ala Gly Ala Asp Ala Leu
            100                 105                 110

Leu Val Val Thr Pro Tyr Tyr Ser Lys Pro Ser Gln Glu Gly Val Tyr
        115                 120                 125

Gln His Phe Lys Ala Val Ala Gly Ala Thr Asp Leu Pro Ile Met Leu
    130                 135                 140

Tyr Asp Ile Pro Pro Arg Ser Ser Ile Pro Ile Glu Gly Asp Thr Ile
145                 150                 155                 160

Arg Arg Leu Ala Glu Leu Pro Thr Ile Arg Ala Val Lys Asp Ala Lys
                165                 170                 175

Gly Asn Met Ala Glu Ser Ala Pro Leu Met His Glu Thr Gly Leu Ala
            180                 185                 190

Trp Tyr Ser Gly Asp Asp Pro Leu Asn Val Pro Trp Leu Ser Leu Gly
        195                 200                 205

Ala Ser Gly Phe Val Ser Val Val Gly His Ala Ala Pro Gln Ala Leu
    210                 215                 220

Arg Glu Leu Tyr Thr Ser Phe Glu Glu Gly Asp Leu Ala Arg Ala Arg
225                 230                 235                 240

Glu Ile Asn Ala Thr Val Leu Ser Pro Leu Ile Ala Ala Gln Gly Arg
                245                 250                 255

Leu Gly Gly Val Ser Met Ala Lys Ala Ala Leu Arg Leu Gln Gly Ile
            260                 265                 270

Glu Val Gly Asp Pro Arg Leu Pro Val Val Ala Pro Asp Glu Gln Glu
        275                 280                 285

Ile Glu Ala Leu Arg Arg Asp Met Glu Lys Ala Gly Val Leu
    290                 295                 300

<210>  3
<211>  1932
<212>  DNA
<213>  Corynebacterium humireducens

<220>
<221>  CDS
<222>  (301)..(1632)
<223>  gene lysA

<400>  3
cggacctctc cctgctcacc cacgagcggg agggcgacct catccgcacc ctcggcgagt      60

tcccgaacgt ggtcaaggcc gcggcggaac tgcgtgagcc gcaccgtatc gcccgttatg     120

ccgaggaact ggccggcgtg ttccaccgct tctacgacaa ctgccagatc ctgccgaagg     180

cggacgaaga ggccgccccg atccacaccg cccgcctggc gctggctgcg gccacccgcc     240

agaccctggc caacgcgctc ggcctggtcg gtgtcaccgc cccggagagg atgtaagcgc     300

atg acc tcg gat ttc aac gat ctg ccg gcc cac gtc tgg ccg cgc aac       348
Met Thr Ser Asp Phe Asn Asp Leu Pro Ala His Val Trp Pro Arg Asn
1               5                   10                  15

gcc gcg cgc cag gag gac ggc gtg gtc acc gtc gcg ggt gtc ccg ctg       396
Ala Ala Arg Gln Glu Asp Gly Val Val Thr Val Ala Gly Val Pro Leu
            20                  25                  30

ccg gag atc gcc gag gag ttc ggt acc ccg gtc ttc gtc gtc gac gag       444
Pro Glu Ile Ala Glu Glu Phe Gly Thr Pro Val Phe Val Val Asp Glu
        35                  40                  45

gac gac ttc cgt tcc cgc tgc cag gac atg gcc cgc gcc ttc ggc ggc       492
Asp Asp Phe Arg Ser Arg Cys Gln Asp Met Ala Arg Ala Phe Gly Gly
    50                  55                  60

ccg gag cac gtc cac tac gcc tcc aag gcg ttc ctc acc cgc acc gtc       540
Pro Glu His Val His Tyr Ala Ser Lys Ala Phe Leu Thr Arg Thr Val
65                  70                  75                  80

gcc cgc tgg gtc gac gag gag ggg ctg tcg ctc gac gtc gcc tcg ctc       588
Ala Arg Trp Val Asp Glu Glu Gly Leu Ser Leu Asp Val Ala Ser Leu
                85                  90                  95

aac gag ctg cgg atc gcg ctg gcc gcc gag ttc ccc gcc gcc cgc atc       636

Asn Glu Leu Arg Ile Ala Leu Ala Ala Glu Phe Pro Ala Ala Arg Ile
            100                 105                 110

acc gcg cac ggc aac aac aag ggc ccg tcc ttc ctg cgg gcg tgc gtc 684
Thr Ala His Gly Asn Asn Lys Gly Pro Ser Phe Leu Arg Ala Cys Val
        115                 120                 125

acc gag ggc gtc ggc cac gtc gtc ctc gac tcc gag cag gag ctc gag 732
Thr Glu Gly Val Gly His Val Val Leu Asp Ser Glu Gln Glu Leu Glu
    130                 135                 140

ctt ctc gac gcc atc gcc gcc ggc gcc ggc gtc atc cag aag gtg atg 780
Leu Leu Asp Ala Ile Ala Ala Gly Ala Gly Val Ile Gln Lys Val Met
145                 150                 155                 160

atc cgc gtc aag ccg ggc atc gag gcg cac acc cac gag gcc atc gcc 828
Ile Arg Val Lys Pro Gly Ile Glu Ala His Thr His Glu Ala Ile Ala
                165                 170                 175

acg tcc cac gag gac cag aag ttc ggc ttc tcc ctg gcc tcc ggc tcg 876
Thr Ser His Glu Asp Gln Lys Phe Gly Phe Ser Leu Ala Ser Gly Ser
            180                 185                 190

gcg ttc cgc gcc gcg gag gcc gcc gtc cgc gcc gag aac ctc gag ctc 924
Ala Phe Arg Ala Ala Glu Ala Ala Val Arg Ala Glu Asn Leu Glu Leu
        195                 200                 205

gtg ggc ctg cac tgc cac gtc ggc tcc cag gtc ttc gac gcc cgg ggc 972
Val Gly Leu His Cys His Val Gly Ser Gln Val Phe Asp Ala Arg Gly
    210                 215                 220

ttc acg atg gcc gcc gac cgc gtg ctg ggc ctc tac tcg cag atc cac 1020
Phe Thr Met Ala Ala Asp Arg Val Leu Gly Leu Tyr Ser Gln Ile His
225                 230                 235                 240

cgc gaa ctc ggc gtg cag ctg ccc gag ctc gat ctc ggc ggc ggc tac 1068
Arg Glu Leu Gly Val Gln Leu Pro Glu Leu Asp Leu Gly Gly Gly Tyr
                245                 250                 255

ggc atc gcc tac acc gag gac gag cag ccc ctc aac gtc gac gag gtc 1116
Gly Ile Ala Tyr Thr Glu Asp Glu Gln Pro Leu Asn Val Asp Glu Val
            260                 265                 270

gcc cac gac ctg ctc acc gcc gtg gcc aag acc gcc gcg gag ctg ggc 1164
Ala His Asp Leu Leu Thr Ala Val Ala Lys Thr Ala Ala Glu Leu Gly
        275                 280                 285

atc gac gcc ccc gcc gtc ctc gtc gag ccg ggc cgc gcc atc gcc ggc 1212
Ile Asp Ala Pro Ala Val Leu Val Glu Pro Gly Arg Ala Ile Ala Gly
    290                 295                 300

ccc ggc acg gtg acg gtc tac gag gtc ggc acc gtc aag gac gtc acc 1260
Pro Gly Thr Val Thr Val Tyr Glu Val Gly Thr Val Lys Asp Val Thr
305                 310                 315                 320

gtc tcc gag gac gag aag cga cgc tac ctc tcc gtc gac ggc ggc atg 1308
Val Ser Glu Asp Glu Lys Arg Arg Tyr Leu Ser Val Asp Gly Gly Met
                325                 330                 335

tcc gac aac atc cgc ccg gcc ctc tac gac gcg ctt tac gac gcc cgc 1356
Ser Asp Asn Ile Arg Pro Ala Leu Tyr Asp Ala Leu Tyr Asp Ala Arg
            340                 345                 350

gtg gtc aac cgt ttc acc gag ggc gag gag atc tcc tcc cgc ctc gtc     1404
Val Val Asn Arg Phe Thr Glu Gly Glu Glu Ile Ser Ser Arg Leu Val
        355                 360                 365

ggt tcc cac tgc gag tcc ggc gac att ctc gtc cgc gac gcg cgc tac     1452
Gly Ser His Cys Glu Ser Gly Asp Ile Leu Val Arg Asp Ala Arg Tyr
    370                 375                 380

ccc gac gac atc gtg gtc ggg gac ctc atg gcc gtc gcc gcg acc ggc     1500
Pro Asp Asp Ile Val Val Gly Asp Leu Met Ala Val Ala Ala Thr Gly
385                 390                 395                 400

gcc tac gcc tac gcc atg tcc tcg cgc tac aac gcc ttc ggc cgt ccg     1548
Ala Tyr Ala Tyr Ala Met Ser Ser Arg Tyr Asn Ala Phe Gly Arg Pro
                405                 410                 415

ccg gtc gtc gcg gtg cgt gcc ggc aag acg aag ctc atg ctg cgc cgc     1596
Pro Val Val Ala Val Arg Ala Gly Lys Thr Lys Leu Met Leu Arg Arg
            420                 425                 430

gag acc gtc gag gac att ctc gcg ctg gag acg tag aaccgacgcc          1642
Glu Thr Val Glu Asp Ile Leu Ala Leu Glu Thr
        435                 440

cgggcagggc gtcagtggaa gaggcccttg aggctgccgg cgtcctccag cagcgccacg   1702

acgagcgagg tgtggatgga accgagcggc acgcccctgg cgggcgtcgg cgcggtcgac   1762

gggtccgggg tggaggaacg ggtcggcgtc gtggacgact cttcgcggtc gacggtgccg   1822

gaggctcccg tggcgcacat gcgcagctcc gcccggtagg cgtccgcctc cgcctgggct   1882

cccggggtgc gctcccaggc ccgcagccag gcctcgtcgg cggccacgga              1932

<210>  4
<211>  443
<212>  PRT
<213>  Corynebacterium humireducens

<400>  4

Met Thr Ser Asp Phe Asn Asp Leu Pro Ala His Val Trp Pro Arg Asn
1               5                   10                  15

Ala Ala Arg Gln Glu Asp Gly Val Val Thr Val Ala Gly Val Pro Leu
            20                  25                  30

Pro Glu Ile Ala Glu Glu Phe Gly Thr Pro Val Phe Val Val Asp Glu
        35                  40                  45

Asp Asp Phe Arg Ser Arg Cys Gln Asp Met Ala Arg Ala Phe Gly Gly
    50                  55                  60

Pro Glu His Val His Tyr Ala Ser Lys Ala Phe Leu Thr Arg Thr Val
65                  70                  75                  80

Ala Arg Trp Val Asp Glu Glu Gly Leu Ser Leu Asp Val Ala Ser Leu

85                  90                  95

Asn Glu Leu Arg Ile Ala Leu Ala Ala Glu Phe Pro Ala Ala Arg Ile
            100                 105                 110

Thr Ala His Gly Asn Asn Lys Gly Pro Ser Phe Leu Arg Ala Cys Val
        115                 120                 125

Thr Glu Gly Val Gly His Val Val Leu Asp Ser Glu Gln Glu Leu Glu
    130                 135                 140

Leu Leu Asp Ala Ile Ala Ala Gly Ala Gly Val Ile Gln Lys Val Met
145                 150                 155                 160

Ile Arg Val Lys Pro Gly Ile Glu Ala His Thr His Glu Ala Ile Ala
                165                 170                 175

Thr Ser His Glu Asp Gln Lys Phe Gly Phe Ser Leu Ala Ser Gly Ser
            180                 185                 190

Ala Phe Arg Ala Ala Glu Ala Ala Val Arg Ala Glu Asn Leu Glu Leu
        195                 200                 205

Val Gly Leu His Cys His Val Gly Ser Gln Val Phe Asp Ala Arg Gly
    210                 215                 220

Phe Thr Met Ala Ala Asp Arg Val Leu Gly Leu Tyr Ser Gln Ile His
225                 230                 235                 240

Arg Glu Leu Gly Val Gln Leu Pro Glu Leu Asp Leu Gly Gly Gly Tyr
                245                 250                 255

Gly Ile Ala Tyr Thr Glu Asp Glu Gln Pro Leu Asn Val Asp Glu Val
            260                 265                 270

Ala His Asp Leu Leu Thr Ala Val Ala Lys Thr Ala Ala Glu Leu Gly
        275                 280                 285

Ile Asp Ala Pro Ala Val Leu Val Glu Pro Gly Arg Ala Ile Ala Gly
    290                 295                 300

Pro Gly Thr Val Thr Val Tyr Glu Val Gly Thr Val Lys Asp Val Thr
305                 310                 315                 320

Val Ser Glu Asp Glu Lys Arg Arg Tyr Leu Ser Val Asp Gly Gly Met
                325                 330                 335

Ser Asp Asn Ile Arg Pro Ala Leu Tyr Asp Ala Leu Tyr Asp Ala Arg
            340                 345                 350

Val Val Asn Arg Phe Thr Glu Gly Glu Glu Ile Ser Ser Arg Leu Val
        355                 360                 365

Gly Ser His Cys Glu Ser Gly Asp Ile Leu Val Arg Asp Ala Arg Tyr
    370                 375                 380

Pro Asp Asp Ile Val Val Gly Asp Leu Met Ala Val Ala Ala Thr Gly
385                 390                 395                 400

Ala Tyr Ala Tyr Ala Met Ser Ser Arg Tyr Asn Ala Phe Gly Arg Pro
                405                 410                 415

Pro Val Val Ala Val Arg Ala Gly Lys Thr Lys Leu Met Leu Arg Arg
            420                 425                 430

Glu Thr Val Glu Asp Ile Leu Ala Leu Glu Thr
        435                 440

<210> 5
<211> 1770
<212> DNA
<213> Corynebacterium humireducens

<220>
<221> CDS
<222> (301)..(1470)
<223> gene aat

<400> 5
cgcctacgcg ctcaccgtcg tcctggccgc cgagaactac ccggccgccc cggtcaccgg 60

tggggtcatc accggtgacg tcgtgaatga cccgtccaag gtcctccacg ccggtaccgc 120

gcgcaacgag gccggcgagc tggtctccgc cggtggccgc gtgctcaaca tcgtgggcgt 180

cggcaagacc ctcgaggagg cgcgtgacga cgcctacgcc accatcgagc agatccagct 240

cgagggttcc ttctaccgcg gcgacatcgc actgccggcc gtggagggac ggatctccct 300

gtg acc ggt cgg cag ctc gtc agc gac cgc ggc cag gtc cag ccc ttc 348
Val Thr Gly Arg Gln Leu Val Ser Asp Arg Gly Gln Val Gln Pro Phe
1               5                   10                  15

cgc gtc atg cag atg ctc gac atc gtg cac cga cgc gcc cgc gag ggg 396
Arg Val Met Gln Met Leu Asp Ile Val His Arg Arg Ala Arg Glu Gly
            20                  25                  30

aag cac acc atc atg ctg tgc gcc ggg cag ccc tcc acg ggt gcc ccg 444
Lys His Thr Ile Met Leu Cys Ala Gly Gln Pro Ser Thr Gly Ala Pro
        35                  40                  45

gcc ccg gtc ctg acc gcg gcg gag gag tcc ctc cac gcc tcg ccc ctc 492

Ala Pro Val Leu Thr Ala Ala Glu Glu Ser Leu His Ala Ser Pro Leu
    50                  55                  60

ggg tac acc gag gtg gtc ggt gac cgc gac ctc cgg gag atc gtc gcg      540
Gly Tyr Thr Glu Val Val Gly Asp Arg Asp Leu Arg Glu Ile Val Ala
65                  70                  75                  80

gcc tgg cac tcc gac acc tac ggc gtg cac acc agc gcg gac aac gtg      588
Ala Trp His Ser Asp Thr Tyr Gly Val His Thr Ser Ala Asp Asn Val
                85                  90                  95

gtg atc acc acc ggc tcc tcc ggt ggc ttc gtc gcg tcc ttc ctg gcc      636
Val Ile Thr Thr Gly Ser Ser Gly Gly Phe Val Ala Ser Phe Leu Ala
            100                 105                 110

gtg ctc gac cac ggc gac gcc gtc gcc ctg tcc tgc ccc ggt tac ccg      684
Val Leu Asp His Gly Asp Ala Val Ala Leu Ser Cys Pro Gly Tyr Pro
        115                 120                 125

gcg tac cgc aac atc ctg gag tcc ctc ggg gcg cgg gtg gtc aac ctc      732
Ala Tyr Arg Asn Ile Leu Glu Ser Leu Gly Ala Arg Val Val Asn Leu
    130                 135                 140

gtg tgc gac gag tcg acc cgc ttc cag ccg acg gcc gcg atg ctc gag      780
Val Cys Asp Glu Ser Thr Arg Phe Gln Pro Thr Ala Ala Met Leu Glu
145                 150                 155                 160

gca ctg ccg gag gct gac agg ccg cgg gcg gtg atc gtc acc tcc ccg      828
Ala Leu Pro Glu Ala Asp Arg Pro Arg Ala Val Ile Val Thr Ser Pro
                165                 170                 175

ggc aac ccc tcc ggc acg acc atc gac ccc gcg gag ctc gag cgc atc      876
Gly Asn Pro Ser Gly Thr Thr Ile Asp Pro Ala Glu Leu Glu Arg Ile
            180                 185                 190

gcc cgc tgg tgc gag gac aac gac gcc gtg ctc atc tcc gac gag gac      924
Ala Arg Trp Cys Glu Asp Asn Asp Ala Val Leu Ile Ser Asp Glu Asp
        195                 200                 205

tac cac ggc atg tcc ttc ggc cgc ccc ctg gcc acc gca cgc cag ttc      972
Tyr His Gly Met Ser Phe Gly Arg Pro Leu Ala Thr Ala Arg Gln Phe
    210                 215                 220

tcc gac cgg gcg atc gtc gtg ggc acg ctg tcg aag tac tac tcc atg     1020
Ser Asp Arg Ala Ile Val Val Gly Thr Leu Ser Lys Tyr Tyr Ser Met
225                 230                 235                 240

acc ggc tgg cgc gtc ggc tgg ctc atc ctc ccc gac gag ctg gtc acc     1068
Thr Gly Trp Arg Val Gly Trp Leu Ile Leu Pro Asp Glu Leu Val Thr
                245                 250                 255

ccc gtg gag aac ctg cag gcc tcg ctg gcg ctc tgc ccg ccg gcg atc     1116
Pro Val Glu Asn Leu Gln Ala Ser Leu Ala Leu Cys Pro Pro Ala Ile
            260                 265                 270

tcg cag gtc gcc ggg cgt gcc gcc ttc acc ccg gag gcg cgc gcc gaa     1164
Ser Gln Val Ala Gly Arg Ala Ala Phe Thr Pro Glu Ala Arg Ala Glu
        275                 280                 285

ctc gac tcc cac gtc gag cgc tac cgc gcc gcc cgc gcg gtg ctg ctc     1212
Leu Asp Ser His Val Glu Arg Tyr Arg Ala Ala Arg Ala Val Leu Leu
    290                 295                 300

gag gag ctg ccg gcg gcc ggt ttc acc acc atg gcg gaa ccc gac ggc     1260
Glu Glu Leu Pro Ala Ala Gly Phe Thr Thr Met Ala Glu Pro Asp Gly
305                 310                 315                 320

ggg ctc tac ctg tgg ctc gac gtc tcc cac gtc acc gac gac tcc gag     1308
Gly Leu Tyr Leu Trp Leu Asp Val Ser His Val Thr Asp Asp Ser Glu
                325                 330                 335

gcc tgg tgc cac gag ctc gtc gag gag atc ggc gtg gcc ttc gcg ccg     1356
Ala Trp Cys His Glu Leu Val Glu Glu Ile Gly Val Ala Phe Ala Pro
            340                 345                 350

ggc gtc gac ttc gac ccg atc aac ggt cac aag tgg gtc cgc ctc acg     1404
Gly Val Asp Phe Asp Pro Ile Asn Gly His Lys Trp Val Arg Leu Thr
        355                 360                 365

ctg tgc gcc ccg gag gag gag acc cgc gag gcg atc cgg cgc ctc ggc     1452
Leu Cys Ala Pro Glu Glu Glu Thr Arg Glu Ala Ile Arg Arg Leu Gly
    370                 375                 380

gag tac gtg cgt cgc tga ccgcgcggcg acctgcggcg cgggggaaac            1500
Glu Tyr Val Arg Arg
385

ggcccggatc cggggactcc cgggccaccc ccgccgctgg tcatgcgata atcgggggcg   1560

tggctgagaa gaagaagatc gcgaacgtcc tgtccaaccg ctacgcctcc gcggagctga   1620

gcaacctgtg gagcccggag tacaagatca tcctggagcg tcagctctgg atcgccgtga   1680

tgaaggcgca gaaggatctg ggtgtcgaca tcccggctga ggccatcgac gcctacgagg   1740

ccgtcatcga ccgggtcgac ctcgagtcga                                    1770


<210>  6
<211>  389
<212>  PRT
<213>  Corynebacterium humireducens

<400>  6

Val Thr Gly Arg Gln Leu Val Ser Asp Arg Gly Gln Val Gln Pro Phe
1               5                   10                  15


Arg Val Met Gln Met Leu Asp Ile Val His Arg Arg Ala Arg Glu Gly
            20                  25                  30


Lys His Thr Ile Met Leu Cys Ala Gly Gln Pro Ser Thr Gly Ala Pro
        35                  40                  45


Ala Pro Val Leu Thr Ala Ala Glu Glu Ser Leu His Ala Ser Pro Leu
    50                  55                  60


Gly Tyr Thr Glu Val Val Gly Asp Arg Asp Leu Arg Glu Ile Val Ala
65                  70                  75                  80


Ala Trp His Ser Asp Thr Tyr Gly Val His Thr Ser Ala Asp Asn Val

                85                  90                  95

Val Ile Thr Thr Gly Ser Ser Gly Gly Phe Val Ala Ser Phe Leu Ala
            100                 105                 110

Val Leu Asp His Gly Asp Ala Val Ala Leu Ser Cys Pro Gly Tyr Pro
        115                 120                 125

Ala Tyr Arg Asn Ile Leu Glu Ser Leu Gly Ala Arg Val Val Asn Leu
    130                 135                 140

Val Cys Asp Glu Ser Thr Arg Phe Gln Pro Thr Ala Ala Met Leu Glu
145                 150                 155                 160

Ala Leu Pro Glu Ala Asp Arg Pro Arg Ala Val Ile Val Thr Ser Pro
                165                 170                 175

Gly Asn Pro Ser Gly Thr Thr Ile Asp Pro Ala Glu Leu Glu Arg Ile
            180                 185                 190

Ala Arg Trp Cys Glu Asp Asn Asp Ala Val Leu Ile Ser Asp Glu Asp
        195                 200                 205

Tyr His Gly Met Ser Phe Gly Arg Pro Leu Ala Thr Ala Arg Gln Phe
    210                 215                 220

Ser Asp Arg Ala Ile Val Val Gly Thr Leu Ser Lys Tyr Tyr Ser Met
225                 230                 235                 240

Thr Gly Trp Arg Val Gly Trp Leu Ile Leu Pro Asp Glu Leu Val Thr
                245                 250                 255

Pro Val Glu Asn Leu Gln Ala Ser Leu Ala Leu Cys Pro Pro Ala Ile
            260                 265                 270

Ser Gln Val Ala Gly Arg Ala Ala Phe Thr Pro Glu Ala Arg Ala Glu
        275                 280                 285

Leu Asp Ser His Val Glu Arg Tyr Arg Ala Ala Arg Ala Val Leu Leu
    290                 295                 300

Glu Glu Leu Pro Ala Ala Gly Phe Thr Thr Met Ala Glu Pro Asp Gly
305                 310                 315                 320

Gly Leu Tyr Leu Trp Leu Asp Val Ser His Val Thr Asp Asp Ser Glu
                325                 330                 335

Ala Trp Cys His Glu Leu Val Glu Glu Ile Gly Val Ala Phe Ala Pro
            340                 345                 350

Gly Val Asp Phe Asp Pro Ile Asn Gly His Lys Trp Val Arg Leu Thr
        355                 360                 365

Leu Cys Ala Pro Glu Glu Glu Thr Arg Glu Ala Ile Arg Arg Leu Gly
    370                 375                 380

Glu Tyr Val Arg Arg
385

<210>  7
<211>  1281
<212>  DNA
<213>  Corynebacterium humireducens

<220>
<221>  CDS
<222>  (301)..(981)
<223>  gene lysE

<400>  7
   gaaggcgatc acctggatgc cgtagcccac cgccgccggg tcgaggcgcg tgtggaagcc      60
   ggtgatcacg cccccgttct ccagcgcccg cagccgccgc aggcaggtgc ttgacgacgc      120
   ccccgtatga tgcgccagtt ctgcggcggt gatgcgtgca ttgttctgga tcagtgacag      180
   aatcatgcgg tcaatgtcgt ccatacgccg gattctgtca cacgtgcagt cccggcgtcc      240
   cccggtgaat gaaaagttca ggagtatcgg ccgcggcgcc gggcgggcac gataccgggc      300
   atg gac atc acg gac ctc ctg agc ttc tgg gcg gtc agc ctc ctg ctg      348
   Met Asp Ile Thr Asp Leu Leu Ser Phe Trp Ala Val Ser Leu Leu Leu
   1               5                   10                  15
   atc gtc acc ccg ggc ccc gac tgg gcg ttc gtg ctc ggg cac gct ttc      396
   Ile Val Thr Pro Gly Pro Asp Trp Ala Phe Val Leu Gly His Ala Phe
               20                  25                  30
   cga cgc cgc ccc ctc gcc ccg ccc ctt ctc ggc atc ggc ctg ggc tac      444
   Arg Arg Arg Pro Leu Ala Pro Pro Leu Leu Gly Ile Gly Leu Gly Tyr
           35                  40                  45
   ctc ggc ctg acc acc gtg gtg gcc ggc ggc ctc ggg gcg ctc gtg gcc      492
   Leu Gly Leu Thr Thr Val Val Ala Gly Gly Leu Gly Ala Leu Val Ala
       50                  55                  60
   cgt cac ccc gcg ctg ctc acg ctc gtc acc gtc gcc ggt gtc ctg gtg      540
   Arg His Pro Ala Leu Leu Thr Leu Val Thr Val Ala Gly Val Leu Val
   65                  70                  75                  80
   ctc ctg cgt ctc ggc tgg cag atg atg cgc gcg gcg ctg gcc ggg ccc      588
   Leu Leu Arg Leu Gly Trp Gln Met Met Arg Ala Ala Leu Ala Gly Pro
                   85                  90                  95
   gtc acg gtg gag gtg gac acc gcg gac ccg gct gtc ccg gag ggg ccg      636
   Val Thr Val Glu Val Asp Thr Ala Asp Pro Ala Val Pro Glu Gly Pro
               100                 105                 110
   gcc ggc ggg gga gtg gcg acg ctg acg cgc ccg cag gtg ggc gtc gag      684
   Ala Gly Gly Gly Val Ala Thr Leu Thr Arg Pro Gln Val Gly Val Glu
           115                 120                 125
   aag cac gcg gtg gtc acc ggg gcc gcg gtg agt ggc ctc aac ccc aag      732
   Lys His Ala Val Val Thr Gly Ala Ala Val Ser Gly Leu Asn Pro Lys
       130                 135                 140
   ggg ctc atg ctg ttc atc gcg ctg ctg ccc cag ttc gtg ggc gcg gag      780
   Gly Leu Met Leu Phe Ile Ala Leu Leu Pro Gln Phe Val Gly Ala Glu
   145                 150                 155                 160
   gcg acg tgg ccg gtg tgg acg cag atg ttc gtc ctc gga ctg gtg ttc      828
   Ala Thr Trp Pro Val Trp Thr Gln Met Phe Val Leu Gly Leu Val Phe
                   165                 170                 175
   atc gcc tcg gcg acc gtg ttc tac gcg ctg ctc ggc gtt ttc gcc ggg      876
   Ile Ala Ser Ala Thr Val Phe Tyr Ala Leu Leu Gly Val Phe Ala Gly
               180                 185                 190
   cgg gtg ctg gcg ggc tcg gag cgg gcc tcc cgg atc ctc acc ggc gtg      924
   Arg Val Leu Ala Gly Ser Glu Arg Ala Ser Arg Ile Leu Thr Gly Val
           195                 200                 205
   gcc ggc ggg gcg atg atg tgt gtc gcg gca ggg atg ctc gcg cag cac      972
   Ala Gly Gly Ala Met Met Cys Val Ala Ala Gly Met Leu Ala Gln His
       210                 215                 220
   ctc ctc tga tgggggtagt tgagtagggg cattcatcgg gtgcgagttc              1021
   Leu Leu
   225
   cgcgcctccc gatgaatgcc cctgctcgga taccctccta ctccacgtgc gccggcgtcg    1081
   acggcgacac cagcggcatc ggcgcgtact cggcacgcag ctcgcgcagc ttccgggcgt    1141
   gctcggagag gcggacgtcc tcggtggtac gggggacgaa ctggcgggcg ggcagggcgt    1201
   tgccgtcggc gtccgtcgcc aggtaggtga cggtggcgtg gatggcggtc tgcaggttct    1261
   cgcggccccc gcgcgggttg                                                1281

<210>  8
<211>  226
<212>  PRT
<213>  Corynebacterium humireducens

<400>  8

Met Asp Ile Thr Asp Leu Leu Ser Phe Trp Ala Val Ser Leu Leu Leu
1               5                   10                  15

Ile Val Thr Pro Gly Pro Asp Trp Ala Phe Val Leu Gly His Ala Phe
            20                  25                  30

Arg Arg Arg Pro Leu Ala Pro Pro Leu Leu Gly Ile Gly Leu Gly Tyr
        35                  40                  45

Leu Gly Leu Thr Thr Val Val Ala Gly Gly Leu Gly Ala Leu Val Ala
    50                  55                  60

Arg His Pro Ala Leu Leu Thr Leu Val Thr Val Ala Gly Val Leu Val
65                  70                  75                  80

Leu Leu Arg Leu Gly Trp Gln Met Met Arg Ala Ala Leu Ala Gly Pro
                85                  90                  95

Val Thr Val Glu Val Asp Thr Ala Asp Pro Ala Val Pro Glu Gly Pro
            100                 105                 110

Ala Gly Gly Gly Val Ala Thr Leu Thr Arg Pro Gln Val Gly Val Glu
        115                 120                 125

Lys His Ala Val Val Thr Gly Ala Ala Val Ser Gly Leu Asn Pro Lys
    130                 135                 140

Gly Leu Met Leu Phe Ile Ala Leu Leu Pro Gln Phe Val Gly Ala Glu
145                 150                 155                 160

Ala Thr Trp Pro Val Trp Thr Gln Met Phe Val Leu Gly Leu Val Phe
                165                 170                 175

Ile Ala Ser Ala Thr Val Phe Tyr Ala Leu Leu Gly Val Phe Ala Gly
            180                 185                 190

Arg Val Leu Ala Gly Ser Glu Arg Ala Ser Arg Ile Leu Thr Gly Val
        195                 200                 205

Ala Gly Gly Ala Met Met Cys Val Ala Ala Gly Met Leu Ala Gln His
    210                 215                 220

Leu Leu
225

<210>  9
<211>  4014
<212>  DNA
<213>  Corynebacterium humireducens

<220>
<221>  CDS
<222>  (301)..(3714)
<223>  gene pyc

<400>  9
cggcaccgga ccaccgcgac gcccacggca ctgcccacac attggggtgg tgcgcgtggg        60

tttcgctgtt cggaacacct gtcggcccat tccgcagggc gggacagaaa cggcctttcg 120

ggcgcccgtc ccgcggagtg cggaaatgga ccgcaccggg tccaccacct gcagataccc 180

cctgtgacta ggggatactc ccccgaagac aaccctcttt cttgcgatac tgtggtgtgc 240

cagagagctc acccgctctc agacacaccc ccgacatgac gttcggaaag gaaccccacc 300

gtg gcc aca acc act ctg cca gcc ttc aca aag gtg ctg gtc gcc aac 348
Val Ala Thr Thr Thr Leu Pro Ala Phe Thr Lys Val Leu Val Ala Asn
1               5                   10                  15

cgc ggc gag atc gcc gtc cgc gcc ttc cgt gcc gcc ttc gag acc ggt 396
Arg Gly Glu Ile Ala Val Arg Ala Phe Arg Ala Ala Phe Glu Thr Gly
            20                  25                  30

gcc tcc acc gtc gcg gtc tac ccg aag gag gac cgc aac tcg cac cac 444
Ala Ser Thr Val Ala Val Tyr Pro Lys Glu Asp Arg Asn Ser His His
        35                  40                  45

cgc tcc ttc gcc tcc gag gcc gtg cgc atc ggc acc gag gga tcg ccg 492
Arg Ser Phe Ala Ser Glu Ala Val Arg Ile Gly Thr Glu Gly Ser Pro
    50                  55                  60

gtc aag gcg tac ctc gac atc gac gag atc atc cgc gcc gcc aag aag 540
Val Lys Ala Tyr Leu Asp Ile Asp Glu Ile Ile Arg Ala Ala Lys Lys
65                  70                  75                  80

acc cac gcc gac gcc gtc tac ccc ggc tac ggt ttc ctc tcc gag aac 588
Thr His Ala Asp Ala Val Tyr Pro Gly Tyr Gly Phe Leu Ser Glu Asn
                85                  90                  95

gcc cag ctc gcc cgc gag tgc gcc gag aac ggc ctc acc ttc atc ggc 636
Ala Gln Leu Ala Arg Glu Cys Ala Glu Asn Gly Leu Thr Phe Ile Gly
            100                 105                 110

ccg ccg ccg gag gtc ctc gac ctg acc ggc gac aag tcc gcc gcc gtc 684
Pro Pro Pro Glu Val Leu Asp Leu Thr Gly Asp Lys Ser Ala Ala Val
        115                 120                 125

gag gcc gcc cgg aag gcc gga ctc ccg gtc ctg cag gac tcc gag ccg 732
Glu Ala Ala Arg Lys Ala Gly Leu Pro Val Leu Gln Asp Ser Glu Pro
    130                 135                 140

tcc acc gac atc gac gag ctc gtc gag atg tcg aag gac ttc acc ttc 780
Ser Thr Asp Ile Asp Glu Leu Val Glu Met Ser Lys Asp Phe Thr Phe
145                 150                 155                 160

ccg atc ttc gtc aag gcc gtc gcc ggt ggc ggt gga cgt ggc atg cgc 828
Pro Ile Phe Val Lys Ala Val Ala Gly Gly Gly Gly Arg Gly Met Arg
                165                 170                 175

ttc atc gag aag ccc gag gac gtc gcc cag ctg gcc acc gag gcc tcc 876
Phe Ile Glu Lys Pro Glu Asp Val Ala Gln Leu Ala Thr Glu Ala Ser
            180                 185                 190

cgc gag gcg gag gcc gcc ttc ggt gac ggt cat gtc tac ctc gag cgt 924
Arg Glu Ala Glu Ala Ala Phe Gly Asp Gly His Val Tyr Leu Glu Arg
        195                 200                 205

gcg gtg ctc aac ccg cag cac atc gag gtc cag atc ctc gcc gac cac 972
Ala Val Leu Asn Pro Gln His Ile Glu Val Gln Ile Leu Ala Asp His

210                 215                 220

acc ggc gac gtc atc cac ctc tac gag cgt gac tgc tcc ctg cag cgc      1020
Thr Gly Asp Val Ile His Leu Tyr Glu Arg Asp Cys Ser Leu Gln Arg
225                 230                 235                 240

cgc cac cag aag gtc gtg gag atc gcc ccg gcc cag cac ctc gac cca      1068
Arg His Gln Lys Val Val Glu Ile Ala Pro Ala Gln His Leu Asp Pro
                245                 250                 255

gcc ctg cgc gac cag atc tgc gcc gac gcc gtg aag ttc tgc cgc tcc      1116
Ala Leu Arg Asp Gln Ile Cys Ala Asp Ala Val Lys Phe Cys Arg Ser
            260                 265                 270

atc ggc tac gtg tgc gcc ggc acc gtc gag ttc ctc gtc gac gag aac      1164
Ile Gly Tyr Val Cys Ala Gly Thr Val Glu Phe Leu Val Asp Glu Asn
        275                 280                 285

ggc aac cac gtc ttc atc gag atg aac ccg cgc atc cag gtc gag cac      1212
Gly Asn His Val Phe Ile Glu Met Asn Pro Arg Ile Gln Val Glu His
    290                 295                 300

acc atc acc gag gag gtc acc tcc gtc gac ctg gtc aag gcg cag atg      1260
Thr Ile Thr Glu Glu Val Thr Ser Val Asp Leu Val Lys Ala Gln Met
305                 310                 315                 320

aac atc gcc gcc ggc gcg acc ctc aag gac ctc ggc ctc acg cag gag      1308
Asn Ile Ala Ala Gly Ala Thr Leu Lys Asp Leu Gly Leu Thr Gln Glu
                325                 330                 335

tcc atc aag ctc cac ggc gcg gcc ctg cag tgc cgc atc acc acc gag      1356
Ser Ile Lys Leu His Gly Ala Ala Leu Gln Cys Arg Ile Thr Thr Glu
            340                 345                 350

gac ccg gcc aac aac ttc cgc ccg gac acc ggc acc atc acc gcg tac      1404
Asp Pro Ala Asn Asn Phe Arg Pro Asp Thr Gly Thr Ile Thr Ala Tyr
        355                 360                 365

cgc tcc ccg ggt ggc gcg ggc gtg cgt ctc gac ggc gcc gcg tcc ctc      1452
Arg Ser Pro Gly Gly Ala Gly Val Arg Leu Asp Gly Ala Ala Ser Leu
    370                 375                 380

ggc ggc gag atc tcc ccg aac ttc gac tcc ctg ctg gtc aag atg acc      1500
Gly Gly Glu Ile Ser Pro Asn Phe Asp Ser Leu Leu Val Lys Met Thr
385                 390                 395                 400

tgc cgc ggc gcc gac ttc gcc acc gcc gtc gcc cgt gcc cag cgc gcg      1548
Cys Arg Gly Ala Asp Phe Ala Thr Ala Val Ala Arg Ala Gln Arg Ala
                405                 410                 415

ctc aac gag ttc acc atc tcg ggc gtg gcc acc aac atc ggc ttc ctc      1596
Leu Asn Glu Phe Thr Ile Ser Gly Val Ala Thr Asn Ile Gly Phe Leu
            420                 425                 430

cgc gcc ctg ctg cgc gag gag gac ttc cag acc cgc cgc atc tcc acc      1644
Arg Ala Leu Leu Arg Glu Glu Asp Phe Gln Thr Arg Arg Ile Ser Thr
        435                 440                 445

aac ttc atc ggc gag cac ctc cac ctg ctg tcc gcc ccg ccg gcc gac      1692
Asn Phe Ile Gly Glu His Leu His Leu Leu Ser Ala Pro Pro Ala Asp
    450                 455                 460

gac gag gcc ggc cgc atc ctc aac tac ctc gcg gac gtc acc gtc aac      1740

Asp Glu Ala Gly Arg Ile Leu Asn Tyr Leu Ala Asp Val Thr Val Asn
465                 470                 475                 480

cgt ccg cac ggc aag cgc ccc acc gag atc cgt ccg gtg acc aag ctg       1788
Arg Pro His Gly Lys Arg Pro Thr Glu Ile Arg Pro Val Thr Lys Leu
                485                 490                 495

ccg gcc cgc gac gac agc ccg ctg ccg cgc ggt tcc cgc gac gtc ctg       1836
Pro Ala Arg Asp Asp Ser Pro Leu Pro Arg Gly Ser Arg Asp Val Leu
            500                 505                 510

cgt gac ctg ggc ccg cag aag ttc gcc gag cag ctg cgc aac cag gac       1884
Arg Asp Leu Gly Pro Gln Lys Phe Ala Glu Gln Leu Arg Asn Gln Asp
        515                 520                 525

gcc ctg gcc gtc acc gac acc acg ttc cgt gac gcg cac cag tcg ctg       1932
Ala Leu Ala Val Thr Asp Thr Thr Phe Arg Asp Ala His Gln Ser Leu
    530                 535                 540

ctg gcc acc cgc atc cgt tcc tcc gcg ctc gtc gcc gcc gcc gag cac       1980
Leu Ala Thr Arg Ile Arg Ser Ser Ala Leu Val Ala Ala Ala Glu His
545                 550                 555                 560

gtc ggc cgc ctg acc ccg gag ctg ctc tcc gtc gag gcg tgg ggc ggt       2028
Val Gly Arg Leu Thr Pro Glu Leu Leu Ser Val Glu Ala Trp Gly Gly
                565                 570                 575

gcg acc tac gac gtc gcg atg cgt ttc ctc cac gag gac ccg tgg gag       2076
Ala Thr Tyr Asp Val Ala Met Arg Phe Leu His Glu Asp Pro Trp Glu
            580                 585                 590

cgt ctc gac gac ctg cgc cgc gcc atg ccg aac gtc aac atc cag atg       2124
Arg Leu Asp Asp Leu Arg Arg Ala Met Pro Asn Val Asn Ile Gln Met
        595                 600                 605

ctg ctg cgc ggc cgc aac acc gtc ggc tac acc tcc tac ccg gac agc       2172
Leu Leu Arg Gly Arg Asn Thr Val Gly Tyr Thr Ser Tyr Pro Asp Ser
    610                 615                 620

gtc ggc cag gcc ttc gtc aag gag gcc gcc gac tcc ggc gtc gac atc       2220
Val Gly Gln Ala Phe Val Lys Glu Ala Ala Asp Ser Gly Val Asp Ile
625                 630                 635                 640

ttc cgc atc ttc gac gcc ctc aac gac gtg gac cag atg cgc ccg gcc       2268
Phe Arg Ile Phe Asp Ala Leu Asn Asp Val Asp Gln Met Arg Pro Ala
                645                 650                 655

atc gat gcc gtc ctg gag acc ggc acc acc gtc gcc gag gtc gcc atg       2316
Ile Asp Ala Val Leu Glu Thr Gly Thr Thr Val Ala Glu Val Ala Met
            660                 665                 670

gcc tac tcc ggc aac ctg ctg gac ccg aac gag aag ctc tac acg ctc       2364
Ala Tyr Ser Gly Asn Leu Leu Asp Pro Asn Glu Lys Leu Tyr Thr Leu
        675                 680                 685

gac tac tac ctc cgt ctg gcc gag gag atc gtc gag tcc ggt gcc cac       2412
Asp Tyr Tyr Leu Arg Leu Ala Glu Glu Ile Val Glu Ser Gly Ala His
    690                 695                 700

atc ctg gcc atc aag gac atg gcc ggt ctg ctc cgc ccg acc gcg gcc       2460
Ile Leu Ala Ile Lys Asp Met Ala Gly Leu Leu Arg Pro Thr Ala Ala
705                 710                 715                 720

tcc cgc ctg gtg aag gcg ctg cgc agc aac ttc gac ctg ccg gtg cac 2508
Ser Arg Leu Val Lys Ala Leu Arg Ser Asn Phe Asp Leu Pro Val His
                725                 730                 735

gtg cac acc cac gac acc gcc ggc ggt cag ctg gcg acc tac ctg gcc 2556
Val His Thr His Asp Thr Ala Gly Gly Gln Leu Ala Thr Tyr Leu Ala
            740                 745                 750

gcc gcg cag gcg ggt gcc gac gcc gtc gac ggc gcc tcc gcc ccg ctg 2604
Ala Ala Gln Ala Gly Ala Asp Ala Val Asp Gly Ala Ser Ala Pro Leu
        755                 760                 765

gcc ggc acg acc tcg cag ccc tcg ctg tcc gcg atc gtc gcg gcc ttc 2652
Ala Gly Thr Thr Ser Gln Pro Ser Leu Ser Ala Ile Val Ala Ala Phe
    770                 775                 780

gcc gac acc cac cgc gac acc ggc ctg tcc ctg cag gcc gtg tcc gac 2700
Ala Asp Thr His Arg Asp Thr Gly Leu Ser Leu Gln Ala Val Ser Asp
785                 790                 795                 800

ctc gag ccc tac tgg gag gcc gtg cgc cag ctc tac gcc ccc ttc gag 2748
Leu Glu Pro Tyr Trp Glu Ala Val Arg Gln Leu Tyr Ala Pro Phe Glu
                805                 810                 815

gcg ggc gtg ccc ggc ccg acg ggc cgc gtg tac aag cac gag atc ccg 2796
Ala Gly Val Pro Gly Pro Thr Gly Arg Val Tyr Lys His Glu Ile Pro
            820                 825                 830

ggt ggt cag ctg tcg aac ctg cgc acg cag gcc aag gcc ctg ggt ctg 2844
Gly Gly Gln Leu Ser Asn Leu Arg Thr Gln Ala Lys Ala Leu Gly Leu
        835                 840                 845

gag gac cgc ttc gag ctc atc gag gac tac tac gcc gcc gtc aac gag 2892
Glu Asp Arg Phe Glu Leu Ile Glu Asp Tyr Tyr Ala Ala Val Asn Glu
    850                 855                 860

atg ctg ggc cgc ccc acc aag gtc acc ccg tcc tcc aag gtc gtc ggc 2940
Met Leu Gly Arg Pro Thr Lys Val Thr Pro Ser Ser Lys Val Val Gly
865                 870                 875                 880

gac ctg gcg ctc cac ctc gtc ggc gcg ggc gtc gac ccg cac gac ttc 2988
Asp Leu Ala Leu His Leu Val Gly Ala Gly Val Asp Pro His Asp Phe
                885                 890                 895

gcc aat gac ccg cag aag tac gac atc ccg gac tcc gtg atc gac ttc 3036
Ala Asn Asp Pro Gln Lys Tyr Asp Ile Pro Asp Ser Val Ile Asp Phe
            900                 905                 910

ctc cgc ggt gac ctc ggc aac ccg ccc ggt ggc tgg ccg atg ctg cgg 3084
Leu Arg Gly Asp Leu Gly Asn Pro Pro Gly Gly Trp Pro Met Leu Arg
        915                 920                 925

gag aag gcc ctg gca ggt cgt tcc tcc acg cag tcc aag ctt gtc gac 3132
Glu Lys Ala Leu Ala Gly Arg Ser Ser Thr Gln Ser Lys Leu Val Asp
    930                 935                 940

gtc gcc ccg gag gac gag gag gcc ctg cag agc gcg gac cgc cag acc 3180
Val Ala Pro Glu Asp Glu Glu Ala Leu Gln Ser Ala Asp Arg Gln Thr
945                 950                 955                 960

cgc cgc tcg acc ctg gac agg ctg ctg ttc ccg aag cag gcc gcc gag 3228
Arg Arg Ser Thr Leu Asp Arg Leu Leu Phe Pro Lys Gln Ala Ala Glu
                965                 970                 975

ttc gcg gag cac cgc cgc cag tac ggc aac acc gcc gcc ctc gag gac 3276
Phe Ala Glu His Arg Arg Gln Tyr Gly Asn Thr Ala Ala Leu Glu Asp
            980                 985                 990

cgc gtg ttc ttc tac ggc ctc aag gag ggc gag gag aac gtc atc cgc 3324
Arg Val Phe Phe Tyr Gly Leu Lys Glu Gly Glu Glu Asn Val Ile Arg
        995                 1000                1005

ctc atc ggg gag cac ggt cag ccg ccg atg gtc gtg cgt ctc gac 3369
Leu Ile Gly Glu His Gly Gln Pro Pro Met Val Val Arg Leu Asp
    1010                1015                1020

gcc gtc ggt gag ccc gac gag aag ggc atg cgc cag gtg gtg acc 3414
Ala Val Gly Glu Pro Asp Glu Lys Gly Met Arg Gln Val Val Thr
    1025                1030                1035

aac gtc aac ggc cag atc cgt ccg atg aag gtc cgt gac cgc tcc 3459
Asn Val Asn Gly Gln Ile Arg Pro Met Lys Val Arg Asp Arg Ser
    1040                1045                1050

gtc gag tcg gtc acc gcc tcc gcg gag aag gcc gac acc acc aac 3504
Val Glu Ser Val Thr Ala Ser Ala Glu Lys Ala Asp Thr Thr Asn
    1055                1060                1065

ccg ggc cac gtc gcc gcg ccg ttc gcg ggt gtc gtc atg gtc acc 3549
Pro Gly His Val Ala Ala Pro Phe Ala Gly Val Val Met Val Thr
    1070                1075                1080

gcg aag gtg ggc gac gag gtc aag gcc ggc gac ccg gta gcc gtg 3594
Ala Lys Val Gly Asp Glu Val Lys Ala Gly Asp Pro Val Ala Val
    1085                1090                1095

atc gag gcc atg aag atg gag gcc acc atc acc gcc tcc aag gac 3639
Ile Glu Ala Met Lys Met Glu Ala Thr Ile Thr Ala Ser Lys Asp
    1100                1105                1110

ggc gtg gtc gac cgc gtc gcc ctg gcc cag gcc acg aag gtg gag 3684
Gly Val Val Asp Arg Val Ala Leu Ala Gln Ala Thr Lys Val Glu
    1115                1120                1125

ggc ggc gac ctc atc gtc gtc atc gcc tag ccgcacaacg cgaaagcccc 3734
Gly Gly Asp Leu Ile Val Val Ile Ala
    1130                1135

gccactcctt ccgggagtgg cggggcttct ctgtgcgggt gggccgctac ttgatctcga 3794

gcaggacctg gcccttggtc acgccggcac cggcctcgac ggacaggccc tcgaccttgc 3854

ccgacttgtg ggccttgacc gggttctcca tcttcatggc ctcgaggacc acgacgacgt 3914

cgccctcggc gacctcctgg ccgtcctcga cgttgacctt gatgacggtg ccctgcatcg 3974

gggccgccac agcgtcaccg gaggcacctg cggcaccgcc 4014

<210> 10
<211> 1137
<212> PRT
<213> Corynebacterium humireducens

<400> 10

Val Ala Thr Thr Thr Leu Pro Ala Phe Thr Lys Val Leu Val Ala Asn
1               5                   10                  15

Arg Gly Glu Ile Ala Val Arg Ala Phe Arg Ala Ala Phe Glu Thr Gly
            20                  25                  30

Ala Ser Thr Val Ala Val Tyr Pro Lys Glu Asp Arg Asn Ser His His
        35                  40                  45

Arg Ser Phe Ala Ser Glu Ala Val Arg Ile Gly Thr Glu Gly Ser Pro
    50                  55                  60

Val Lys Ala Tyr Leu Asp Ile Asp Glu Ile Ile Arg Ala Ala Lys Lys
65                  70                  75                  80

Thr His Ala Asp Ala Val Tyr Pro Gly Tyr Gly Phe Leu Ser Glu Asn
                85                  90                  95

Ala Gln Leu Ala Arg Glu Cys Ala Glu Asn Gly Leu Thr Phe Ile Gly
            100                 105                 110

Pro Pro Pro Glu Val Leu Asp Leu Thr Gly Asp Lys Ser Ala Ala Val
        115                 120                 125

Glu Ala Ala Arg Lys Ala Gly Leu Pro Val Leu Gln Asp Ser Glu Pro
    130                 135                 140

Ser Thr Asp Ile Asp Glu Leu Val Glu Met Ser Lys Asp Phe Thr Phe
145                 150                 155                 160

Pro Ile Phe Val Lys Ala Val Ala Gly Gly Gly Gly Arg Gly Met Arg
                165                 170                 175

Phe Ile Glu Lys Pro Glu Asp Val Ala Gln Leu Ala Thr Glu Ala Ser
            180                 185                 190

Arg Glu Ala Glu Ala Ala Phe Gly Asp Gly His Val Tyr Leu Glu Arg
        195                 200                 205

Ala Val Leu Asn Pro Gln His Ile Glu Val Gln Ile Leu Ala Asp His
    210                 215                 220

Thr Gly Asp Val Ile His Leu Tyr Glu Arg Asp Cys Ser Leu Gln Arg
225                 230                 235                 240

Arg His Gln Lys Val Val Glu Ile Ala Pro Ala Gln His Leu Asp Pro
                245                 250                 255

Ala Leu Arg Asp Gln Ile Cys Ala Asp Ala Val Lys Phe Cys Arg Ser
            260                 265                 270

Ile Gly Tyr Val Cys Ala Gly Thr Val Glu Phe Leu Val Asp Glu Asn
        275                 280                 285

Gly Asn His Val Phe Ile Glu Met Asn Pro Arg Ile Gln Val Glu His
    290                 295                 300

Thr Ile Thr Glu Glu Val Thr Ser Val Asp Leu Val Lys Ala Gln Met
305                 310                 315                 320

Asn Ile Ala Ala Gly Ala Thr Leu Lys Asp Leu Gly Leu Thr Gln Glu
                325                 330                 335

Ser Ile Lys Leu His Gly Ala Ala Leu Gln Cys Arg Ile Thr Thr Glu
            340                 345                 350

Asp Pro Ala Asn Asn Phe Arg Pro Asp Thr Gly Thr Ile Thr Ala Tyr
        355                 360                 365

Arg Ser Pro Gly Gly Ala Gly Val Arg Leu Asp Gly Ala Ala Ser Leu
    370                 375                 380

Gly Gly Glu Ile Ser Pro Asn Phe Asp Ser Leu Leu Val Lys Met Thr
385                 390                 395                 400

Cys Arg Gly Ala Asp Phe Ala Thr Ala Val Ala Arg Ala Gln Arg Ala
                405                 410                 415

Leu Asn Glu Phe Thr Ile Ser Gly Val Ala Thr Asn Ile Gly Phe Leu
            420                 425                 430

Arg Ala Leu Leu Arg Glu Glu Asp Phe Gln Thr Arg Arg Ile Ser Thr
        435                 440                 445

Asn Phe Ile Gly Glu His Leu His Leu Leu Ser Ala Pro Pro Ala Asp
    450                 455                 460

Asp Glu Ala Gly Arg Ile Leu Asn Tyr Leu Ala Asp Val Thr Val Asn
465                 470                 475                 480

Arg Pro His Gly Lys Arg Pro Thr Glu Ile Arg Pro Val Thr Lys Leu
                485                 490                 495

Pro Ala Arg Asp Asp Ser Pro Leu Pro Arg Gly Ser Arg Asp Val Leu
            500                 505                 510

Arg Asp Leu Gly Pro Gln Lys Phe Ala Glu Gln Leu Arg Asn Gln Asp
        515                 520                 525

Ala Leu Ala Val Thr Asp Thr Thr Phe Arg Asp Ala His Gln Ser Leu
    530                 535                 540

Leu Ala Thr Arg Ile Arg Ser Ser Ala Leu Val Ala Ala Ala Glu His
545                 550                 555                 560

Val Gly Arg Leu Thr Pro Glu Leu Leu Ser Val Glu Ala Trp Gly Gly
                565                 570                 575

Ala Thr Tyr Asp Val Ala Met Arg Phe Leu His Glu Asp Pro Trp Glu
            580                 585                 590

Arg Leu Asp Asp Leu Arg Arg Ala Met Pro Asn Val Asn Ile Gln Met
        595                 600                 605

Leu Leu Arg Gly Arg Asn Thr Val Gly Tyr Thr Ser Tyr Pro Asp Ser
    610                 615                 620

Val Gly Gln Ala Phe Val Lys Glu Ala Ala Asp Ser Gly Val Asp Ile
625                 630                 635                 640

Phe Arg Ile Phe Asp Ala Leu Asn Asp Val Asp Gln Met Arg Pro Ala
                645                 650                 655

Ile Asp Ala Val Leu Glu Thr Gly Thr Thr Val Ala Glu Val Ala Met
            660                 665                 670

Ala Tyr Ser Gly Asn Leu Leu Asp Pro Asn Glu Lys Leu Tyr Thr Leu
        675                 680                 685

Asp Tyr Tyr Leu Arg Leu Ala Glu Glu Ile Val Glu Ser Gly Ala His
    690                 695                 700

Ile Leu Ala Ile Lys Asp Met Ala Gly Leu Leu Arg Pro Thr Ala Ala
705                 710                 715                 720

Ser Arg Leu Val Lys Ala Leu Arg Ser Asn Phe Asp Leu Pro Val His
                725                 730                 735

Val His Thr His Asp Thr Ala Gly Gly Gln Leu Ala Thr Tyr Leu Ala
            740                 745                 750

Ala Ala Gln Ala Gly Ala Asp Ala Val Asp Gly Ala Ser Ala Pro Leu

755                 760                 765

Ala Gly Thr Thr Ser Gln Pro Ser Leu Ser Ala Ile Val Ala Ala Phe
    770                 775                 780

Ala Asp Thr His Arg Asp Thr Gly Leu Ser Leu Gln Ala Val Ser Asp
785                 790                 795                 800

Leu Glu Pro Tyr Trp Glu Ala Val Arg Gln Leu Tyr Ala Pro Phe Glu
                805                 810                 815

Ala Gly Val Pro Gly Pro Thr Gly Arg Val Tyr Lys His Glu Ile Pro
            820                 825                 830

Gly Gly Gln Leu Ser Asn Leu Arg Thr Gln Ala Lys Ala Leu Gly Leu
        835                 840                 845

Glu Asp Arg Phe Glu Leu Ile Glu Asp Tyr Tyr Ala Ala Val Asn Glu
    850                 855                 860

Met Leu Gly Arg Pro Thr Lys Val Thr Pro Ser Ser Lys Val Val Gly
865                 870                 875                 880

Asp Leu Ala Leu His Leu Val Gly Ala Gly Val Asp Pro His Asp Phe
                885                 890                 895

Ala Asn Asp Pro Gln Lys Tyr Asp Ile Pro Asp Ser Val Ile Asp Phe
            900                 905                 910

Leu Arg Gly Asp Leu Gly Asn Pro Pro Gly Gly Trp Pro Met Leu Arg
        915                 920                 925

Glu Lys Ala Leu Ala Gly Arg Ser Ser Thr Gln Ser Lys Leu Val Asp
    930                 935                 940

Val Ala Pro Glu Asp Glu Glu Ala Leu Gln Ser Ala Asp Arg Gln Thr
945                 950                 955                 960

Arg Arg Ser Thr Leu Asp Arg Leu Leu Phe Pro Lys Gln Ala Ala Glu
                965                 970                 975

Phe Ala Glu His Arg Arg Gln Tyr Gly Asn Thr Ala Ala Leu Glu Asp
            980                 985                 990

Arg Val Phe Phe Tyr Gly Leu Lys  Glu Gly Glu Glu Asn  Val Ile Arg
        995                 1000                 1005

Leu Ile Gly Glu His Gly Gln Pro Pro Met Val Val Arg Leu Asp
    1010                1015                1020

Ala Val Gly Glu Pro Asp Glu Lys Gly Met Arg Gln Val Val Thr
    1025                1030                1035

Asn Val Asn Gly Gln Ile Arg Pro Met Lys Val Arg Asp Arg Ser
    1040                1045                1050

Val Glu Ser Val Thr Ala Ser Ala Glu Lys Ala Asp Thr Thr Asn
    1055                1060                1065

Pro Gly His Val Ala Ala Pro Phe Ala Gly Val Val Met Val Thr
    1070                1075                1080

Ala Lys Val Gly Asp Glu Val Lys Ala Gly Asp Pro Val Ala Val
    1085                1090                1095

Ile Glu Ala Met Lys Met Glu Ala Thr Ile Thr Ala Ser Lys Asp
    1100                1105                1110

Gly Val Val Asp Arg Val Ala Leu Ala Gln Ala Thr Lys Val Glu
    1115                1120                1125

Gly Gly Asp Leu Ile Val Val Ile Ala
    1130                1135

<210>  11
<211>  1866
<212>  DNA
<213>  Corynebacterium humireducens

<220>
<221>  CDS
<222>  (301)..(1566)
<223>  gene lysC

<400>  11
gccggtgccc gcccaccaca ggggacggct gatcgcgttc cagaaggggg aacccttgag      60

ggagccgtcg gcgggggcct cctccgcgat gcggtggcgc accacggttc cccacgcgat     120

ggtgagggcg gaggccagtg cgaacagcac ggccagcaga ttgctgtgca cctcatccac     180

cttagtgaac gggggccttt ccgcagggcc gggacccgcc cactcgacta ctcggacgac     240

cccggctccg ggtacgatga agcagaactt tggcacccca gacgaaaggc ggcctacacg     300

gtg gct ctg atc gtt cag aaa tat gga ggc tcc tcc ctg gag acc gcg      348
Val Ala Leu Ile Val Gln Lys Tyr Gly Gly Ser Ser Leu Glu Thr Ala
1               5                   10                  15

gag cgg atc cgc gct gtc gcc gaa cgg atc gtc gcc acg aaa aag gcc      396

Glu Arg Ile Arg Ala Val Ala Glu Arg Ile Val Ala Thr Lys Lys Ala
            20                  25                  30

ggc aac gac gtc gtc gtg gtc tgc tcc gcg atg ggc gac acc acg gac      444
Gly Asn Asp Val Val Val Val Cys Ser Ala Met Gly Asp Thr Thr Asp
        35                  40                  45

gaa ctc ctg gac ctc gcc gcc cag gtg aac ccg gtt ccg ccg gcc cgt      492
Glu Leu Leu Asp Leu Ala Ala Gln Val Asn Pro Val Pro Pro Ala Arg
    50                  55                  60

gag atg gac atg ctg ctc acc gcg ggt gag cgc atc tcc aac gcg ctg      540
Glu Met Asp Met Leu Leu Thr Ala Gly Glu Arg Ile Ser Asn Ala Leu
65                  70                  75                  80

gtc gcg atg gcg atc tcc tcg ctc ggc gcg aag gcg cag tcc ttc acc      588
Val Ala Met Ala Ile Ser Ser Leu Gly Ala Lys Ala Gln Ser Phe Thr
                85                  90                  95

ggc tcg cag gcc ggt gtg ctc acc acc gag cgt cac ggc aac gcc cgc      636
Gly Ser Gln Ala Gly Val Leu Thr Thr Glu Arg His Gly Asn Ala Arg
            100                 105                 110

atc gtc gac gtc acc ccg gac cgc gtc cgc gag gcg ctg gac gag ggc      684
Ile Val Asp Val Thr Pro Asp Arg Val Arg Glu Ala Leu Asp Glu Gly
        115                 120                 125

aac atc tgc atc gtc gcc ggt ttc cag ggc atc aac aag gac acc cgc      732
Asn Ile Cys Ile Val Ala Gly Phe Gln Gly Ile Asn Lys Asp Thr Arg
    130                 135                 140

gac gtc acc acc ctg ggt cgt ggc ggt tcc gac acc aca gcg gtg gcc      780
Asp Val Thr Thr Leu Gly Arg Gly Gly Ser Asp Thr Thr Ala Val Ala
145                 150                 155                 160

ctg gcg gcg gcg ctc aac gcc gac gtg tgc gag atc tac tcc gac gtc      828
Leu Ala Ala Ala Leu Asn Ala Asp Val Cys Glu Ile Tyr Ser Asp Val
                165                 170                 175

gac ggc gtc tac acc gcc gat ccg cgc atc gtc ccg aac gcg cag aag      876
Asp Gly Val Tyr Thr Ala Asp Pro Arg Ile Val Pro Asn Ala Gln Lys
            180                 185                 190

ctc gag aag ctc tcc ttc gag gag atg ctc gag ctg gcg gcc gtc ggt      924
Leu Glu Lys Leu Ser Phe Glu Glu Met Leu Glu Leu Ala Ala Val Gly
        195                 200                 205

tcg aag atc ctg gtg ctg cgt gcc gtc gag tac gca cgt gcc ttc aat      972
Ser Lys Ile Leu Val Leu Arg Ala Val Glu Tyr Ala Arg Ala Phe Asn
    210                 215                 220

gtt ccc ctg cga gtt cgc tcg tct tac agc aat gac acc ggc acc ctg     1020
Val Pro Leu Arg Val Arg Ser Ser Tyr Ser Asn Asp Thr Gly Thr Leu
225                 230                 235                 240

atc gcc ggt tcg atg gag gat atc ccc gtg gaa gaa gca gtt ctc act     1068
Ile Ala Gly Ser Met Glu Asp Ile Pro Val Glu Glu Ala Val Leu Thr
                245                 250                 255

ggc gtt gcc acc gac aag tcc gag gcc aag gtc acc atc ctg ggt att     1116
Gly Val Ala Thr Asp Lys Ser Glu Ala Lys Val Thr Ile Leu Gly Ile
            260                 265                 270

ccc gac aag ccg ggc gag gcg gcc acc gtc ttc cgc gcc gtc gcg gac 1164
Pro Asp Lys Pro Gly Glu Ala Ala Thr Val Phe Arg Ala Val Ala Asp
        275                 280                 285

gcg gag atc aac atc gac atg gtc ctg cag aac gtc tcc tcc ctg gag 1212
Ala Glu Ile Asn Ile Asp Met Val Leu Gln Asn Val Ser Ser Leu Glu
    290                 295                 300

gac ggc acg acc gac atc acc ttc acc tgc ccg cgt gcc gac ggc ccc 1260
Asp Gly Thr Thr Asp Ile Thr Phe Thr Cys Pro Arg Ala Asp Gly Pro
305                 310                 315                 320

cgc gcc atc gag ctg ctc aag aag atg cag gcg aag agc ggc tgg tcc 1308
Arg Ala Ile Glu Leu Leu Lys Lys Met Gln Ala Lys Ser Gly Trp Ser
                325                 330                 335

aac gtg ctc tac gac gac cag gtg ggc aag gtc tcc ctc gtg ggt gcc 1356
Asn Val Leu Tyr Asp Asp Gln Val Gly Lys Val Ser Leu Val Gly Ala
            340                 345                 350

ggc atg aag tcc cac ccg ggc gtc acc gcc gac ttc gcc gag gcg ctc 1404
Gly Met Lys Ser His Pro Gly Val Thr Ala Asp Phe Ala Glu Ala Leu
        355                 360                 365

cgc gac gcc ggc gtg aac atc gag cta atc tcc acc tcg gag atc cgc 1452
Arg Asp Ala Gly Val Asn Ile Glu Leu Ile Ser Thr Ser Glu Ile Arg
    370                 375                 380

atc tcg gtg ctc acc cgc gag gcc gac ctg gac gcc gcc gcc cgt gcc 1500
Ile Ser Val Leu Thr Arg Glu Ala Asp Leu Asp Ala Ala Ala Arg Ala
385                 390                 395                 400

ctg cac gag aag ttc gag ctc ggc ggc gac gcc gag gcc acc gtc tac 1548
Leu His Glu Lys Phe Glu Leu Gly Gly Asp Ala Glu Ala Thr Val Tyr
                405                 410                 415

gcc ggc acc ggc cgc tag acaatccctt ccacagagga gctttcactc 1596
Ala Gly Thr Gly Arg
            420

tcatgaccac cattgctgtc gtcggtgcca ccggccaggt cggccgcgtg atgcgctcga 1656

tcctggagga gcgcaacttc ccggccgaca aggtccgttt cttcgcgtcc ccgcgctccg 1716

cgggcaccgt catcgagttc cgcggtgagg acatcaccgt cgaggacctc acccagatca 1776

ccccggagtc cgtcaaggac gtcgacatcg ccctcttctc cgccggtggt tccacctccc 1836

gtgagtgggc cccggtcttc gccgaggccg 1866

<210> 12
<211> 421
<212> PRT
<213> Corynebacterium humireducens

<400> 12

Val Ala Leu Ile Val Gln Lys Tyr Gly Gly Ser Ser Leu Glu Thr Ala
1               5                   10                  15

Glu Arg Ile Arg Ala Val Ala Glu Arg Ile Val Ala Thr Lys Lys Ala

20                  25                  30

Gly Asn Asp Val Val Val Val Cys Ser Ala Met Gly Asp Thr Thr Asp
        35                  40                  45

Glu Leu Leu Asp Leu Ala Ala Gln Val Asn Pro Val Pro Pro Ala Arg
    50                  55                  60

Glu Met Asp Met Leu Leu Thr Ala Gly Glu Arg Ile Ser Asn Ala Leu
65                  70                  75                  80

Val Ala Met Ala Ile Ser Ser Leu Gly Ala Lys Ala Gln Ser Phe Thr
                85                  90                  95

Gly Ser Gln Ala Gly Val Leu Thr Thr Glu Arg His Gly Asn Ala Arg
            100                 105                 110

Ile Val Asp Val Thr Pro Asp Arg Val Arg Glu Ala Leu Asp Glu Gly
        115                 120                 125

Asn Ile Cys Ile Val Ala Gly Phe Gln Gly Ile Asn Lys Asp Thr Arg
    130                 135                 140

Asp Val Thr Thr Leu Gly Arg Gly Gly Ser Asp Thr Thr Ala Val Ala
145                 150                 155                 160

Leu Ala Ala Ala Leu Asn Ala Asp Val Cys Glu Ile Tyr Ser Asp Val
                165                 170                 175

Asp Gly Val Tyr Thr Ala Asp Pro Arg Ile Val Pro Asn Ala Gln Lys
            180                 185                 190

Leu Glu Lys Leu Ser Phe Glu Glu Met Leu Glu Leu Ala Ala Val Gly
        195                 200                 205

Ser Lys Ile Leu Val Leu Arg Ala Val Glu Tyr Ala Arg Ala Phe Asn
    210                 215                 220

Val Pro Leu Arg Val Arg Ser Ser Tyr Ser Asn Asp Thr Gly Thr Leu
225                 230                 235                 240

Ile Ala Gly Ser Met Glu Asp Ile Pro Val Glu Glu Ala Val Leu Thr
                245                 250                 255

Gly Val Ala Thr Asp Lys Ser Glu Ala Lys Val Thr Ile Leu Gly Ile
            260                 265                 270

Pro Asp Lys Pro Gly Glu Ala Ala Thr Val Phe Arg Ala Val Ala Asp
        275                 280                 285

Ala Glu Ile Asn Ile Asp Met Val Leu Gln Asn Val Ser Ser Leu Glu
    290                 295                 300

Asp Gly Thr Thr Asp Ile Thr Phe Thr Cys Pro Arg Ala Asp Gly Pro
305                 310                 315                 320

Arg Ala Ile Glu Leu Leu Lys Lys Met Gln Ala Lys Ser Gly Trp Ser
                325                 330                 335

Asn Val Leu Tyr Asp Asp Gln Val Gly Lys Val Ser Leu Val Gly Ala
            340                 345                 350

Gly Met Lys Ser His Pro Gly Val Thr Ala Asp Phe Ala Glu Ala Leu
        355                 360                 365

Arg Asp Ala Gly Val Asn Ile Glu Leu Ile Ser Thr Ser Glu Ile Arg
    370                 375                 380

Ile Ser Val Leu Thr Arg Glu Ala Asp Leu Asp Ala Ala Ala Arg Ala
385                 390                 395                 400

Leu His Glu Lys Phe Glu Leu Gly Gly Asp Ala Glu Ala Thr Val Tyr
                405                 410                 415

Ala Gly Thr Gly Arg
            420

<210> 13
<211> 1347
<212> DNA
<213> Corynebacterium humireducens

<220>
<221> CDS
<222> (301)..(1047)
<223> gene dapB

<400> 13
ccggtgccga aggagtcgag ccaggtgcgc agggagttgc ggttgactcc gagatcggag 60

gcgatcgcgt tgatcgttgc tccgggagtc gactcgtaca acgccactgc gtcacgcttg 120

aactgctcgg tgtaggtctt gcgtggcatg gtggaaaggt acctcacttc cccagcgaaa 180

tgctggtttc agcgtgtcca ccatcaaggg gtcaggtccg ctggtgatgt tgatgcgcat 240

gcaacgattc tatgcgccta gactggggca ctgaatcttt caggaactca aggaggaacc 300

atg gcg atc aag gtg gga gtg ctc ggc gcg aag ggc cgt gtc ggc tcg 348

Met Ala Ile Lys Val Gly Val Leu Gly Ala Lys Gly Arg Val Gly Ser
1               5                   10                  15

gcg gtg tgc gag ggc gtc aac ggg gcg gag ggc ctc gag ctc gtc gcg      396
Ala Val Cys Glu Gly Val Asn Gly Ala Glu Gly Leu Glu Leu Val Ala
            20                  25                  30

cag gtc gat cag gac gat gac ctg cag ctg ctt ctc gac gcc ggg gcg      444
Gln Val Asp Gln Asp Asp Asp Leu Gln Leu Leu Leu Asp Ala Gly Ala
        35                  40                  45

gag gtc gtc gtc gac ttc acc act ccg ggt tcc gtg atg ggc aac ctg      492
Glu Val Val Val Asp Phe Thr Thr Pro Gly Ser Val Met Gly Asn Leu
    50                  55                  60

gag ttc tgc atc gcc aac ggc atc cac gcg gtg gtc ggc acc acc ggc      540
Glu Phe Cys Ile Ala Asn Gly Ile His Ala Val Val Gly Thr Thr Gly
65                  70                  75                  80

ttc gac gag gag cgc ctc gcg cag gtc cgt gag tgg tgt gcc gcc gag      588
Phe Asp Glu Glu Arg Leu Ala Gln Val Arg Glu Trp Cys Ala Ala Glu
                85                  90                  95

ggg gcg ggc aac gtc ctc atc gcc ccg aac ttc gcg atc tct gca gtg      636
Gly Ala Gly Asn Val Leu Ile Ala Pro Asn Phe Ala Ile Ser Ala Val
            100                 105                 110

ctg gcc atg gag ttc gcg cgg cag gcc gcc cgc ttc ttc gac tcc gcc      684
Leu Ala Met Glu Phe Ala Arg Gln Ala Ala Arg Phe Phe Asp Ser Ala
        115                 120                 125

gag gtc gtc gag tac cac cac ccc tac aag ctg gac gcc ccc tcc ggc      732
Glu Val Val Glu Tyr His His Pro Tyr Lys Leu Asp Ala Pro Ser Gly
    130                 135                 140

acc gcg atc cac acc gcg cag ggc atc gcc gcc gcc cgc cgg gag gcc      780
Thr Ala Ile His Thr Ala Gln Gly Ile Ala Ala Ala Arg Arg Glu Ala
145                 150                 155                 160

ggc ctc ggc ccc atc ccg gat gcc acc gag cag acc ctc gag ggt gcc      828
Gly Leu Gly Pro Ile Pro Asp Ala Thr Glu Gln Thr Leu Glu Gly Ala
                165                 170                 175

cgc ggc gcc gac gtc gac ggc gtg cgt gtc cac ggc gtg cgc atg acc      876
Arg Gly Ala Asp Val Asp Gly Val Arg Val His Gly Val Arg Met Thr
            180                 185                 190

ggc atg gtc gcc cac gag gac atc atc ttc ggt gcc cag ggc cag tcc      924
Gly Met Val Ala His Glu Asp Ile Ile Phe Gly Ala Gln Gly Gln Ser
        195                 200                 205

ctg acc atc cgc cag gac tcc tac gac cgc acc tcc ttc gtg ccg ggc      972
Leu Thr Ile Arg Gln Asp Ser Tyr Asp Arg Thr Ser Phe Val Pro Gly
    210                 215                 220

gtg ctc ctc ggt gtc cgt gag atc gcg aac cac gac ggc ctg acc gtg      1020
Val Leu Leu Gly Val Arg Glu Ile Ala Asn His Asp Gly Leu Thr Val
225                 230                 235                 240

ggg ctc gac agc tac ctg gga ctc tag atggccaagc ccgtcgacct            1067
Gly Leu Asp Ser Tyr Leu Gly Leu
                245

ggacgtccag ctcatcgcgg ccacgcagtt caccccgccc gccggggtcg actgggaggc 1127

cgacgacacc gccaccggcg ccgaggcgct cgtcgagttc gcgggccgcg cctgctacga 1187

gaccttcgac aaacccaacc cgcgcaccgc ggccaacgac gcctacctcc gccacatcat 1247

ggaggtcgga cacaccgcac tgctcgagca cgccaccgcg acgctctaca tccgcggcat 1307

ctcccgctcc gccacccacg agctggtccg ccaccgcaac 1347

<210> 14
<211> 248
<212> PRT
<213> Corynebacterium humireducens

<400> 14

Met Ala Ile Lys Val Gly Val Leu Gly Ala Lys Gly Arg Val Gly Ser
1               5                   10                  15

Ala Val Cys Glu Gly Val Asn Gly Ala Glu Gly Leu Glu Leu Val Ala
            20                  25                  30

Gln Val Asp Gln Asp Asp Asp Leu Gln Leu Leu Leu Asp Ala Gly Ala
        35                  40                  45

Glu Val Val Val Asp Phe Thr Thr Pro Gly Ser Val Met Gly Asn Leu
    50                  55                  60

Glu Phe Cys Ile Ala Asn Gly Ile His Ala Val Val Gly Thr Thr Gly
65                  70                  75                  80

Phe Asp Glu Glu Arg Leu Ala Gln Val Arg Glu Trp Cys Ala Ala Glu
                85                  90                  95

Gly Ala Gly Asn Val Leu Ile Ala Pro Asn Phe Ala Ile Ser Ala Val
            100                 105                 110

Leu Ala Met Glu Phe Ala Arg Gln Ala Ala Arg Phe Phe Asp Ser Ala
        115                 120                 125

Glu Val Val Glu Tyr His His Pro Tyr Lys Leu Asp Ala Pro Ser Gly
    130                 135                 140

Thr Ala Ile His Thr Ala Gln Gly Ile Ala Ala Ala Arg Arg Glu Ala
145                 150                 155                 160

Gly Leu Gly Pro Ile Pro Asp Ala Thr Glu Gln Thr Leu Glu Gly Ala
                165                 170                 175

Arg Gly Ala Asp Val Asp Gly Val Arg Val His Gly Val Arg Met Thr

180                 185                 190

Gly Met Val Ala His Glu Asp Ile Ile Phe Gly Ala Gln Gly Gln Ser
        195                 200                 205

Leu Thr Ile Arg Gln Asp Ser Tyr Asp Arg Thr Ser Phe Val Pro Gly
    210                 215                 220

Val Leu Leu Gly Val Arg Glu Ile Ala Asn His Asp Gly Leu Thr Val
225                 230                 235                 240

Gly Leu Asp Ser Tyr Leu Gly Leu
                245

<210>  15
<211>  2145
<212>  DNA
<213>  Corynebacterium humireducens

<220>
<221>  CDS
<222>  (301)..(1845)
<223>  gene glucose-6-phosphate dehydrogenase

<400>  15
cgaccctcta cgtcaccgag ctggccggcc cggagaccgt caacaccatg ccggagccga      60

ccatcgacgc cgtgctcgag cagggtggcc tccacggcga caccctgagc ggcaccgcgg     120

acgacgcgca ggccgtcttc gagcagctgg tgcaggtcgg catcgacatg gaggacgtct     180

tcgcggtgct ggagcgcgag ggcgtcgaga agttcgtgtc ctcctggtcc gagctgctcg     240

actccatgga gtcccgactg cgctagcagt cccctccccg acaggaaagg acctgagcgc     300

atg ccc ctc ccc gtg acc ggc acc gac gcc tgg atc aac ccg ctg cgg      348
Met Pro Leu Pro Val Thr Gly Thr Asp Ala Trp Ile Asn Pro Leu Arg
1               5                   10                  15

aac acc gtc gac aag cgg ctg ccc cgc atc gcc ggc ccc tcc ggc atg      396
Asn Thr Val Asp Lys Arg Leu Pro Arg Ile Ala Gly Pro Ser Gly Met
            20                  25                  30

gtg atc ttc ggc gtg acc ggt gac ctg gcc cgc aag aag ctg ctc ccg      444
Val Ile Phe Gly Val Thr Gly Asp Leu Ala Arg Lys Lys Leu Leu Pro
        35                  40                  45

gcc atc tac gac ctg gcc aac cgt ggc ctc ctg ccg gcc ggt ttc acc      492
Ala Ile Tyr Asp Leu Ala Asn Arg Gly Leu Leu Pro Ala Gly Phe Thr
    50                  55                  60

ctc atc ggt ttc ggc cgt cgt ccc tgg agc aaa ccc gag ttc gag gag      540
Leu Ile Gly Phe Gly Arg Arg Pro Trp Ser Lys Pro Glu Phe Glu Glu
65                  70                  75                  80

tac gtg cgg gag gcc gtc gag agc ggc gcc cgc acc gag ttc cgc gaa      588
Tyr Val Arg Glu Ala Val Glu Ser Gly Ala Arg Thr Glu Phe Arg Glu

85                  90                  95

cac gtg tgg gag cgg ctg gcc gag ggc atg cac ttc gtc gag ggc aac          636
His Val Trp Glu Arg Leu Ala Glu Gly Met His Phe Val Glu Gly Asn
            100                 105                 110

ttc gac gac gag ggc ttc gac cgc ctc gcc gcc acc ctc gcc gag atg          684
Phe Asp Asp Glu Gly Phe Asp Arg Leu Ala Ala Thr Leu Ala Glu Met
        115                 120                 125

gac acc tcc cgc ggc acg gcg ggc aac tgg gcc ttc tac ctg tcg gtc          732
Asp Thr Ser Arg Gly Thr Ala Gly Asn Trp Ala Phe Tyr Leu Ser Val
    130                 135                 140

ccg ccg gac ttc ttc acc gcc gtc tgc cac cag ctg gag cgc tcc ggc          780
Pro Pro Asp Phe Phe Thr Ala Val Cys His Gln Leu Glu Arg Ser Gly
145                 150                 155                 160

ctg gcg gag gcc ggt gag gag tcg tgg cgt cgc gtg atc atc gag aag          828
Leu Ala Glu Ala Gly Glu Glu Ser Trp Arg Arg Val Ile Ile Glu Lys
                165                 170                 175

ccc ttc ggc cac gac cag gac acg gcc cgg gag ctc aac cgg gtg gtc          876
Pro Phe Gly His Asp Gln Asp Thr Ala Arg Glu Leu Asn Arg Val Val
            180                 185                 190

aac gcc gtc ttc ccg gag gag tcg gtg ttc cgc atc gac cac tac ctg          924
Asn Ala Val Phe Pro Glu Glu Ser Val Phe Arg Ile Asp His Tyr Leu
        195                 200                 205

ggc aag gag acg gtg cag aac atc ctg gcg ctg cgc ttc gcc aac cag          972
Gly Lys Glu Thr Val Gln Asn Ile Leu Ala Leu Arg Phe Ala Asn Gln
    210                 215                 220

ctc ttc gac ccg atc tgg aac gcg cac tac gtc gac cac gtg cag atc          1020
Leu Phe Asp Pro Ile Trp Asn Ala His Tyr Val Asp His Val Gln Ile
225                 230                 235                 240

acc atg gcc gag gac atc ggt ctc ggt ggt cgg gcc ggc tac tac gac          1068
Thr Met Ala Glu Asp Ile Gly Leu Gly Gly Arg Ala Gly Tyr Tyr Asp
                245                 250                 255

ggc atc ggc gcc gcc cgc gac gtc atg cag aac cac ctc ctc cag ctc          1116
Gly Ile Gly Ala Ala Arg Asp Val Met Gln Asn His Leu Leu Gln Leu
            260                 265                 270

ctg gcg ctg gtg gcc atg gag gag ccc atc gcc ttc gac ccg gct gag          1164
Leu Ala Leu Val Ala Met Glu Glu Pro Ile Ala Phe Asp Pro Ala Glu
        275                 280                 285

ctg cag gcg gag aag gtc aag gtc ctg cgc gcg acc aac ccc gtc ttc          1212
Leu Gln Ala Glu Lys Val Lys Val Leu Arg Ala Thr Asn Pro Val Phe
    290                 295                 300

ccg ctg aac aag acc acc gcc cgt ggc cag tac gcg gcg ggc tgg cag          1260
Pro Leu Asn Lys Thr Thr Ala Arg Gly Gln Tyr Ala Ala Gly Trp Gln
305                 310                 315                 320

ggc tcg cac aag gtg cgt ggc ctg cgt gag gag gag ggt ttc gac ccg          1308
Gly Ser His Lys Val Arg Gly Leu Arg Glu Glu Glu Gly Phe Asp Pro
                325                 330                 335

gac tcc acc acg gag acc tac gcc gcc tgc acc ctg gag atc aac tcc          1356

Asp Ser Thr Thr Glu Thr Tyr Ala Ala Cys Thr Leu Glu Ile Asn Ser
            340                 345                 350

cgc cgc tgg gcg ggc gtg ccc ttc tac ctg cgg acc ggc aag cgc ctg      1404
Arg Arg Trp Ala Gly Val Pro Phe Tyr Leu Arg Thr Gly Lys Arg Leu
        355                 360                 365

ggg cgc cgc gtc acg gag atc gcc ctc gtg ttc aag gag gcg ccg cac      1452
Gly Arg Arg Val Thr Glu Ile Ala Leu Val Phe Lys Glu Ala Pro His
    370                 375                 380

cag ccc ttc ctc gag ggg cag acg tcc tcg ctg ggg cag aac gcg atc      1500
Gln Pro Phe Leu Glu Gly Gln Thr Ser Ser Leu Gly Gln Asn Ala Ile
385                 390                 395                 400

gtc atc cgc gtc cag ccc gac gag ggc gtg ctc atg cgc ttc ggc tcc      1548
Val Ile Arg Val Gln Pro Asp Glu Gly Val Leu Met Arg Phe Gly Ser
                405                 410                 415

aag gtg ccc ggc tcc gcg atg gag gtc cgt gac gtc aac atg gac ttc      1596
Lys Val Pro Gly Ser Ala Met Glu Val Arg Asp Val Asn Met Asp Phe
            420                 425                 430

tcc tac gcg gag gcc ttc acc gag gcg tcc ccg gag gcc tat gag cgg      1644
Ser Tyr Ala Glu Ala Phe Thr Glu Ala Ser Pro Glu Ala Tyr Glu Arg
        435                 440                 445

ctc atc ctc gat gcg ctt ctc gac gag tcc tcg ctc ttc ccc acc aac      1692
Leu Ile Leu Asp Ala Leu Leu Asp Glu Ser Ser Leu Phe Pro Thr Asn
    450                 455                 460

gag gag gtc gag ctc tcc tgg gcg atc ctg gac ccg atc ctc gag ttc      1740
Glu Glu Val Glu Leu Ser Trp Ala Ile Leu Asp Pro Ile Leu Glu Phe
465                 470                 475                 480

tgg gct gag aag ggc cag ccc gag gag tac gag tcc ggc acc tgg ggg      1788
Trp Ala Glu Lys Gly Gln Pro Glu Glu Tyr Glu Ser Gly Thr Trp Gly
                485                 490                 495

ccc gcc tcc gcc gac cgc atg ctg gaa cgg acc ggc cgc gcc tgg cgc      1836
Pro Ala Ser Ala Asp Arg Met Leu Glu Arg Thr Gly Arg Ala Trp Arg
            500                 505                 510

cgc ccc tga cctggaaggg acccgacttg aagatcgaac tgccggacac              1885
Arg Pro

cgacacccgg aacatcgccg ccagcctgct cgaggcccgg gagcacaact cccagaccac    1945

cgggcgggtg ctcaccctca tcgtcgtcgc cgcgctcgac gacgacgtcg accacatcat    2005

ctccgtcacc cgcgacgcct cccacgagca ccccgcccgt gtgctggtgc tgctcaccgg    2065

ccgggaggac gctgactcgc gtctcgacgc cgaggtgcgc gtcggcggtc acgccggcgc    2125

ctcggagatg gtcatcatgc                                                2145

<210>  16
<211>  514
<212>  PRT
<213>  Corynebacterium humireducens

<400>  16

Met Pro Leu Pro Val Thr Gly Thr Asp Ala Trp Ile Asn Pro Leu Arg
1               5                   10                  15

Asn Thr Val Asp Lys Arg Leu Pro Arg Ile Ala Gly Pro Ser Gly Met
            20                  25                  30

Val Ile Phe Gly Val Thr Gly Asp Leu Ala Arg Lys Lys Leu Leu Pro
        35                  40                  45

Ala Ile Tyr Asp Leu Ala Asn Arg Gly Leu Leu Pro Ala Gly Phe Thr
    50                  55                  60

Leu Ile Gly Phe Gly Arg Arg Pro Trp Ser Lys Pro Glu Phe Glu Glu
65                  70                  75                  80

Tyr Val Arg Glu Ala Val Glu Ser Gly Ala Arg Thr Glu Phe Arg Glu
                85                  90                  95

His Val Trp Glu Arg Leu Ala Glu Gly Met His Phe Val Glu Gly Asn
            100                 105                 110

Phe Asp Asp Glu Gly Phe Asp Arg Leu Ala Ala Thr Leu Ala Glu Met
        115                 120                 125

Asp Thr Ser Arg Gly Thr Ala Gly Asn Trp Ala Phe Tyr Leu Ser Val
    130                 135                 140

Pro Pro Asp Phe Phe Thr Ala Val Cys His Gln Leu Glu Arg Ser Gly
145                 150                 155                 160

Leu Ala Glu Ala Gly Glu Glu Ser Trp Arg Arg Val Ile Ile Glu Lys
                165                 170                 175

Pro Phe Gly His Asp Gln Asp Thr Ala Arg Glu Leu Asn Arg Val Val
            180                 185                 190

Asn Ala Val Phe Pro Glu Glu Ser Val Phe Arg Ile Asp His Tyr Leu
        195                 200                 205

Gly Lys Glu Thr Val Gln Asn Ile Leu Ala Leu Arg Phe Ala Asn Gln
    210                 215                 220

Leu Phe Asp Pro Ile Trp Asn Ala His Tyr Val Asp His Val Gln Ile
225                 230                 235                 240

Thr Met Ala Glu Asp Ile Gly Leu Gly Gly Arg Ala Gly Tyr Tyr Asp

245                 250                 255

Gly Ile Gly Ala Ala Arg Asp Val Met Gln Asn His Leu Leu Gln Leu
            260                 265                 270

Leu Ala Leu Val Ala Met Glu Glu Pro Ile Ala Phe Asp Pro Ala Glu
        275                 280                 285

Leu Gln Ala Glu Lys Val Lys Val Leu Arg Ala Thr Asn Pro Val Phe
    290                 295                 300

Pro Leu Asn Lys Thr Thr Ala Arg Gly Gln Tyr Ala Ala Gly Trp Gln
305                 310                 315                 320

Gly Ser His Lys Val Arg Gly Leu Arg Glu Glu Glu Gly Phe Asp Pro
                325                 330                 335

Asp Ser Thr Thr Glu Thr Tyr Ala Ala Cys Thr Leu Glu Ile Asn Ser
            340                 345                 350

Arg Arg Trp Ala Gly Val Pro Phe Tyr Leu Arg Thr Gly Lys Arg Leu
        355                 360                 365

Gly Arg Arg Val Thr Glu Ile Ala Leu Val Phe Lys Glu Ala Pro His
    370                 375                 380

Gln Pro Phe Leu Glu Gly Gln Thr Ser Ser Leu Gly Gln Asn Ala Ile
385                 390                 395                 400

Val Ile Arg Val Gln Pro Asp Glu Gly Val Leu Met Arg Phe Gly Ser
                405                 410                 415

Lys Val Pro Gly Ser Ala Met Glu Val Arg Asp Val Asn Met Asp Phe
            420                 425                 430

Ser Tyr Ala Glu Ala Phe Thr Glu Ala Ser Pro Glu Ala Tyr Glu Arg
        435                 440                 445

Leu Ile Leu Asp Ala Leu Leu Asp Glu Ser Ser Leu Phe Pro Thr Asn
    450                 455                 460

Glu Glu Val Glu Leu Ser Trp Ala Ile Leu Asp Pro Ile Leu Glu Phe
465                 470                 475                 480

Trp Ala Glu Lys Gly Gln Pro Glu Glu Tyr Glu Ser Gly Thr Trp Gly
                485                 490                 495

Pro Ala Ser Ala Asp Arg Met Leu Glu Arg Thr Gly Arg Ala Trp Arg
            500                 505                 510

Arg Pro

<210> 17
<211> 2055
<212> DNA
<213> Corynebacterium humireducens

<220>
<221> CDS
<222> (301)..(1755)
<223> gene gnd

<400> 17
aaggctgccc acggactcgg ccagcgcgca gaacacgccc ccgttgacca ggccgacggg 60

ctgccggtgc tggtccccca cctcgaggcg ggcgcggacg cggccgtcgg cgatctgcgt 120

gtagacgatc ccgaggtggg cgtcgaggcc gacgttgcgg tcgttgagct gcgcgagctg 180

cgcggcgtcg agggccttgc ctgtcccggc gaggcggaag aggtcggtga aactgtaggt 240

ctcggtcatg agtgccgact ctactgaccc ttcgaaaatg gtgtgtctaa actctgtgcc 300

atg acc tcc cct gag aat ctc gcc cag atc ggc gtc gtc gga ctc gcc 348
Met Thr Ser Pro Glu Asn Leu Ala Gln Ile Gly Val Val Gly Leu Ala
1               5                   10                  15

gtc atg ggt tcc aac ctg gcc cgc aac ttc gcc cgc aac ggc cac acc 396
Val Met Gly Ser Asn Leu Ala Arg Asn Phe Ala Arg Asn Gly His Thr
            20                  25                  30

gtc gcc gtg tac aac cgt tcc ccc gag aag acc cgc gac ctc atc gcc 444
Val Ala Val Tyr Asn Arg Ser Pro Glu Lys Thr Arg Asp Leu Ile Ala
        35                  40                  45

gag cac ggt gac gag ggc acc ttc ctc gcc tcc gag acc gtc gag gac 492
Glu His Gly Asp Glu Gly Thr Phe Leu Ala Ser Glu Thr Val Glu Asp
    50                  55                  60

ttc gtc gcc tcc ctc gag cgc ccg cgt aag gcg atc atc atg gtc cag 540
Phe Val Ala Ser Leu Glu Arg Pro Arg Lys Ala Ile Ile Met Val Gln
65                  70                  75                  80

gcc ggc gcg gcc acc gac gcg gtg atc aac cag ctg gcc gac gcc atg 588
Ala Gly Ala Ala Thr Asp Ala Val Ile Asn Gln Leu Ala Asp Ala Met
                85                  90                  95

gag gac ggc gac atc atc atc gac ggc ggc aac gcc ctc tac agc gac 636
Glu Asp Gly Asp Ile Ile Ile Asp Gly Gly Asn Ala Leu Tyr Ser Asp
            100                 105                 110

acc atc cgc cgg gag cgg gag atg gcc gag cgc aac cgt cac ttc gtc 684
Thr Ile Arg Arg Glu Arg Glu Met Ala Glu Arg Asn Arg His Phe Val
        115                 120                 125

ggc gcc ggc atc tcc ggc ggc gag gag ggg gcc ctc aac ggc ccg tcg 732

Gly Ala Gly Ile Ser Gly Gly Glu Glu Gly Ala Leu Asn Gly Pro Ser
    130                 135                 140

atc atg ccg ggt ggc ccg aag gag tcc tgg gag acc ctc ggc ccg ctg      780
Ile Met Pro Gly Gly Pro Lys Glu Ser Trp Glu Thr Leu Gly Pro Leu
145                 150                 155                 160

ctg gag tcc gtc gct gcc gtc gtc gac ggc acc ccg tgt gtc acg cac      828
Leu Glu Ser Val Ala Ala Val Val Asp Gly Thr Pro Cys Val Thr His
                165                 170                 175

atc ggc ccg gac ggt gcg ggc cac ttc gtc aag atg gtc cac aac ggc      876
Ile Gly Pro Asp Gly Ala Gly His Phe Val Lys Met Val His Asn Gly
            180                 185                 190

atc gag tac gcc gac atg cag gtc atc ggc gag gcg tac cag ctg ctg      924
Ile Glu Tyr Ala Asp Met Gln Val Ile Gly Glu Ala Tyr Gln Leu Leu
        195                 200                 205

cgc tac ggc gcg ggc atg agc ccg gcc gag atc tcc gag gtc ttc cgc      972
Arg Tyr Gly Ala Gly Met Ser Pro Ala Glu Ile Ser Glu Val Phe Arg
    210                 215                 220

gag tgg aac tcc ggc gac ctc gac tcc tac ctc atc gag atc acc gcc     1020
Glu Trp Asn Ser Gly Asp Leu Asp Ser Tyr Leu Ile Glu Ile Thr Ala
225                 230                 235                 240

gag gtc ctc gcg cag gtc gac gcc gag acg ggg aag ccg ctc atc gac     1068
Glu Val Leu Ala Gln Val Asp Ala Glu Thr Gly Lys Pro Leu Ile Asp
                245                 250                 255

gtc atc gtc gac gcc gcc ggc cag aag ggc acc ggc cgc tgg acc gtc     1116
Val Ile Val Asp Ala Ala Gly Gln Lys Gly Thr Gly Arg Trp Thr Val
            260                 265                 270

aag gcc gcc ctc gac ctg ggc atc ccg gtc acc ggc atc ggc gag gcc     1164
Lys Ala Ala Leu Asp Leu Gly Ile Pro Val Thr Gly Ile Gly Glu Ala
        275                 280                 285

gtc ttc gcc cgc gcc ctg tcc tcc tcc ctc gag cag cgt gcc gcc gcg     1212
Val Phe Ala Arg Ala Leu Ser Ser Ser Leu Glu Gln Arg Ala Ala Ala
    290                 295                 300

cag ggc gtg ctg ccc gcc ggt gag ctc gcc acc ttc gag tcc ctg ggt     1260
Gln Gly Val Leu Pro Ala Gly Glu Leu Ala Thr Phe Glu Ser Leu Gly
305                 310                 315                 320

ctg gac cgc gac gcc ttc atc gag gac gtc cgc cgc gcc ctc tac gcc     1308
Leu Asp Arg Asp Ala Phe Ile Glu Asp Val Arg Arg Ala Leu Tyr Ala
                325                 330                 335

tcc aag ctg gtc gcc tac gcg cag ggc ttc gac gag atc cgg gcc ggc     1356
Ser Lys Leu Val Ala Tyr Ala Gln Gly Phe Asp Glu Ile Arg Ala Gly
            340                 345                 350

tcc gcc gag cac gcc tgg gac gtc gac ccc cgc gac ctg gcc acc atc     1404
Ser Ala Glu His Ala Trp Asp Val Asp Pro Arg Asp Leu Ala Thr Ile
        355                 360                 365

tgg cgg ggc ggc tgc atc atc cgc gcc aag ttc ctc aac cgc atc gtc     1452
Trp Arg Gly Gly Cys Ile Ile Arg Ala Lys Phe Leu Asn Arg Ile Val
    370                 375                 380

gag gcc tac gac gcc gac ccg cag ctg ccg tcc ctg ctt ctc gac gcc     1500
Glu Ala Tyr Asp Ala Asp Pro Gln Leu Pro Ser Leu Leu Leu Asp Ala
385                 390                 395                 400

tac ttc ctc gac gag ctg cgc gac ctc atc gac tcc tgg cgt cgc gtc     1548
Tyr Phe Leu Asp Glu Leu Arg Asp Leu Ile Asp Ser Trp Arg Arg Val
                405                 410                 415

atc gtc gcc gcc acc cag ctg ggc ctg ccg gtg ccg gtg ttc gcg tcc     1596
Ile Val Ala Ala Thr Gln Leu Gly Leu Pro Val Pro Val Phe Ala Ser
            420                 425                 430

tcc ctg tcc tac tac gac tcg ctg cgc gcc gag cgt ctg ccg gcc gcc     1644
Ser Leu Ser Tyr Tyr Asp Ser Leu Arg Ala Glu Arg Leu Pro Ala Ala
        435                 440                 445

gtg atc cag ggc cag cgt gac tac ttc ggt gcg cac acc tac ggg cgc     1692
Val Ile Gln Gly Gln Arg Asp Tyr Phe Gly Ala His Thr Tyr Gly Arg
    450                 455                 460

gtc gac aag ccg ggt gcc ttc cac acc ctg tgg tcc ggg gac cgc agc     1740
Val Asp Lys Pro Gly Ala Phe His Thr Leu Trp Ser Gly Asp Arg Ser
465                 470                 475                 480

gag atc gag gtg taa tgcccgtgtg ggcgaggatc tcccgtttcc tgccggcggc     1795
Glu Ile Glu Val

ggccatcgtg gtggtgaccg ccagggcagt gcaggaggtc cttgcccacg ggcagtgggc     1855

ggtgtggcgg ctgtcgcggc gggagacccg cctgttcgtc gaggtattcc ggacggtgct     1915

gggcctgttc ctcgtcgtcg gcctcacccg ggtggtcgcc gcgcctccct cccccacgga     1975

actggccgga tgggcgctca ccttggggtt cctgtggatc ctcaccctca cggagacgga     2035

acccggcccc gaccccgagc                                                 2055

<210>  18
<211>  484
<212>  PRT
<213>  Corynebacterium humireducens

<400>  18

Met Thr Ser Pro Glu Asn Leu Ala Gln Ile Gly Val Val Gly Leu Ala
1               5                   10                  15

Val Met Gly Ser Asn Leu Ala Arg Asn Phe Ala Arg Asn Gly His Thr
            20                  25                  30

Val Ala Val Tyr Asn Arg Ser Pro Glu Lys Thr Arg Asp Leu Ile Ala
        35                  40                  45

Glu His Gly Asp Glu Gly Thr Phe Leu Ala Ser Glu Thr Val Glu Asp
    50                  55                  60

Phe Val Ala Ser Leu Glu Arg Pro Arg Lys Ala Ile Ile Met Val Gln

65                  70                  75                  80

Ala Gly Ala Ala Thr Asp Ala Val Ile Asn Gln Leu Ala Asp Ala Met
                85                  90                  95

Glu Asp Gly Asp Ile Ile Ile Asp Gly Gly Asn Ala Leu Tyr Ser Asp
            100                 105                 110

Thr Ile Arg Arg Glu Arg Glu Met Ala Glu Arg Asn Arg His Phe Val
        115                 120                 125

Gly Ala Gly Ile Ser Gly Gly Glu Glu Gly Ala Leu Asn Gly Pro Ser
    130                 135                 140

Ile Met Pro Gly Gly Pro Lys Glu Ser Trp Glu Thr Leu Gly Pro Leu
145                 150                 155                 160

Leu Glu Ser Val Ala Ala Val Val Asp Gly Thr Pro Cys Val Thr His
                165                 170                 175

Ile Gly Pro Asp Gly Ala Gly His Phe Val Lys Met Val His Asn Gly
            180                 185                 190

Ile Glu Tyr Ala Asp Met Gln Val Ile Gly Glu Ala Tyr Gln Leu Leu
        195                 200                 205

Arg Tyr Gly Ala Gly Met Ser Pro Ala Glu Ile Ser Glu Val Phe Arg
    210                 215                 220

Glu Trp Asn Ser Gly Asp Leu Asp Ser Tyr Leu Ile Glu Ile Thr Ala
225                 230                 235                 240

Glu Val Leu Ala Gln Val Asp Ala Glu Thr Gly Lys Pro Leu Ile Asp
                245                 250                 255

Val Ile Val Asp Ala Ala Gly Gln Lys Gly Thr Gly Arg Trp Thr Val
            260                 265                 270

Lys Ala Ala Leu Asp Leu Gly Ile Pro Val Thr Gly Ile Gly Glu Ala
        275                 280                 285

Val Phe Ala Arg Ala Leu Ser Ser Ser Leu Glu Gln Arg Ala Ala Ala
    290                 295                 300

Gln Gly Val Leu Pro Ala Gly Glu Leu Ala Thr Phe Glu Ser Leu Gly
305                 310                 315                 320

Leu Asp Arg Asp Ala Phe Ile Glu Asp Val Arg Arg Ala Leu Tyr Ala
                325                 330                 335

Ser Lys Leu Val Ala Tyr Ala Gln Gly Phe Asp Glu Ile Arg Ala Gly
            340                 345                 350

Ser Ala Glu His Ala Trp Asp Val Asp Pro Arg Asp Leu Ala Thr Ile
        355                 360                 365

Trp Arg Gly Gly Cys Ile Ile Arg Ala Lys Phe Leu Asn Arg Ile Val
    370                 375                 380

Glu Ala Tyr Asp Ala Asp Pro Gln Leu Pro Ser Leu Leu Leu Asp Ala
385                 390                 395                 400

Tyr Phe Leu Asp Glu Leu Arg Asp Leu Ile Asp Ser Trp Arg Arg Val
                405                 410                 415

Ile Val Ala Ala Thr Gln Leu Gly Leu Pro Val Pro Val Phe Ala Ser
            420                 425                 430

Ser Leu Ser Tyr Tyr Asp Ser Leu Arg Ala Glu Arg Leu Pro Ala Ala
        435                 440                 445

Val Ile Gln Gly Gln Arg Asp Tyr Phe Gly Ala His Thr Tyr Gly Arg
    450                 455                 460

Val Asp Lys Pro Gly Ala Phe His Thr Leu Trp Ser Gly Asp Arg Ser
465                 470                 475                 480

Glu Ile Glu Val

<210>  19
<211>  2103
<212>  DNA
<213>  Corynebacterium humireducens

<220>
<221>  CDS
<222>  (301)..(1803)
<223>  gene mqo

<400>  19
cgagaatgtc ctcataccag tcggagaagg cgggaacgct catgatgacc acactagtgg      60

gggtacgggg gcagggggcg ggggagtgag gggcgtcgat aagttcgtgt tttccggacc     120

ttcgtcacac ccccgtgaac ctaaggcgta aggttgatgt ttaccggaca aggggagcgg     180

atctgtgccg aggacgtcag agtccgcacc cggaattgtc ctgcccaccg ggagtagtgc     240

cgacacccgg tgtgggccat gactggccag aaccttcgac gacgaatgag gtaatgcaag     300

gtg tcc acc gaa aac aag aac gct ccc aag gtc act gac gag gtc gat     348
Val Ser Thr Glu Asn Lys Asn Ala Pro Lys Val Thr Asp Glu Val Asp
1               5                   10                  15

gtc gcc ctc gtc ggc gcg ggt gtc atg agc gcc acc ctg ggt gcg atg     396
Val Ala Leu Val Gly Ala Gly Val Met Ser Ala Thr Leu Gly Ala Met
            20                  25                  30

atc cgc gag ctc gag ccg agc tgg tcc cag atg atc ttc gag cgc ctg     444
Ile Arg Glu Leu Glu Pro Ser Trp Ser Gln Met Ile Phe Glu Arg Leu
        35                  40                  45

gac ggc ccc gcc cag gag tcc tcc tcc ccg tgg aac aac gcc ggt acc     492
Asp Gly Pro Ala Gln Glu Ser Ser Ser Pro Trp Asn Asn Ala Gly Thr
    50                  55                  60

ggc cac tcc gca ctc tgc gag ctg aac tac acc ccc gag gtc aac ggc     540
Gly His Ser Ala Leu Cys Glu Leu Asn Tyr Thr Pro Glu Val Asn Gly
65                  70                  75                  80

aag atc gag gtg gcc aag gcc atc ggc atc aac gag aag ttc cag gtc     588
Lys Ile Glu Val Ala Lys Ala Ile Gly Ile Asn Glu Lys Phe Gln Val
                85                  90                  95

tcc cgc cag ttc tgg tcc cac cag atc gac cac ggc gtg ctg gac gac     636
Ser Arg Gln Phe Trp Ser His Gln Ile Asp His Gly Val Leu Asp Asp
            100                 105                 110

ccc aag gag tgg atc aac ccc gtc gcc cac ctg gcc ttc gcc cag ggc     684
Pro Lys Glu Trp Ile Asn Pro Val Ala His Leu Ala Phe Ala Gln Gly
        115                 120                 125

gac gac cag gtc gac tac ctc aag cgc cgc cac gag gcg ctg gcc gac     732
Asp Asp Gln Val Asp Tyr Leu Lys Arg Arg His Glu Ala Leu Ala Asp
    130                 135                 140

aac atc ctc ttc ccg ggc atg gag tgg gtc gag gac gac gcc aag ttc     780
Asn Ile Leu Phe Pro Gly Met Glu Trp Val Glu Asp Asp Ala Lys Phe
145                 150                 155                 160

gcc gag cgc ctc ccg gtc atg tcc gag ggc cgt gac ttc gcc gcc gag     828
Ala Glu Arg Leu Pro Val Met Ser Glu Gly Arg Asp Phe Ala Ala Glu
                165                 170                 175

aag gtc gcc tac tcc ggc acc gac atc ggc acc gac gtc aac ttc ggt     876
Lys Val Ala Tyr Ser Gly Thr Asp Ile Gly Thr Asp Val Asn Phe Gly
            180                 185                 190

gcc ctg acc gag cag ttc ctc act gcc gcc gag gcc tcc ggc acg gag     924
Ala Leu Thr Glu Gln Phe Leu Thr Ala Ala Glu Ala Ser Gly Thr Glu
        195                 200                 205

atc cgc tac ggc cac gag gtc aag aac atc aag aag gac ggt ggc aag     972
Ile Arg Tyr Gly His Glu Val Lys Asn Ile Lys Lys Asp Gly Gly Lys
    210                 215                 220

tgg aag atc acc gtc aag aac gtc cac acg ggt gac acc tcc gtg gtc     1020
Trp Lys Ile Thr Val Lys Asn Val His Thr Gly Asp Thr Ser Val Val
225                 230                 235                 240

aag gcc aag ttc gtg ttc gtc ggc gcc ggc ggc tac gcc ctc gac ctg 1068
Lys Ala Lys Phe Val Phe Val Gly Ala Gly Gly Tyr Ala Leu Asp Leu
                245                 250                 255

ctg cgc aag gca ggc gtc ccg gag gtc agc ggt tac gcc ggc ttc ccg 1116
Leu Arg Lys Ala Gly Val Pro Glu Val Ser Gly Tyr Ala Gly Phe Pro
            260                 265                 270

gtg tcc ggc ctg tgg ctg cgc tgc acc aac gag gag ctg atc aac cag 1164
Val Ser Gly Leu Trp Leu Arg Cys Thr Asn Glu Glu Leu Ile Asn Gln
        275                 280                 285

cac gac gcc aag gtc tac ggc aag gcc aag gtc ggt gcc ccg ccg atg 1212
His Asp Ala Lys Val Tyr Gly Lys Ala Lys Val Gly Ala Pro Pro Met
    290                 295                 300

tcc gtc ccg cac ctt gac acc cgc gtc atc gac ggc aag aag ggc ctc 1260
Ser Val Pro His Leu Asp Thr Arg Val Ile Asp Gly Lys Lys Gly Leu
305                 310                 315                 320

ctg ttc ggc ccc tac ggc ggc tgg agc ccg aag ttc ctc aag gag ggt 1308
Leu Phe Gly Pro Tyr Gly Gly Trp Ser Pro Lys Phe Leu Lys Glu Gly
                325                 330                 335

tcg tac ctc gac ctg ttc aag tcg atc cgc ctg gac aac ctc ccg tcc 1356
Ser Tyr Leu Asp Leu Phe Lys Ser Ile Arg Leu Asp Asn Leu Pro Ser
            340                 345                 350

tac ctc ggt gtc gcc gcc cag gag ttc gcc ctg acc aag tac ctc atc 1404
Tyr Leu Gly Val Ala Ala Gln Glu Phe Ala Leu Thr Lys Tyr Leu Ile
        355                 360                 365

acc gag gtg gcg aag gac ttc gag aag cgc ctc gtc gac ctg cgt gac 1452
Thr Glu Val Ala Lys Asp Phe Glu Lys Arg Leu Val Asp Leu Arg Asp
    370                 375                 380

tac gtc ccg tcc gcc aag gcc gag gac tgg gag acc gtg gtc gcc ggt 1500
Tyr Val Pro Ser Ala Lys Ala Glu Asp Trp Glu Thr Val Val Ala Gly
385                 390                 395                 400

cag cgc gtc cag gtg atc aag ccg gcc cgc gcg ccg cgc ttc ggc tcc 1548
Gln Arg Val Gln Val Ile Lys Pro Ala Arg Ala Pro Arg Phe Gly Ser
                405                 410                 415

ctc gag ttc ggc acc gcc ctg atc aac agc acc gac ggc tcc atc gcc 1596
Leu Glu Phe Gly Thr Ala Leu Ile Asn Ser Thr Asp Gly Ser Ile Ala
            420                 425                 430

ggc ctg ctg ggt gcc tcc ccg ggt gcc tcc atc gcc ccg gcc gcc atg 1644
Gly Leu Leu Gly Ala Ser Pro Gly Ala Ser Ile Ala Pro Ala Ala Met
        435                 440                 445

ctc gag ctc gtc gag cgc tgc ttc ggt gac cgc atg atc cag tgg ggc 1692
Leu Glu Leu Val Glu Arg Cys Phe Gly Asp Arg Met Ile Gln Trp Gly
    450                 455                 460

ccg aag ctc aag gag atg atc ccg tcc tac ggc acc aag ctg ggc gac 1740
Pro Lys Leu Lys Glu Met Ile Pro Ser Tyr Gly Thr Lys Leu Gly Asp
465                 470                 475                 480

gac gag aag atg ttc aac gag ctc tgg gac tac acc cag aag acc ctg 1788
Asp Glu Lys Met Phe Asn Glu Leu Trp Asp Tyr Thr Gln Lys Thr Leu
                485                 490                 495

aag ctc gag aag tag ttccactgtg agggccgcga ccaccgggtc gcggccttcg 1843
Lys Leu Glu Lys
            500

ccgattccgt aggctggtgg ccatgcaacg catcgtgccc accatctggt tcaaccggaa 1903

cgccgacgag gccgccgagt tctacgccga cgtcttcccc gacacgcttg tcgacgcccg 1963

cgtcccccac cccgaatccg gcgagaccct caccgtcgac gtgctcatcg acggctaccg 2023

cgtcaccctc ctcaacggtg ggccggagtt cggcccgacc cccgcgatca gcttcttcgt 2083

caacttcgac ccctcccgca 2103

<210> 20
<211> 500
<212> PRT
<213> Corynebacterium humireducens

<400> 20

Val Ser Thr Glu Asn Lys Asn Ala Pro Lys Val Thr Asp Glu Val Asp
1               5                   10                  15

Val Ala Leu Val Gly Ala Gly Val Met Ser Ala Thr Leu Gly Ala Met
            20                  25                  30

Ile Arg Glu Leu Glu Pro Ser Trp Ser Gln Met Ile Phe Glu Arg Leu
        35                  40                  45

Asp Gly Pro Ala Gln Glu Ser Ser Ser Pro Trp Asn Asn Ala Gly Thr
    50                  55                  60

Gly His Ser Ala Leu Cys Glu Leu Asn Tyr Thr Pro Glu Val Asn Gly
65                  70                  75                  80

Lys Ile Glu Val Ala Lys Ala Ile Gly Ile Asn Glu Lys Phe Gln Val
                85                  90                  95

Ser Arg Gln Phe Trp Ser His Gln Ile Asp His Gly Val Leu Asp Asp
            100                 105                 110

Pro Lys Glu Trp Ile Asn Pro Val Ala His Leu Ala Phe Ala Gln Gly
        115                 120                 125

Asp Asp Gln Val Asp Tyr Leu Lys Arg Arg His Glu Ala Leu Ala Asp
    130                 135                 140

Asn Ile Leu Phe Pro Gly Met Glu Trp Val Glu Asp Asp Ala Lys Phe
145                 150                 155                 160

Ala Glu Arg Leu Pro Val Met Ser Glu Gly Arg Asp Phe Ala Ala Glu
                165                 170                 175

Lys Val Ala Tyr Ser Gly Thr Asp Ile Gly Thr Asp Val Asn Phe Gly
            180                 185                 190

Ala Leu Thr Glu Gln Phe Leu Thr Ala Ala Glu Ala Ser Gly Thr Glu
        195                 200                 205

Ile Arg Tyr Gly His Glu Val Lys Asn Ile Lys Lys Asp Gly Gly Lys
    210                 215                 220

Trp Lys Ile Thr Val Lys Asn Val His Thr Gly Asp Thr Ser Val Val
225                 230                 235                 240

Lys Ala Lys Phe Val Phe Val Gly Ala Gly Gly Tyr Ala Leu Asp Leu
                245                 250                 255

Leu Arg Lys Ala Gly Val Pro Glu Val Ser Gly Tyr Ala Gly Phe Pro
            260                 265                 270

Val Ser Gly Leu Trp Leu Arg Cys Thr Asn Glu Glu Leu Ile Asn Gln
        275                 280                 285

His Asp Ala Lys Val Tyr Gly Lys Ala Lys Val Gly Ala Pro Pro Met
    290                 295                 300

Ser Val Pro His Leu Asp Thr Arg Val Ile Asp Gly Lys Lys Gly Leu
305                 310                 315                 320

Leu Phe Gly Pro Tyr Gly Gly Trp Ser Pro Lys Phe Leu Lys Glu Gly
                325                 330                 335

Ser Tyr Leu Asp Leu Phe Lys Ser Ile Arg Leu Asp Asn Leu Pro Ser
            340                 345                 350

Tyr Leu Gly Val Ala Ala Gln Glu Phe Ala Leu Thr Lys Tyr Leu Ile
        355                 360                 365

Thr Glu Val Ala Lys Asp Phe Glu Lys Arg Leu Val Asp Leu Arg Asp
    370                 375                 380

Tyr Val Pro Ser Ala Lys Ala Glu Asp Trp Glu Thr Val Val Ala Gly
385                 390                 395                 400

Gln Arg Val Gln Val Ile Lys Pro Ala Arg Ala Pro Arg Phe Gly Ser
                405                 410                 415

Leu Glu Phe Gly Thr Ala Leu Ile Asn Ser Thr Asp Gly Ser Ile Ala
            420                 425                 430

Gly Leu Leu Gly Ala Ser Pro Gly Ala Ser Ile Ala Pro Ala Ala Met
        435                 440                 445

Leu Glu Leu Val Glu Arg Cys Phe Gly Asp Arg Met Ile Gln Trp Gly
    450                 455                 460

Pro Lys Leu Lys Glu Met Ile Pro Ser Tyr Gly Thr Lys Leu Gly Asp
465                 470                 475                 480

Asp Glu Lys Met Phe Asn Glu Leu Trp Asp Tyr Thr Gln Lys Thr Leu
                485                 490                 495

Lys Leu Glu Lys
            500

<210>  21
<211>  3357
<212>  DNA
<213>  Corynebacterium humireducens

<220>
<221>  CDS
<222>  (301)..(3057)
<223>  gene aceE

<400>  21
gtgggagatg accctcgttg ccgccccttg tgggctggtt gtgacctcgg gggtgggagc      60

cggggtttgc aatctccgcc acacacctcg cggagaggcg ggacaggcct gcgacaaggg     120

gcgatgggac gtcgataagc ggaacgccgg acccgttcat cttcgcaagc tttacaggaa     180

tgcagaacta cgttggccaa tgaaaacacg tatcctggac tggacactcg ggcctgtccg     240

gaccaccttg ccggcgggcc cgttgctcca ccacgacacc cagaaacctg gaggcacatc     300

atg gca gat gac ctg aac gcg aac ggc cgc aag ggc aac tcc cac tac      348
Met Ala Asp Asp Leu Asn Ala Asn Gly Arg Lys Gly Asn Ser His Tyr
1               5                   10                  15

ccg gcg atc cgc gac ggc gtc gcg tcc tac ctc aaa gac acc gac ccg      396
Pro Ala Ile Arg Asp Gly Val Ala Ser Tyr Leu Lys Asp Thr Asp Pro
            20                  25                  30

gag gag acc cag gag tgg atg gac tcc ctc gac ggt ctc ctc gcg gag      444
Glu Glu Thr Gln Glu Trp Met Asp Ser Leu Asp Gly Leu Leu Ala Glu
        35                  40                  45

aag gac ccg gcc cgc gcc cgc tac ctc atg ctc cgc atg ctg gag cgc      492
Lys Asp Pro Ala Arg Ala Arg Tyr Leu Met Leu Arg Met Leu Glu Arg
    50                  55                  60

gcg gcc ctc aag cgt gtc ccg ctg ccg aac ctg acc tcc aca gac ttc 540
Ala Ala Leu Lys Arg Val Pro Leu Pro Asn Leu Thr Ser Thr Asp Phe
65                  70                  75                  80

gtc aac acg atc ccc acc acc atg gag ccg gag ttc ccg ggt gac gag 588
Val Asn Thr Ile Pro Thr Thr Met Glu Pro Glu Phe Pro Gly Asp Glu
                85                  90                  95

gag atg gag aag cgc tac cgc cgc tgg atg cgc tgg aac gcc gcc atc 636
Glu Met Glu Lys Arg Tyr Arg Arg Trp Met Arg Trp Asn Ala Ala Ile
            100                 105                 110

atg gtg cac cgc gcc cag cgc ccc ggc atc ggc gtc ggc ggc cac atc 684
Met Val His Arg Ala Gln Arg Pro Gly Ile Gly Val Gly Gly His Ile
        115                 120                 125

tcc acc tac gcc tcc gcc gcc ccg ctc tac gag gtc ggc ttc aat cac 732
Ser Thr Tyr Ala Ser Ala Ala Pro Leu Tyr Glu Val Gly Phe Asn His
    130                 135                 140

ttc tgg cgc ggc aag gac cac ccg ggc ggc ggc gac cag atc ttc ttc 780
Phe Trp Arg Gly Lys Asp His Pro Gly Gly Gly Asp Gln Ile Phe Phe
145                 150                 155                 160

cag ggc cac gcc tcc ccc ggc atc tac gcc cgc gcc ttc ctc gag ggg 828
Gln Gly His Ala Ser Pro Gly Ile Tyr Ala Arg Ala Phe Leu Glu Gly
                165                 170                 175

cgc ctg agc gag gac gac ctc gac ggc ttc cgc cag gag gtc tcc cgc 876
Arg Leu Ser Glu Asp Asp Leu Asp Gly Phe Arg Gln Glu Val Ser Arg
            180                 185                 190

ggt gag ggc aac ggc atc ccg tcc tac ccg cac ccg cac ggc atg cct 924
Gly Glu Gly Asn Gly Ile Pro Ser Tyr Pro His Pro His Gly Met Pro
        195                 200                 205

gat ttc tgg gag ttc ccg acc gtc tcc atg gga ctg ggc ccg atg gac 972
Asp Phe Trp Glu Phe Pro Thr Val Ser Met Gly Leu Gly Pro Met Asp
    210                 215                 220

gcg atc tac cag gca cgt ttc aac cgt tac ctg cac aac cgc ggc atc 1020
Ala Ile Tyr Gln Ala Arg Phe Asn Arg Tyr Leu His Asn Arg Gly Ile
225                 230                 235                 240

aag gac acc tcc gac cag cac gtg tgg gcc ttc ctc ggc gac ggc gag 1068
Lys Asp Thr Ser Asp Gln His Val Trp Ala Phe Leu Gly Asp Gly Glu
                245                 250                 255

atg gac gag ccg gag tcc cgc ggc ctc atc cag cac gcc gcg ctg aac 1116
Met Asp Glu Pro Glu Ser Arg Gly Leu Ile Gln His Ala Ala Leu Asn
            260                 265                 270

aac ctg gac aac ctc acc ttc gtg gtc aac tgc aac ctg cag cgt ctc 1164
Asn Leu Asp Asn Leu Thr Phe Val Val Asn Cys Asn Leu Gln Arg Leu
        275                 280                 285

gac ggc ccg gtc cgc ggc aac acg cag atc atc cag gag ctc gag tcc 1212
Asp Gly Pro Val Arg Gly Asn Thr Gln Ile Ile Gln Glu Leu Glu Ser
    290                 295                 300

ttc ttc cgg ggt gcc ggc tgg tcc gtc atc aag gtc atc tgg ggc cgc 1260
Phe Phe Arg Gly Ala Gly Trp Ser Val Ile Lys Val Ile Trp Gly Arg
305                 310                 315                 320

gag tgg gac gcc ctg ctg gag aag gac gag tcc ggc gcg ctg gtc aac     1308
Glu Trp Asp Ala Leu Leu Glu Lys Asp Glu Ser Gly Ala Leu Val Asn
                325                 330                 335

gtc atg aac acc acc ccg gac ggt gac tac cag acc ttc aag gcc aac     1356
Val Met Asn Thr Thr Pro Asp Gly Asp Tyr Gln Thr Phe Lys Ala Asn
            340                 345                 350

gac ggc gcc tac gtc cgc gag cac ttc ttc ggc cgc gac gag cgc acc     1404
Asp Gly Ala Tyr Val Arg Glu His Phe Phe Gly Arg Asp Glu Arg Thr
        355                 360                 365

gcc aaa ctc gtc gag gac atg acc gac gag gag atc tgg gcc ctg ccg     1452
Ala Lys Leu Val Glu Asp Met Thr Asp Glu Glu Ile Trp Ala Leu Pro
    370                 375                 380

cgc ggt ggc cac gac tac cgc aag gtc cac gcc gcc tac aag cgg gcg     1500
Arg Gly Gly His Asp Tyr Arg Lys Val His Ala Ala Tyr Lys Arg Ala
385                 390                 395                 400

ctg gag acc aag gac cgc ccg acc gtc atc ctc gcg ttc acc atc aag     1548
Leu Glu Thr Lys Asp Arg Pro Thr Val Ile Leu Ala Phe Thr Ile Lys
                405                 410                 415

ggc tac ggc ctg ggc cac aac ttc gag ggc cgc aac gcg acg cac cag     1596
Gly Tyr Gly Leu Gly His Asn Phe Glu Gly Arg Asn Ala Thr His Gln
            420                 425                 430

atg aag aag ctc gcc gcc gag gac ctg aag aac ttc cgc gac aag cag     1644
Met Lys Lys Leu Ala Ala Glu Asp Leu Lys Asn Phe Arg Asp Lys Gln
        435                 440                 445

tcc atc ccg atc ccg gac gag gtc ctc gag gcc gac ccg tac ctg ccg     1692
Ser Ile Pro Ile Pro Asp Glu Val Leu Glu Ala Asp Pro Tyr Leu Pro
    450                 455                 460

ccg tac tac cac ccg ggc gag gac gcc ccg gag atc cag tac atg aag     1740
Pro Tyr Tyr His Pro Gly Glu Asp Ala Pro Glu Ile Gln Tyr Met Lys
465                 470                 475                 480

aag cgt cgt cag gag ctg ggc ggt ttc atc ccg gag cgt cgc cag acc     1788
Lys Arg Arg Gln Glu Leu Gly Gly Phe Ile Pro Glu Arg Arg Gln Thr
                485                 490                 495

tac acc ccg ctg cag gtc ccg gac atc gag aag ctg cgc agc gtg cgc     1836
Tyr Thr Pro Leu Gln Val Pro Asp Ile Glu Lys Leu Arg Ser Val Arg
            500                 505                 510

aag ggc tcc ggc aag cag gag gtc gcc tcc acc atg gcg ctc gtg cgt     1884
Lys Gly Ser Gly Lys Gln Glu Val Ala Ser Thr Met Ala Leu Val Arg
        515                 520                 525

gcc ttc aag gag ctc atg cgt gac aag gag ctg ggc cgc cgc gtc gtg     1932
Ala Phe Lys Glu Leu Met Arg Asp Lys Glu Leu Gly Arg Arg Val Val
    530                 535                 540

ccg atc atc ccg gac gag gcc cgc acc ttc ggc ctc gac tcc tgg ttc     1980
Pro Ile Ile Pro Asp Glu Ala Arg Thr Phe Gly Leu Asp Ser Trp Phe
545                 550                 555                 560

ccg acc ctg aag atc tac aac ccg cac ggc cag aac tac gtg ccg gtc     2028
Pro Thr Leu Lys Ile Tyr Asn Pro His Gly Gln Asn Tyr Val Pro Val

565                 570                 575

gac cat gac ctc atg ctg tcc tac cgg gag gcc acc gac ggc cag atc        2076
Asp His Asp Leu Met Leu Ser Tyr Arg Glu Ala Thr Asp Gly Gln Ile
            580                 585                 590

ctc cac gag ggc atc aac gag gcc ggt tcc gtc gcg tcc ttc atg gcc        2124
Leu His Glu Gly Ile Asn Glu Ala Gly Ser Val Ala Ser Phe Met Ala
        595                 600                 605

gcc ggc acc tcc tac gcc acc cac ggc gaa gcg atg atc ccg ctg tac        2172
Ala Gly Thr Ser Tyr Ala Thr His Gly Glu Ala Met Ile Pro Leu Tyr
    610                 615                 620

atc ttc tac tcg atg ttc ggt ttc cag cgc acc ggc gac ggc atc tgg        2220
Ile Phe Tyr Ser Met Phe Gly Phe Gln Arg Thr Gly Asp Gly Ile Trp
625                 630                 635                 640

gcc gcc gcc gac cag atg gcc cgt ggc ttc ctc atc ggc gcc acc gcc        2268
Ala Ala Ala Asp Gln Met Ala Arg Gly Phe Leu Ile Gly Ala Thr Ala
                645                 650                 655

ggc cgc acc acc ctg acg ggt gag ggc ctg cag cac atg gac ggc cac        2316
Gly Arg Thr Thr Leu Thr Gly Glu Gly Leu Gln His Met Asp Gly His
            660                 665                 670

tcg cag atc ctc gcc tcc acg aac ccg ggc gtc gtg tcc tac gac ccg        2364
Ser Gln Ile Leu Ala Ser Thr Asn Pro Gly Val Val Ser Tyr Asp Pro
        675                 680                 685

gcg ttc gcc tac gag atc gcg cac ctg ctc cat gag ggc atc gac cgc        2412
Ala Phe Ala Tyr Glu Ile Ala His Leu Leu His Glu Gly Ile Asp Arg
    690                 695                 700

atg tac ggc ccg ggc cgc ggt gag aac gtc atc tac tac ctc acc gtg        2460
Met Tyr Gly Pro Gly Arg Gly Glu Asn Val Ile Tyr Tyr Leu Thr Val
705                 710                 715                 720

tac aac gag ccg gtc ccg cag ccg gcc gag ccg gag aac ctc gac gtc        2508
Tyr Asn Glu Pro Val Pro Gln Pro Ala Glu Pro Glu Asn Leu Asp Val
                725                 730                 735

gag ggc ctg cac aag ggc atg tac ctg ttc tcc cgc gcg gag gag ctc        2556
Glu Gly Leu His Lys Gly Met Tyr Leu Phe Ser Arg Ala Glu Glu Leu
            740                 745                 750

gag gtc gag gac ggc cac gag gcc tcc atc ctg gcc tcc ggc atc ggc        2604
Glu Val Glu Asp Gly His Glu Ala Ser Ile Leu Ala Ser Gly Ile Gly
        755                 760                 765

atg cag tgg gcg ctc aag gcc cag cag atg ctg gcc gag gag tac aac        2652
Met Gln Trp Ala Leu Lys Ala Gln Gln Met Leu Ala Glu Glu Tyr Asn
    770                 775                 780

gtg cgt gcc aac gtc ttc tcc gtc acc tcc tgg gtc gag ctg gcc cgc        2700
Val Arg Ala Asn Val Phe Ser Val Thr Ser Trp Val Glu Leu Ala Arg
785                 790                 795                 800

gag ggt gcg gcg aag aac aag gag cag ctg cgc aac ccg gcc gca gag        2748
Glu Gly Ala Ala Lys Asn Lys Glu Gln Leu Arg Asn Pro Ala Ala Glu
                805                 810                 815

atc gag gag gcc ttc gcc acc agg cag ctg aag cag gtc gac ggc ggc        2796

Ile Glu Glu Ala Phe Ala Thr Arg Gln Leu Lys Gln Val Asp Gly Gly
            820                 825                 830

acc ttc gtg gcc gtc tcc gac ttc gcg acc gac ctg cag gag cag atc      2844
Thr Phe Val Ala Val Ser Asp Phe Ala Thr Asp Leu Gln Glu Gln Ile
        835                 840                 845

cgc gcc tac gtc ccg ggc gag tac atc gtc ctg ggc gcc gac ggc ttc      2892
Arg Ala Tyr Val Pro Gly Glu Tyr Ile Val Leu Gly Ala Asp Gly Phe
    850                 855                 860

ggc ttc tcc gac acc cgt ccg gcc gcc cgc cgc ttc ttc aac atc gac      2940
Gly Phe Ser Asp Thr Arg Pro Ala Ala Arg Arg Phe Phe Asn Ile Asp
865                 870                 875                 880

gcc gag tcc atc gtc gtc gcg gtc ctc atg ggc ctg gcc cgc gag ggc      2988
Ala Glu Ser Ile Val Val Ala Val Leu Met Gly Leu Ala Arg Glu Gly
                885                 890                 895

aag atc gac atc tct gtg gcc cag cag gcc gcc gag aag tac cag ctg      3036
Lys Ile Asp Ile Ser Val Ala Gln Gln Ala Ala Glu Lys Tyr Gln Leu
            900                 905                 910

gac gat ccg acc gct gtg taa ctgatccccc ggtcaaggcc ctgctccggc      3087
Asp Asp Pro Thr Ala Val
        915

agggccttgc tgctttcccg cttgtcgacg cccctctcac tccctgttga ggtaccgcgg      3147

ccaggaggtg tcgcgggtgg ccagcgagat ctccaccgcg gtgtcgagct ccgctgccag      3207

gggctcgggc agcttgtgcc ggagcgcgtc gccgaggcgc gcgatgacct ccaggcgcag      3267

caggttgcgg gtctccgtga tgtagatgcg cgggtaccgg gtggccagct cgacctcgga      3327

ggggtcggcg ggcccgccgt acatctcgac                                      3357

<210>  22
<211>  918
<212>  PRT
<213>  Corynebacterium humireducens

<400>  22

Met Ala Asp Asp Leu Asn Ala Asn Gly Arg Lys Gly Asn Ser His Tyr
1               5                   10                  15

Pro Ala Ile Arg Asp Gly Val Ala Ser Tyr Leu Lys Asp Thr Asp Pro
            20                  25                  30

Glu Glu Thr Gln Glu Trp Met Asp Ser Leu Asp Gly Leu Leu Ala Glu
        35                  40                  45

Lys Asp Pro Ala Arg Ala Arg Tyr Leu Met Leu Arg Met Leu Glu Arg
    50                  55                  60

Ala Ala Leu Lys Arg Val Pro Leu Pro Asn Leu Thr Ser Thr Asp Phe
65                  70                  75                  80

Val Asn Thr Ile Pro Thr Thr Met Glu Pro Glu Phe Pro Gly Asp Glu
                85                  90                  95

Glu Met Glu Lys Arg Tyr Arg Arg Trp Met Arg Trp Asn Ala Ala Ile
            100                 105                 110

Met Val His Arg Ala Gln Arg Pro Gly Ile Gly Val Gly Gly His Ile
        115                 120                 125

Ser Thr Tyr Ala Ser Ala Ala Pro Leu Tyr Glu Val Gly Phe Asn His
    130                 135                 140

Phe Trp Arg Gly Lys Asp His Pro Gly Gly Gly Asp Gln Ile Phe Phe
145                 150                 155                 160

Gln Gly His Ala Ser Pro Gly Ile Tyr Ala Arg Ala Phe Leu Glu Gly
                165                 170                 175

Arg Leu Ser Glu Asp Asp Leu Asp Gly Phe Arg Gln Glu Val Ser Arg
            180                 185                 190

Gly Glu Gly Asn Gly Ile Pro Ser Tyr Pro His Pro His Gly Met Pro
        195                 200                 205

Asp Phe Trp Glu Phe Pro Thr Val Ser Met Gly Leu Gly Pro Met Asp
    210                 215                 220

Ala Ile Tyr Gln Ala Arg Phe Asn Arg Tyr Leu His Asn Arg Gly Ile
225                 230                 235                 240

Lys Asp Thr Ser Asp Gln His Val Trp Ala Phe Leu Gly Asp Gly Glu
                245                 250                 255

Met Asp Glu Pro Glu Ser Arg Gly Leu Ile Gln His Ala Ala Leu Asn
            260                 265                 270

Asn Leu Asp Asn Leu Thr Phe Val Val Asn Cys Asn Leu Gln Arg Leu
        275                 280                 285

Asp Gly Pro Val Arg Gly Asn Thr Gln Ile Ile Gln Glu Leu Glu Ser
    290                 295                 300

Phe Phe Arg Gly Ala Gly Trp Ser Val Ile Lys Val Ile Trp Gly Arg
305                 310                 315                 320

Glu Trp Asp Ala Leu Leu Glu Lys Asp Glu Ser Gly Ala Leu Val Asn

                325                 330                 335

Val Met Asn Thr Thr Pro Asp Gly Asp Tyr Gln Thr Phe Lys Ala Asn
            340                 345                 350

Asp Gly Ala Tyr Val Arg Glu His Phe Phe Gly Arg Asp Glu Arg Thr
        355                 360                 365

Ala Lys Leu Val Glu Asp Met Thr Asp Glu Glu Ile Trp Ala Leu Pro
    370                 375                 380

Arg Gly Gly His Asp Tyr Arg Lys Val His Ala Ala Tyr Lys Arg Ala
385                 390                 395                 400

Leu Glu Thr Lys Asp Arg Pro Thr Val Ile Leu Ala Phe Thr Ile Lys
                405                 410                 415

Gly Tyr Gly Leu Gly His Asn Phe Glu Gly Arg Asn Ala Thr His Gln
            420                 425                 430

Met Lys Lys Leu Ala Ala Glu Asp Leu Lys Asn Phe Arg Asp Lys Gln
        435                 440                 445

Ser Ile Pro Ile Pro Asp Glu Val Leu Glu Ala Asp Pro Tyr Leu Pro
    450                 455                 460

Pro Tyr Tyr His Pro Gly Glu Asp Ala Pro Glu Ile Gln Tyr Met Lys
465                 470                 475                 480

Lys Arg Arg Gln Glu Leu Gly Gly Phe Ile Pro Glu Arg Arg Gln Thr
                485                 490                 495

Tyr Thr Pro Leu Gln Val Pro Asp Ile Glu Lys Leu Arg Ser Val Arg
            500                 505                 510

Lys Gly Ser Gly Lys Gln Glu Val Ala Ser Thr Met Ala Leu Val Arg
        515                 520                 525

Ala Phe Lys Glu Leu Met Arg Asp Lys Glu Leu Gly Arg Arg Val Val
    530                 535                 540

Pro Ile Ile Pro Asp Glu Ala Arg Thr Phe Gly Leu Asp Ser Trp Phe
545                 550                 555                 560

Pro Thr Leu Lys Ile Tyr Asn Pro His Gly Gln Asn Tyr Val Pro Val
                565                 570                 575

Asp His Asp Leu Met Leu Ser Tyr Arg Glu Ala Thr Asp Gly Gln Ile
            580                 585                 590

Leu His Glu Gly Ile Asn Glu Ala Gly Ser Val Ala Ser Phe Met Ala
        595                 600                 605

Ala Gly Thr Ser Tyr Ala Thr His Gly Glu Ala Met Ile Pro Leu Tyr
    610                 615                 620

Ile Phe Tyr Ser Met Phe Gly Phe Gln Arg Thr Gly Asp Gly Ile Trp
625                 630                 635                 640

Ala Ala Ala Asp Gln Met Ala Arg Gly Phe Leu Ile Gly Ala Thr Ala
                645                 650                 655

Gly Arg Thr Thr Leu Thr Gly Glu Gly Leu Gln His Met Asp Gly His
            660                 665                 670

Ser Gln Ile Leu Ala Ser Thr Asn Pro Gly Val Val Ser Tyr Asp Pro
        675                 680                 685

Ala Phe Ala Tyr Glu Ile Ala His Leu Leu His Glu Gly Ile Asp Arg
    690                 695                 700

Met Tyr Gly Pro Gly Arg Gly Glu Asn Val Ile Tyr Tyr Leu Thr Val
705                 710                 715                 720

Tyr Asn Glu Pro Val Pro Gln Pro Ala Glu Pro Glu Asn Leu Asp Val
                725                 730                 735

Glu Gly Leu His Lys Gly Met Tyr Leu Phe Ser Arg Ala Glu Glu Leu
            740                 745                 750

Glu Val Glu Asp Gly His Glu Ala Ser Ile Leu Ala Ser Gly Ile Gly
        755                 760                 765

Met Gln Trp Ala Leu Lys Ala Gln Gln Met Leu Ala Glu Glu Tyr Asn
    770                 775                 780

Val Arg Ala Asn Val Phe Ser Val Thr Ser Trp Val Glu Leu Ala Arg
785                 790                 795                 800

Glu Gly Ala Ala Lys Asn Lys Glu Gln Leu Arg Asn Pro Ala Ala Glu
                805                 810                 815

Ile Glu Glu Ala Phe Ala Thr Arg Gln Leu Lys Gln Val Asp Gly Gly
            820                 825                 830

Thr Phe Val Ala Val Ser Asp Phe Ala Thr Asp Leu Gln Glu Gln Ile
        835                 840                 845

Arg Ala Tyr Val Pro Gly Glu Tyr Ile Val Leu Gly Ala Asp Gly Phe
    850                 855                 860

Gly Phe Ser Asp Thr Arg Pro Ala Ala Arg Arg Phe Phe Asn Ile Asp
865                 870                 875                 880

Ala Glu Ser Ile Val Val Ala Val Leu Met Gly Leu Ala Arg Glu Gly
                885                 890                 895

Lys Ile Asp Ile Ser Val Ala Gln Gln Ala Ala Glu Lys Tyr Gln Leu
            900                 905                 910

Asp Asp Pro Thr Ala Val
        915

<210>  23
<211>  1752
<212>  DNA
<213>  Corynebacterium humireducens

<220>
<221>  CDS
<222>  (301)..(1452)
<223>  gene gltA

<400>  23
agctgctctc cgcccagcag ctcgaggagc tcggcttcaa cgccgtcatc tacccggtga      60

ccaccctgcg catcgccatg ggacaggtcg aggaggctct cgcggagatc gccgccaccg     120

gcacccagac cgggtgggtc gaccgcatgc agcaccgctc ccgcctctac gaactgctcc     180

gctacaacga gtacaacgcc ttcgaccagg aggttttcac gtactccgcc gacacctacc     240

agcccacctt ccagtagccc accagcaact cctgacattc cttgaaaggg gatgagtacc     300

atg tcc gac aac acc aac atc gag gtc cgc aag ggt ctc tac ggc gtg       348
Met Ser Asp Asn Thr Asn Ile Glu Val Arg Lys Gly Leu Tyr Gly Val
1               5                   10                  15

atc gcc gac tac acc gca gtc tcc aag gtc atg ccg gag acc aac tcc       396
Ile Ala Asp Tyr Thr Ala Val Ser Lys Val Met Pro Glu Thr Asn Ser
            20                  25                  30

ctg acc tac cgc gga tac gcc gtc cag gac ctc gtc gca gag tgc tcc       444
Leu Thr Tyr Arg Gly Tyr Ala Val Gln Asp Leu Val Ala Glu Cys Ser
        35                  40                  45

ttc gag gag gtc ttc tac ctc ctg tgg aac ggt gag ctg ccg aac gag       492
Phe Glu Glu Val Phe Tyr Leu Leu Trp Asn Gly Glu Leu Pro Asn Glu
    50                  55                  60

gag cag ctg gcg gag ttc aac agg cgc ggc cgt tcc tac cgc aag ctc 540
Glu Gln Leu Ala Glu Phe Asn Arg Arg Gly Arg Ser Tyr Arg Lys Leu
65                  70                  75                  80

gac gcc ggc ctg atc gca ctc atc cac tcc ctg ccg ctc gac tgt cac 588
Asp Ala Gly Leu Ile Ala Leu Ile His Ser Leu Pro Leu Asp Cys His
                85                  90                  95

ccg atg gac gtc atg cgc acc gcc gtg tcc tac atg ggc acc aag gac 636
Pro Met Asp Val Met Arg Thr Ala Val Ser Tyr Met Gly Thr Lys Asp
            100                 105                 110

tcc gag cac ttc acc ccg gac gcc gac cac atc acc cac gtc ggc cac 684
Ser Glu His Phe Thr Pro Asp Ala Asp His Ile Thr His Val Gly His
        115                 120                 125

aac ctc ctc gcc cag ctg ccg atg gtg ctg gcc atg gac atc cgc cgc 732
Asn Leu Leu Ala Gln Leu Pro Met Val Leu Ala Met Asp Ile Arg Arg
    130                 135                 140

cgt cag ggc ctc gac atc gtc gcc ccg gac ccg gac aag tcc gtc gcc 780
Arg Gln Gly Leu Asp Ile Val Ala Pro Asp Pro Asp Lys Ser Val Ala
145                 150                 155                 160

cac aac ctg ctg tcg atg gtc ttc ggc aac ggc ccg gag tcc ccg gcg 828
His Asn Leu Leu Ser Met Val Phe Gly Asn Gly Pro Glu Ser Pro Ala
                165                 170                 175

tcc aac ccg gac gac gtg cgt gac ttc gag aag tcg ctc atc ctc tac 876
Ser Asn Pro Asp Asp Val Arg Asp Phe Glu Lys Ser Leu Ile Leu Tyr
            180                 185                 190

gcc gag cac tcc ttc aac gcc tcg acc ttc acc tcg cgt gtg atc acc 924
Ala Glu His Ser Phe Asn Ala Ser Thr Phe Thr Ser Arg Val Ile Thr
        195                 200                 205

tcg acc cgc tcg gac gtc tac tcc gcg atc acc ggt gcc atc ggc gcc 972
Ser Thr Arg Ser Asp Val Tyr Ser Ala Ile Thr Gly Ala Ile Gly Ala
    210                 215                 220

ctc aag ggc ccg ctg cac ggc ggc gcc aac gag ttc gtc atg cac acc 1020
Leu Lys Gly Pro Leu His Gly Gly Ala Asn Glu Phe Val Met His Thr
225                 230                 235                 240

atg ctg gag atc gac gac ccg gag aag gcc gcc gac tgg gtc aac aac 1068
Met Leu Glu Ile Asp Asp Pro Glu Lys Ala Ala Asp Trp Val Asn Asn
                245                 250                 255

gcc ctg gac aac aag gac ctc atc atg ggc ttc ggt cac cgc gtg tac 1116
Ala Leu Asp Asn Lys Asp Leu Ile Met Gly Phe Gly His Arg Val Tyr
            260                 265                 270

aag aag ggt gac tcg cgt gtc ccg tcc atg gag gcg tcc ttc cgt gac 1164
Lys Lys Gly Asp Ser Arg Val Pro Ser Met Glu Ala Ser Phe Arg Asp
        275                 280                 285

ctc gcc gag cgt cac gac ggc gcc aag tgg gtc gcc atg tac gag aac 1212
Leu Ala Glu Arg His Asp Gly Ala Lys Trp Val Ala Met Tyr Glu Asn
    290                 295                 300

atg cgc gac gcc atg gac gcc cgc acc ggc atc aag ccg aac ctg gac 1260
Met Arg Asp Ala Met Asp Ala Arg Thr Gly Ile Lys Pro Asn Leu Asp
305                 310                 315                 320

ttc ccg gcc ggc ccg gcg tac cac ctg ctc ggc ttc ccg gtc gac ttc 1308
Phe Pro Ala Gly Pro Ala Tyr His Leu Leu Gly Phe Pro Val Asp Phe
                325                 330                 335

ttc acc ccg ctg ttc gtc gtc gcc cgc atc gcc ggc tgg acc gcc cac 1356
Phe Thr Pro Leu Phe Val Val Ala Arg Ile Ala Gly Trp Thr Ala His
            340                 345                 350

atc gtg gag cag tac aac gac aac tcc ctg att cgt ccg ctc tcc gcc 1404
Ile Val Glu Gln Tyr Asn Asp Asn Ser Leu Ile Arg Pro Leu Ser Ala
        355                 360                 365

tac aac ggc ccc gag cag cgc cag gtc acc ccg ctg gcc gcc cgt tag 1452
Tyr Asn Gly Pro Glu Gln Arg Gln Val Thr Pro Leu Ala Ala Arg
    370                 375                 380

ccccacggcc ggcaccggac caccgcgacg cccacggcac tgcccacaca ttggggtggt 1512

gcgcgtgggt ttcgctgttc ggaacacctg tcggcccatt ccgcagggcg ggacagaaac 1572

ggcctttcgg gcgcccgtcc cgcggagtgc ggaaatggac cgcaccgggt ccaccacctg 1632

cagatacccc ctgtgactag gggatactcc cccgaagaca accctctttc ttgcgatact 1692

gtggtgtgcc agagagctca cccgctctca gacacacccc cgacatgacg ttcggaaagg 1752

<210> 24
<211> 383
<212> PRT
<213> Corynebacterium humireducens

<400> 24

Met Ser Asp Asn Thr Asn Ile Glu Val Arg Lys Gly Leu Tyr Gly Val
1               5                   10                  15

Ile Ala Asp Tyr Thr Ala Val Ser Lys Val Met Pro Glu Thr Asn Ser
            20                  25                  30

Leu Thr Tyr Arg Gly Tyr Ala Val Gln Asp Leu Val Ala Glu Cys Ser
        35                  40                  45

Phe Glu Glu Val Phe Tyr Leu Leu Trp Asn Gly Glu Leu Pro Asn Glu
    50                  55                  60

Glu Gln Leu Ala Glu Phe Asn Arg Arg Gly Arg Ser Tyr Arg Lys Leu
65                  70                  75                  80

Asp Ala Gly Leu Ile Ala Leu Ile His Ser Leu Pro Leu Asp Cys His
                85                  90                  95

Pro Met Asp Val Met Arg Thr Ala Val Ser Tyr Met Gly Thr Lys Asp
            100                 105                 110

Ser Glu His Phe Thr Pro Asp Ala Asp His Ile Thr His Val Gly His
        115                 120                 125

Asn Leu Leu Ala Gln Leu Pro Met Val Leu Ala Met Asp Ile Arg Arg
    130                 135                 140

Arg Gln Gly Leu Asp Ile Val Ala Pro Asp Pro Asp Lys Ser Val Ala
145                 150                 155                 160

His Asn Leu Leu Ser Met Val Phe Gly Asn Gly Pro Glu Ser Pro Ala
                165                 170                 175

Ser Asn Pro Asp Asp Val Arg Asp Phe Glu Lys Ser Leu Ile Leu Tyr
            180                 185                 190

Ala Glu His Ser Phe Asn Ala Ser Thr Phe Thr Ser Arg Val Ile Thr
        195                 200                 205

Ser Thr Arg Ser Asp Val Tyr Ser Ala Ile Thr Gly Ala Ile Gly Ala
    210                 215                 220

Leu Lys Gly Pro Leu His Gly Gly Ala Asn Glu Phe Val Met His Thr
225                 230                 235                 240

Met Leu Glu Ile Asp Asp Pro Glu Lys Ala Ala Asp Trp Val Asn Asn
                245                 250                 255

Ala Leu Asp Asn Lys Asp Leu Ile Met Gly Phe Gly His Arg Val Tyr
            260                 265                 270

Lys Lys Gly Asp Ser Arg Val Pro Ser Met Glu Ala Ser Phe Arg Asp
        275                 280                 285

Leu Ala Glu Arg His Asp Gly Ala Lys Trp Val Ala Met Tyr Glu Asn
    290                 295                 300

Met Arg Asp Ala Met Asp Ala Arg Thr Gly Ile Lys Pro Asn Leu Asp
305                 310                 315                 320

Phe Pro Ala Gly Pro Ala Tyr His Leu Leu Gly Phe Pro Val Asp Phe
                325                 330                 335

Phe Thr Pro Leu Phe Val Val Ala Arg Ile Ala Gly Trp Thr Ala His
            340                 345                 350

Ile Val Glu Gln Tyr Asn Asp Asn Ser Leu Ile Arg Pro Leu Ser Ala
        355                 360                 365

Tyr Asn Gly Pro Glu Gln Arg Gln Val Thr Pro Leu Ala Ala Arg
    370                 375                 380

<210>  25
<211>  1578
<212>  DNA
<213>  Corynebacterium humireducens

<220>
<221>  CDS
<222>  (301)..(1278)
<223>  gene mdh

<400>  25
gaacatgcgg ggtcgtccct ccgggggcac gggtcggggt ggaagggtct ggaagggggc      60

ggtgccggcg ggtctgtgcg ggccgcaacc ggcgctgtga tccatcgtgc ctgatgtatc     120

ggtcggtggt ggggcggtgc ggctgagaag atgctggacc acccgatgta gcgacgttct     180

tccagcggaa acagccggat ctccggcgag gttacgccca tccgggggtg gcggctcgga     240

acggtctttg aggctagact cgcaggcgtt gatgcattag accgctcgaa aggatcccgc     300

atg acc cag aac ccc gtg aag atc gcc gtc acc ggt gcc gcc ggc aac       348
Met Thr Gln Asn Pro Val Lys Ile Ala Val Thr Gly Ala Ala Gly Asn
1               5                   10                  15

atc gcc tac tcc ctc ctg tgg cgc atc gcc gca ggt gac gtc tac ggc       396
Ile Ala Tyr Ser Leu Leu Trp Arg Ile Ala Ala Gly Asp Val Tyr Gly
            20                  25                  30

cgc gac acc ccg atc gag ctc acc ctc ctc gag atc ccg gcc gcc gta       444
Arg Asp Thr Pro Ile Glu Leu Thr Leu Leu Glu Ile Pro Ala Ala Val
        35                  40                  45

cgc ggc gcc gag ggc gtg gcc atg gag ctc aac gac tcc gcc ttc ccc       492
Arg Gly Ala Glu Gly Val Ala Met Glu Leu Asn Asp Ser Ala Phe Pro
    50                  55                  60

ctc ctg cgc aac atc acc gtc acc gac agc gcc gac gtc gcc ttc gac       540
Leu Leu Arg Asn Ile Thr Val Thr Asp Ser Ala Asp Val Ala Phe Asp
65                  70                  75                  80

ggc gcc aac gcc gcc ttc ctt gtc ggc gcg aag ccg cgc ggt gca ggc       588
Gly Ala Asn Ala Ala Phe Leu Val Gly Ala Lys Pro Arg Gly Ala Gly
                85                  90                  95

gag gag cgc gcc gac ctg ctg aag aac aac ggc ggc atc ttc ggc ccg       636
Glu Glu Arg Ala Asp Leu Leu Lys Asn Asn Gly Gly Ile Phe Gly Pro
            100                 105                 110

cag ggc agg gcc atc aac gac cgc gcc gcg gac gac gtc cgc gtg ctc       684
Gln Gly Arg Ala Ile Asn Asp Arg Ala Ala Asp Asp Val Arg Val Leu
        115                 120                 125

gtc gtc ggc aac ccg gcc aac acc aac gcc ctg atc gcc cag cgc aac       732
Val Val Gly Asn Pro Ala Asn Thr Asn Ala Leu Ile Ala Gln Arg Asn
    130                 135                 140

gcc ccg gac gtc ccg gcc gag cgt ttc acc gcg ctg atg cgc ctg gac 780
Ala Pro Asp Val Pro Ala Glu Arg Phe Thr Ala Leu Met Arg Leu Asp
145                 150                 155                 160

cac agc cgt gcg ctg tcc cag ctg gcc gag aag ctc ggc gtg ctc acc 828
His Ser Arg Ala Leu Ser Gln Leu Ala Glu Lys Leu Gly Val Leu Thr
                165                 170                 175

acc gac ttc gag aac gtc gtc gtg tgg ggc aac cac tcc gcg acc cag 876
Thr Asp Phe Glu Asn Val Val Val Trp Gly Asn His Ser Ala Thr Gln
            180                 185                 190

ttc ccg gac gtg acc ttc gcc acc gtc aag ggt gag ccg gtc atc gag 924
Phe Pro Asp Val Thr Phe Ala Thr Val Lys Gly Glu Pro Val Ile Glu
        195                 200                 205

aag atc gac cat gac tgg tac gtc aac gag ttc atc ccg cgc gtc gcg 972
Lys Ile Asp His Asp Trp Tyr Val Asn Glu Phe Ile Pro Arg Val Ala
    210                 215                 220

aag cgt ggc gcc gag atc atc gag gtc cgc ggc aag tcc tcc gcc gcc 1020
Lys Arg Gly Ala Glu Ile Ile Glu Val Arg Gly Lys Ser Ser Ala Ala
225                 230                 235                 240

tcc gcc gcc tcc gcc gcg gtg gac cac atg cgt gac tgg gtc cag ggc 1068
Ser Ala Ala Ser Ala Ala Val Asp His Met Arg Asp Trp Val Gln Gly
                245                 250                 255

act gag cgg tgg gcc tcc gcc gcc ctg ccg tcc gac ggc tcc tac ggc 1116
Thr Glu Arg Trp Ala Ser Ala Ala Leu Pro Ser Asp Gly Ser Tyr Gly
            260                 265                 270

atc gac gag ggt ctc gtc gcc ggc ctg ccg acc gtc tcc cgc aac ggc 1164
Ile Asp Glu Gly Leu Val Ala Gly Leu Pro Thr Val Ser Arg Asn Gly
        275                 280                 285

tcc tgg gag atc gtc cag ggc ctc gag ctc aac gac ttc cag cgc gag 1212
Ser Trp Glu Ile Val Gln Gly Leu Glu Leu Asn Asp Phe Gln Arg Glu
    290                 295                 300

atg atc gcc cgg aac gtc gcg gag ctc aag gcc gag cgt gac gca gtc 1260
Met Ile Ala Arg Asn Val Ala Glu Leu Lys Ala Glu Arg Asp Ala Val
305                 310                 315                 320

gct gac ctg ctt cct tag cgcgtcaact cgcatccgcc gccaatcccg 1308
Ala Asp Leu Leu Pro
                325

gtagtgttca ggaacatgcg cttcgggatt gatttcggaa ccacccgtac cgtcattgcg 1368

gcggtcgacc ggggaaacta cccggtcgtc aacgtcacgg acacggacgg cgactcacgg 1428

gagtacatcc cctcggtcgt cgcgctggat ggtgaacggt tggtcggtgg ctgggaggcg 1488

ctggcggtag gtggcccgac cctcacccgc tccttcaagc gtctgctgac ctcccgcacc 1548

gtcaccgcgg agacccccgt ccggctgggc 1578

<210> 26
<211> 325
<212> PRT
<213> Corynebacterium humireducens

<400>  26

Met Thr Gln Asn Pro Val Lys Ile Ala Val Thr Gly Ala Ala Gly Asn
1               5                   10                  15

Ile Ala Tyr Ser Leu Leu Trp Arg Ile Ala Ala Gly Asp Val Tyr Gly
            20                  25                  30

Arg Asp Thr Pro Ile Glu Leu Thr Leu Leu Glu Ile Pro Ala Ala Val
        35                  40                  45

Arg Gly Ala Glu Gly Val Ala Met Glu Leu Asn Asp Ser Ala Phe Pro
    50                  55                  60

Leu Leu Arg Asn Ile Thr Val Thr Asp Ser Ala Asp Val Ala Phe Asp
65                  70                  75                  80

Gly Ala Asn Ala Ala Phe Leu Val Gly Ala Lys Pro Arg Gly Ala Gly
                85                  90                  95

Glu Glu Arg Ala Asp Leu Leu Lys Asn Asn Gly Gly Ile Phe Gly Pro
            100                 105                 110

Gln Gly Arg Ala Ile Asn Asp Arg Ala Ala Asp Asp Val Arg Val Leu
        115                 120                 125

Val Val Gly Asn Pro Ala Asn Thr Asn Ala Leu Ile Ala Gln Arg Asn
    130                 135                 140

Ala Pro Asp Val Pro Ala Glu Arg Phe Thr Ala Leu Met Arg Leu Asp
145                 150                 155                 160

His Ser Arg Ala Leu Ser Gln Leu Ala Glu Lys Leu Gly Val Leu Thr
                165                 170                 175

Thr Asp Phe Glu Asn Val Val Val Trp Gly Asn His Ser Ala Thr Gln
            180                 185                 190

Phe Pro Asp Val Thr Phe Ala Thr Val Lys Gly Glu Pro Val Ile Glu
        195                 200                 205

Lys Ile Asp His Asp Trp Tyr Val Asn Glu Phe Ile Pro Arg Val Ala
    210                 215                 220

Lys Arg Gly Ala Glu Ile Ile Glu Val Arg Gly Lys Ser Ser Ala Ala
225                 230                 235                 240

Ser Ala Ala Ser Ala Ala Val Asp His Met Arg Asp Trp Val Gln Gly
                245                 250                 255

Thr Glu Arg Trp Ala Ser Ala Ala Leu Pro Ser Asp Gly Ser Tyr Gly
            260                 265                 270

Ile Asp Glu Gly Leu Val Ala Gly Leu Pro Thr Val Ser Arg Asn Gly
        275                 280                 285

Ser Trp Glu Ile Val Gln Gly Leu Glu Leu Asn Asp Phe Gln Arg Glu
    290                 295                 300

Met Ile Ala Arg Asn Val Ala Glu Leu Lys Ala Glu Arg Asp Ala Val
305                 310                 315                 320

Ala Asp Leu Leu Pro
                325

<210>  27
<211>  2100
<212>  DNA
<213>  Corynebacterium humireducens

<220>
<221>  CDS
<222>  (301)..(1800)
<223>  gene murE

<400>  27
gccggcaaga cgggtacggc ccagaaggtc gacccgcgga cgggcgcgta ctcgaactcg        60

gactactgga tcaccttcgc cggcatcgcc ccggcggatg acccgcgctt cgtcgtcgcc       120

gtcatggtcg acgagccccg cgccggcgtc gaggcgggag gcggcggtgg ccagtcctcg       180

gcgccgttgt tccgtgacat cgcgtcctgg ctgctcaacc gggacaacat cccggcgtca       240

ccgcccatgg aggacatgct gatcctccac gcccactaac ccagcccacc acggaggagt       300

atg acc atg gag acc aca gtg acc aca ctg tcc cac ctc gcc cgt atc       348
Met Thr Met Glu Thr Thr Val Thr Thr Leu Ser His Leu Ala Arg Ile
1               5                   10                  15

gcc ggg gga gag ctg acc gga tcc gtg gcc ccg gtc gtc acg tcg gtg       396
Ala Gly Gly Glu Leu Thr Gly Ser Val Ala Pro Val Val Thr Ser Val
            20                  25                  30

gag ctc gac tcc tcc cgc ctc gcg gac gag ggc gcg ctc ttc gcc gcc       444
Glu Leu Asp Ser Ser Arg Leu Ala Asp Glu Gly Ala Leu Phe Ala Ala
        35                  40                  45

gtc ccc ggc acg cgc cgc cac ggt gcg gag ttc gcg cgc ggc acg ccg       492
Val Pro Gly Thr Arg Arg His Gly Ala Glu Phe Ala Arg Gly Thr Pro
    50                  55                  60

gcc gtc gcg atc ctc acc gac gcc gcc gga aag gag ctt ctc gac gcc       540

Ala Val Ala Ile Leu Thr Asp Ala Ala Gly Lys Glu Leu Leu Asp Ala
65                  70                  75                  80

gcc ggc gac acc cgc cct gtc atc ctc gtc gat gac gtc cgc gcg gtc      588
Ala Gly Asp Thr Arg Pro Val Ile Leu Val Asp Asp Val Arg Ala Val
                85                  90                  95

ctc ggg gcc gtg gcc gcg gag atc cac ggc cac ccc tcg gag cgt ctc      636
Leu Gly Ala Val Ala Ala Glu Ile His Gly His Pro Ser Glu Arg Leu
            100                 105                 110

acc ctg ctc ggc atc acc ggc acc tcc ggg aag acg acc acc agc tac      684
Thr Leu Leu Gly Ile Thr Gly Thr Ser Gly Lys Thr Thr Thr Ser Tyr
        115                 120                 125

ctg ctc gag cag ggg ctc atg gcg gcc gac ctg aag gtc ggc ctc atc      732
Leu Leu Glu Gln Gly Leu Met Ala Ala Asp Leu Lys Val Gly Leu Ile
    130                 135                 140

ggc acg acg ggc acg cgc atc aac ggc cgt ccc gtc ccc acc gac ctc      780
Gly Thr Thr Gly Thr Arg Ile Asn Gly Arg Pro Val Pro Thr Asp Leu
145                 150                 155                 160

acg acc ccg gag gcc acg acc ctg cag ccc ctg ttc gtg cgg atg ctc      828
Thr Thr Pro Glu Ala Thr Thr Leu Gln Pro Leu Phe Val Arg Met Leu
                165                 170                 175

gcg gag ggc gtc acg cac gtg gtc atg gag gtc tcc tcc cac gcc ctg      876
Ala Glu Gly Val Thr His Val Val Met Glu Val Ser Ser His Ala Leu
            180                 185                 190

gag ctg ggc cgg gtg gcg ggc acg cgt ttc gac gtc gcc ggg ttc acc      924
Glu Leu Gly Arg Val Ala Gly Thr Arg Phe Asp Val Ala Gly Phe Thr
        195                 200                 205

aac ctc tcc cag gac cac ctc gac ttc cac ccg acg atg gag gag tac      972
Asn Leu Ser Gln Asp His Leu Asp Phe His Pro Thr Met Glu Glu Tyr
    210                 215                 220

ttc gcg gcc aag gcc cgc ttc ttc gac ccg gcg tcg ccg ctg gcc gcg      1020
Phe Ala Ala Lys Ala Arg Phe Phe Asp Pro Ala Ser Pro Leu Ala Ala
225                 230                 235                 240

gag cgg tcc gtc gtc tgc gtc gac gac gag tgg ggg cgg cgg atg gcc      1068
Glu Arg Ser Val Val Cys Val Asp Asp Glu Trp Gly Arg Arg Met Ala
                245                 250                 255

gac ctg gcc tcg gac ccg gtg acc gtc gcg acc cgc ggc gcg gag gcc      1116
Asp Leu Ala Ser Asp Pro Val Thr Val Ala Thr Arg Gly Ala Glu Ala
            260                 265                 270

gac gtc acc gcc gtc cag agc tcg ctc acg ccc acc ggc gcc cag gtc      1164
Asp Val Thr Ala Val Gln Ser Ser Leu Thr Pro Thr Gly Ala Gln Val
        275                 280                 285

atc cgg ctc acc ctg ccg gag ggg gcc gtc gag gcg gag ctg ccg ctg      1212
Ile Arg Leu Thr Leu Pro Glu Gly Ala Val Glu Ala Glu Leu Pro Leu
    290                 295                 300

ccg ggt gac ttc aac atc gcc aac gcc gcc ctg gcc acc gcc atg gcg      1260
Pro Gly Asp Phe Asn Ile Ala Asn Ala Ala Leu Ala Thr Ala Met Ala
305                 310                 315                 320

tgg gcc gtg ggc gtc gac ccg cag gcc ttc acc gcc ggt ttc ctc gac     1308
Trp Ala Val Gly Val Asp Pro Gln Ala Phe Thr Ala Gly Phe Leu Asp
                325                 330                 335

gtc gcg gtc ccc ggg cgc atg gag cgt atc gac gag ggc cag gac ttc     1356
Val Ala Val Pro Gly Arg Met Glu Arg Ile Asp Glu Gly Gln Asp Phe
            340                 345                 350

ctc gcc gtc gtc gac tac gcc cac aag ccg gcc gcc gtc gcc gcc gtc     1404
Leu Ala Val Val Asp Tyr Ala His Lys Pro Ala Ala Val Ala Ala Val
        355                 360                 365

ctc gac acc ctc cgc ggg cag gtc cgc ggc cgc ctc ggc gtg gtc gtc     1452
Leu Asp Thr Leu Arg Gly Gln Val Arg Gly Arg Leu Gly Val Val Val
    370                 375                 380

ggg gcc ggt ggt gac cgc gac gcc acc aag cgt ccc ctc atg ggg gcg     1500
Gly Ala Gly Gly Asp Arg Asp Ala Thr Lys Arg Pro Leu Met Gly Ala
385                 390                 395                 400

gag gcc gcc cac cgc gcc gac ctg gtc atc atc acc gac gac aac ccg     1548
Glu Ala Ala His Arg Ala Asp Leu Val Ile Ile Thr Asp Asp Asn Pro
                405                 410                 415

cgc agc gag gtg ccc gcc acc atc cgg gcg gcc gtg ctg cag ggg gcg     1596
Arg Ser Glu Val Pro Ala Thr Ile Arg Ala Ala Val Leu Gln Gly Ala
            420                 425                 430

gtc gac gcc ggg acg gac gcg gag atc cgc gag atc ggg gac cgc gcc     1644
Val Asp Ala Gly Thr Asp Ala Glu Ile Arg Glu Ile Gly Asp Arg Ala
        435                 440                 445

gcc gcc atc gac gcg ctg atc gag tgg gcc cgc ccg ggg gac ggc gtc     1692
Ala Ala Ile Asp Ala Leu Ile Glu Trp Ala Arg Pro Gly Asp Gly Val
    450                 455                 460

gtc gtc gcc ggc aag ggc cac gag gtc ggc cag ctg gtc aac ggc gtc     1740
Val Val Ala Gly Lys Gly His Glu Val Gly Gln Leu Val Asn Gly Val
465                 470                 475                 480

aac cac cat ttc gac gac cgg gaa gag gtc cgg cgc gca ctc aag gag     1788
Asn His His Phe Asp Asp Arg Glu Glu Val Arg Arg Ala Leu Lys Glu
                485                 490                 495

aag aag cat tga tcccctcac tctcgcggag atcgcggaca tcaccggcgg     1840
Lys Lys His

tgaactctcg cacgtgaccg acccggagac ccgggtgacc gggtccgtcg agttcgactc     1900

ccgccgggtc accccgggcg ggctgttcct cgccctgccg ggggcgcgcg tcgacggaca     1960

cgacttcgcc gccaccgccg tcgagtccgg cgcggtcgcc gtcctggcgg cgcggccggt     2020

gggggtgccg gcggtcgtcg tgaagccgcg gggccgcagt gactccaacg ccgacatcta     2080

cgcccacgac gaggacggct     2100

<210>  28
<211>  499
<212>  PRT
<213>  Corynebacterium humireducens

<400>  28

Met Thr Met Glu Thr Thr Val Thr Thr Leu Ser His Leu Ala Arg Ile
1               5                   10                  15

Ala Gly Gly Glu Leu Thr Gly Ser Val Ala Pro Val Val Thr Ser Val
            20                  25                  30

Glu Leu Asp Ser Ser Arg Leu Ala Asp Glu Gly Ala Leu Phe Ala Ala
        35                  40                  45

Val Pro Gly Thr Arg Arg His Gly Ala Glu Phe Ala Arg Gly Thr Pro
    50                  55                  60

Ala Val Ala Ile Leu Thr Asp Ala Ala Gly Lys Glu Leu Leu Asp Ala
65                  70                  75                  80

Ala Gly Asp Thr Arg Pro Val Ile Leu Val Asp Asp Val Arg Ala Val
                85                  90                  95

Leu Gly Ala Val Ala Ala Glu Ile His Gly His Pro Ser Glu Arg Leu
            100                 105                 110

Thr Leu Leu Gly Ile Thr Gly Thr Ser Gly Lys Thr Thr Thr Ser Tyr
        115                 120                 125

Leu Leu Glu Gln Gly Leu Met Ala Ala Asp Leu Lys Val Gly Leu Ile
    130                 135                 140

Gly Thr Thr Gly Thr Arg Ile Asn Gly Arg Pro Val Pro Thr Asp Leu
145                 150                 155                 160

Thr Thr Pro Glu Ala Thr Thr Leu Gln Pro Leu Phe Val Arg Met Leu
                165                 170                 175

Ala Glu Gly Val Thr His Val Val Met Glu Val Ser Ser His Ala Leu
            180                 185                 190

Glu Leu Gly Arg Val Ala Gly Thr Arg Phe Asp Val Ala Gly Phe Thr
        195                 200                 205

Asn Leu Ser Gln Asp His Leu Asp Phe His Pro Thr Met Glu Glu Tyr
    210                 215                 220

Phe Ala Ala Lys Ala Arg Phe Phe Asp Pro Ala Ser Pro Leu Ala Ala
225                 230                 235                 240

Glu Arg Ser Val Val Cys Val Asp Asp Glu Trp Gly Arg Arg Met Ala
                245                 250                 255

Asp Leu Ala Ser Asp Pro Val Thr Val Ala Thr Arg Gly Ala Glu Ala
            260                 265                 270

Asp Val Thr Ala Val Gln Ser Ser Leu Thr Pro Thr Gly Ala Gln Val
        275                 280                 285

Ile Arg Leu Thr Leu Pro Glu Gly Ala Val Glu Ala Glu Leu Pro Leu
    290                 295                 300

Pro Gly Asp Phe Asn Ile Ala Asn Ala Ala Leu Ala Thr Ala Met Ala
305                 310                 315                 320

Trp Ala Val Gly Val Asp Pro Gln Ala Phe Thr Ala Gly Phe Leu Asp
                325                 330                 335

Val Ala Val Pro Gly Arg Met Glu Arg Ile Asp Glu Gly Gln Asp Phe
            340                 345                 350

Leu Ala Val Val Asp Tyr Ala His Lys Pro Ala Ala Val Ala Ala Val
        355                 360                 365

Leu Asp Thr Leu Arg Gly Gln Val Arg Gly Arg Leu Gly Val Val Val
    370                 375                 380

Gly Ala Gly Gly Asp Arg Asp Ala Thr Lys Arg Pro Leu Met Gly Ala
385                 390                 395                 400

Glu Ala Ala His Arg Ala Asp Leu Val Ile Ile Thr Asp Asp Asn Pro
                405                 410                 415

Arg Ser Glu Val Pro Ala Thr Ile Arg Ala Ala Val Leu Gln Gly Ala
            420                 425                 430

Val Asp Ala Gly Thr Asp Ala Glu Ile Arg Glu Ile Gly Asp Arg Ala
        435                 440                 445

Ala Ala Ile Asp Ala Leu Ile Glu Trp Ala Arg Pro Gly Asp Gly Val
    450                 455                 460

Val Val Ala Gly Lys Gly His Glu Val Gly Gln Leu Val Asn Gly Val
465                 470                 475                 480

Asn His His Phe Asp Asp Arg Glu Glu Val Arg Arg Ala Leu Lys Glu
                485                 490                 495

Lys Lys His

<210> 29
<211> 1632
<212> DNA
<213> Corynebacterium humireducens

<220>
<221> CDS
<222> (301)..(1332)
<223> gene asd

<400> 29
cggctggtcc aacgtgctct acgacgacca ggtgggcaag gtctccctcg tgggtgccgg 60

catgaagtcc cacccgggcg tcaccgccga cttcgccgag gcgctccgcg acgccggcgt 120

gaacatcgag ctaatctcca cctcggagat ccgcatctcg gtgctcaccc gcgaggccga 180

cctggacgcc gccgcccgtg ccctgcacga gaagttcgag ctcggcggcg acgccgaggc 240

caccgtctac gccggcaccg gccgctagac aatcccttcc acagaggagc tttcactctc 300

atg acc acc att gct gtc gtc ggt gcc acc ggc cag gtc ggc cgc gtg 348
Met Thr Thr Ile Ala Val Val Gly Ala Thr Gly Gln Val Gly Arg Val
1               5                   10                  15

atg cgc tcg atc ctg gag gag cgc aac ttc ccg gcc gac aag gtc cgt 396
Met Arg Ser Ile Leu Glu Glu Arg Asn Phe Pro Ala Asp Lys Val Arg
            20                  25                  30

ttc ttc gcg tcc ccg cgc tcc gcg ggc acc gtc atc gag ttc cgc ggt 444
Phe Phe Ala Ser Pro Arg Ser Ala Gly Thr Val Ile Glu Phe Arg Gly
        35                  40                  45

gag gac atc acc gtc gag gac ctc acc cag atc acc ccg gag tcc gtc 492
Glu Asp Ile Thr Val Glu Asp Leu Thr Gln Ile Thr Pro Glu Ser Val
    50                  55                  60

aag gac gtc gac atc gcc ctc ttc tcc gcc ggt ggt tcc acc tcc cgt 540
Lys Asp Val Asp Ile Ala Leu Phe Ser Ala Gly Gly Ser Thr Ser Arg
65                  70                  75                  80

gag tgg gcc ccg gtc ttc gcc gag gcc ggc gcg acc gtc atc gac aac 588
Glu Trp Ala Pro Val Phe Ala Glu Ala Gly Ala Thr Val Ile Asp Asn
                85                  90                  95

tcc tcc gcc tgg cgc aag gac gac gag gtc ccg ctc gtc gtc gcc gag 636
Ser Ser Ala Trp Arg Lys Asp Asp Glu Val Pro Leu Val Val Ala Glu
            100                 105                 110

gtc aac ggc gag gag gcg aag aac ccg tcc aag ggc atc gtc gcc aac 684
Val Asn Gly Glu Glu Ala Lys Asn Pro Ser Lys Gly Ile Val Ala Asn
        115                 120                 125

ccg aac tgc acc acc atg gcc gcc atg ccg gtg ctc aag ccg ctg cac 732
Pro Asn Cys Thr Thr Met Ala Ala Met Pro Val Leu Lys Pro Leu His
    130                 135                 140

gac gcc gcc ggc ctc atc cgc ctg cat gtc tcc tcc tac cag gcc gtc 780
Asp Ala Ala Gly Leu Ile Arg Leu His Val Ser Ser Tyr Gln Ala Val
145                 150                 155                 160

tcc ggc tcc ggc ctc gcc ggt gtg gag acc ctg gcg aag cag acc gcc 828
Ser Gly Ser Gly Leu Ala Gly Val Glu Thr Leu Ala Lys Gln Thr Ala
                165                 170                 175

gag ctc ggt gag gac aac gtc aag ctg gtc cat gac ggt tcc gtc gag 876
Glu Leu Gly Glu Asp Asn Val Lys Leu Val His Asp Gly Ser Val Glu
            180                 185                 190

gcc ccc gcc gac ctg ggt ccc tac gtc gcc ccc atc gcg ttc aac gcc 924
Ala Pro Ala Asp Leu Gly Pro Tyr Val Ala Pro Ile Ala Phe Asn Ala
        195                 200                 205

ctg ccg ctg gcc ggc gcg ctg gtc gat gac ggc tcc ctg gag acg gat 972
Leu Pro Leu Ala Gly Ala Leu Val Asp Asp Gly Ser Leu Glu Thr Asp
    210                 215                 220

gag gag cag aag ctg cgc aac gag tcc cgc aag atc ctg gac atc ccg 1020
Glu Glu Gln Lys Leu Arg Asn Glu Ser Arg Lys Ile Leu Asp Ile Pro
225                 230                 235                 240

ggt ctg cgc gtc gcc ggc acc tgc gtc cgc gtg ccg gtg ttc acc ggt 1068
Gly Leu Arg Val Ala Gly Thr Cys Val Arg Val Pro Val Phe Thr Gly
                245                 250                 255

cac acc ctg acc atc cac gcc gag ttc gag cgt ccg atc acc gtc gac 1116
His Thr Leu Thr Ile His Ala Glu Phe Glu Arg Pro Ile Thr Val Asp
            260                 265                 270

gag gcc aag gcc ctg ctc gcc gac gcc ccg ggt gtc gag ctt gtc gac 1164
Glu Ala Lys Ala Leu Leu Ala Asp Ala Pro Gly Val Glu Leu Val Asp
        275                 280                 285

gtc ccg acc ccg ctg gag gcc gcc ggc aag gac acc tcc ctg gtc ggc 1212
Val Pro Thr Pro Leu Glu Ala Ala Gly Lys Asp Thr Ser Leu Val Gly
    290                 295                 300

cgt atc cgc cag gac cag tcc gtc gac gac aac aag ggc ctc gtg ctc 1260
Arg Ile Arg Gln Asp Gln Ser Val Asp Asp Asn Lys Gly Leu Val Leu
305                 310                 315                 320

gtc gtc tcc ggt gac aac ctg cgc aag ggc gcg gcg ctg aac acg gtg 1308
Val Val Ser Gly Asp Asn Leu Arg Lys Gly Ala Ala Leu Asn Thr Val
                325                 330                 335

cag atc gcg gag ctg ctg gtg tag tccaccgcct ccactgatat cgggcccctc 1362
Gln Ile Ala Glu Leu Leu Val
            340

accatgttgt ggtgaggggc ccgttcttgt cggtggtctg gtaggggagg ccttcattct 1422

gtccagacaa ttcttgtggt gtcctggggc gtcggcatgc ggcgtcgggc gggtatcggc 1482

gttcccgttc ctcgtttggc ggttcactgc cggacaatgg aggattttct caggaacagg 1542

gatccctgct gatctcgtat ttcgccccgg aactccgctg gacgctcagc ttgtccccag 1602

aattggggtc tttccgcagc gaattgtgag 1632

<210>  30
<211>  343
<212>  PRT
<213>  Corynebacterium humireducens

<400>  30

Met Thr Thr Ile Ala Val Val Gly Ala Thr Gly Gln Val Gly Arg Val
1               5                   10                  15

Met Arg Ser Ile Leu Glu Glu Arg Asn Phe Pro Ala Asp Lys Val Arg
            20                  25                  30

Phe Phe Ala Ser Pro Arg Ser Ala Gly Thr Val Ile Glu Phe Arg Gly
        35                  40                  45

Glu Asp Ile Thr Val Glu Asp Leu Thr Gln Ile Thr Pro Glu Ser Val
    50                  55                  60

Lys Asp Val Asp Ile Ala Leu Phe Ser Ala Gly Gly Ser Thr Ser Arg
65                  70                  75                  80

Glu Trp Ala Pro Val Phe Ala Glu Ala Gly Ala Thr Val Ile Asp Asn
                85                  90                  95

Ser Ser Ala Trp Arg Lys Asp Asp Glu Val Pro Leu Val Val Ala Glu
            100                 105                 110

Val Asn Gly Glu Glu Ala Lys Asn Pro Ser Lys Gly Ile Val Ala Asn
        115                 120                 125

Pro Asn Cys Thr Thr Met Ala Ala Met Pro Val Leu Lys Pro Leu His
    130                 135                 140

Asp Ala Ala Gly Leu Ile Arg Leu His Val Ser Ser Tyr Gln Ala Val
145                 150                 155                 160

Ser Gly Ser Gly Leu Ala Gly Val Glu Thr Leu Ala Lys Gln Thr Ala
                165                 170                 175

Glu Leu Gly Glu Asp Asn Val Lys Leu Val His Asp Gly Ser Val Glu
            180                 185                 190

Ala Pro Ala Asp Leu Gly Pro Tyr Val Ala Pro Ile Ala Phe Asn Ala
        195                 200                 205

Leu Pro Leu Ala Gly Ala Leu Val Asp Asp Gly Ser Leu Glu Thr Asp
    210                 215                 220

Glu Glu Gln Lys Leu Arg Asn Glu Ser Arg Lys Ile Leu Asp Ile Pro
225                 230                 235                 240

Gly Leu Arg Val Ala Gly Thr Cys Val Arg Val Pro Val Phe Thr Gly
                245                 250                 255

His Thr Leu Thr Ile His Ala Glu Phe Glu Arg Pro Ile Thr Val Asp
            260                 265                 270

Glu Ala Lys Ala Leu Leu Ala Asp Ala Pro Gly Val Glu Leu Val Asp
        275                 280                 285

Val Pro Thr Pro Leu Glu Ala Ala Gly Lys Asp Thr Ser Leu Val Gly
    290                 295                 300

Arg Ile Arg Gln Asp Gln Ser Val Asp Asp Asn Lys Gly Leu Val Leu
305                 310                 315                 320

Val Val Ser Gly Asp Asn Leu Arg Lys Gly Ala Ala Leu Asn Thr Val
                325                 330                 335

Gln Ile Ala Glu Leu Leu Val
            340

<210>  31
<211>  2145
<212>  DNA
<213>  Corynebacterium humireducens

<220>
<221>  CDS
<222>  (301)..(1845)
<223>  gene zwf

<400>  31
cgaccctcta cgtcaccgag ctggccggcc cggagaccgt caacaccatg ccggagccga      60

ccatcgacgc cgtgctcgag cagggtggcc tccacggcga caccctgagc ggcaccgcgg     120

acgacgcgca ggccgtcttc gagcagctgg tgcaggtcgg catcgacatg gaggacgtct     180

tcgcggtgct ggagcgcgag ggcgtcgaga agttcgtgtc ctcctggtcc gagctgctcg     240

actccatgga gtcccgactg cgctagcagt cccctccccg acaggaaagg acctgagcgc     300

atg ccc ctc ccc gtg acc ggc acc gac gcc tgg atc aac ccg ctg cgg       348
Met Pro Leu Pro Val Thr Gly Thr Asp Ala Trp Ile Asn Pro Leu Arg
1               5                   10                  15

aac acc gtc gac aag cgg ctg ccc cgc atc gcc ggc ccc tcc ggc atg       396
Asn Thr Val Asp Lys Arg Leu Pro Arg Ile Ala Gly Pro Ser Gly Met
            20                  25                  30

gtg atc ttc ggc gtg acc ggt gac ctg gcc cgc aag aag ctg ctc ccg       444

Val Ile Phe Gly Val Thr Gly Asp Leu Ala Arg Lys Lys Leu Leu Pro
        35                  40                  45

gcc atc tac gac ctg gcc aac cgt ggc ctc ctg ccg gcc ggt ttc acc      492
Ala Ile Tyr Asp Leu Ala Asn Arg Gly Leu Leu Pro Ala Gly Phe Thr
    50                  55                  60

ctc atc ggt ttc ggc cgt cgt ccc tgg agc aaa ccc gag ttc gag gag      540
Leu Ile Gly Phe Gly Arg Arg Pro Trp Ser Lys Pro Glu Phe Glu Glu
65                  70                  75                  80

tac gtg cgg gag gcc gtc gag agc ggc gcc cgc acc gag ttc cgc gaa      588
Tyr Val Arg Glu Ala Val Glu Ser Gly Ala Arg Thr Glu Phe Arg Glu
                85                  90                  95

cac gtg tgg gag cgg ctg gcc gag ggc atg cac ttc gtc gag ggc aac      636
His Val Trp Glu Arg Leu Ala Glu Gly Met His Phe Val Glu Gly Asn
            100                 105                 110

ttc gac gac gag ggc ttc gac cgc ctc gcc gcc acc ctc gcc gag atg      684
Phe Asp Asp Glu Gly Phe Asp Arg Leu Ala Ala Thr Leu Ala Glu Met
        115                 120                 125

gac acc tcc cgc ggc acg gcg ggc aac tgg gcc ttc tac ctg tcg gtc      732
Asp Thr Ser Arg Gly Thr Ala Gly Asn Trp Ala Phe Tyr Leu Ser Val
    130                 135                 140

ccg ccg gac ttc ttc acc gcc gtc tgc cac cag ctg gag cgc tcc ggc      780
Pro Pro Asp Phe Phe Thr Ala Val Cys His Gln Leu Glu Arg Ser Gly
145                 150                 155                 160

ctg gcg gag gcc ggt gag gag tcg tgg cgt cgc gtg atc atc gag aag      828
Leu Ala Glu Ala Gly Glu Glu Ser Trp Arg Arg Val Ile Ile Glu Lys
                165                 170                 175

ccc ttc ggc cac gac cag gac acg gcc cgg gag ctc aac cgg gtg gtc      876
Pro Phe Gly His Asp Gln Asp Thr Ala Arg Glu Leu Asn Arg Val Val
            180                 185                 190

aac gcc gtc ttc ccg gag gag tcg gtg ttc cgc atc gac cac tac ctg      924
Asn Ala Val Phe Pro Glu Glu Ser Val Phe Arg Ile Asp His Tyr Leu
        195                 200                 205

ggc aag gag acg gtg cag aac atc ctg gcg ctg cgc ttc gcc aac cag      972
Gly Lys Glu Thr Val Gln Asn Ile Leu Ala Leu Arg Phe Ala Asn Gln
    210                 215                 220

ctc ttc gac ccg atc tgg aac gcg cac tac gtc gac cac gtg cag atc     1020
Leu Phe Asp Pro Ile Trp Asn Ala His Tyr Val Asp His Val Gln Ile
225                 230                 235                 240

acc atg gcc gag gac atc ggt ctc ggt ggt cgg gcc ggc tac tac gac     1068
Thr Met Ala Glu Asp Ile Gly Leu Gly Gly Arg Ala Gly Tyr Tyr Asp
                245                 250                 255

ggc atc ggc gcc gcc cgc gac gtc atg cag aac cac ctc ctc cag ctc     1116
Gly Ile Gly Ala Ala Arg Asp Val Met Gln Asn His Leu Leu Gln Leu
            260                 265                 270

ctg gcg ctg gtg gcc atg gag gag ccc atc gcc ttc gac ccg gct gag     1164
Leu Ala Leu Val Ala Met Glu Glu Pro Ile Ala Phe Asp Pro Ala Glu
        275                 280                 285

ctg cag gcg gag aag gtc aag gtc ctg cgc gcg acc aac ccc gtc ttc 1212
Leu Gln Ala Glu Lys Val Lys Val Leu Arg Ala Thr Asn Pro Val Phe
    290                 295                 300

ccg ctg aac aag acc acc gcc cgt ggc cag tac gcg gcg ggc tgg cag 1260
Pro Leu Asn Lys Thr Thr Ala Arg Gly Gln Tyr Ala Ala Gly Trp Gln
305                 310                 315                 320

ggc tcg cac aag gtg cgt ggc ctg cgt gag gag gag ggt ttc gac ccg 1308
Gly Ser His Lys Val Arg Gly Leu Arg Glu Glu Glu Gly Phe Asp Pro
                325                 330                 335

gac tcc acc acg gag acc tac gcc gcc tgc acc ctg gag atc aac tcc 1356
Asp Ser Thr Thr Glu Thr Tyr Ala Ala Cys Thr Leu Glu Ile Asn Ser
            340                 345                 350

cgc cgc tgg gcg ggc gtg ccc ttc tac ctg cgg acc ggc aag cgc ctg 1404
Arg Arg Trp Ala Gly Val Pro Phe Tyr Leu Arg Thr Gly Lys Arg Leu
        355                 360                 365

ggg cgc cgc gtc acg gag atc gcc ctc gtg ttc aag gag gcg ccg cac 1452
Gly Arg Arg Val Thr Glu Ile Ala Leu Val Phe Lys Glu Ala Pro His
    370                 375                 380

cag ccc ttc ctc gag ggg cag acg tcc tcg ctg ggg cag aac gcg atc 1500
Gln Pro Phe Leu Glu Gly Gln Thr Ser Ser Leu Gly Gln Asn Ala Ile
385                 390                 395                 400

gtc atc cgc gtc cag ccc gac gag ggc gtg ctc atg cgc ttc ggc tcc 1548
Val Ile Arg Val Gln Pro Asp Glu Gly Val Leu Met Arg Phe Gly Ser
                405                 410                 415

aag gtg ccc ggc tcc gcg atg gag gtc cgt gac gtc aac atg gac ttc 1596
Lys Val Pro Gly Ser Ala Met Glu Val Arg Asp Val Asn Met Asp Phe
            420                 425                 430

tcc tac gcg gag gcc ttc acc gag gcg tcc ccg gag gcc tat gag cgg 1644
Ser Tyr Ala Glu Ala Phe Thr Glu Ala Ser Pro Glu Ala Tyr Glu Arg
        435                 440                 445

ctc atc ctc gat gcg ctt ctc gac gag tcc tcg ctc ttc ccc acc aac 1692
Leu Ile Leu Asp Ala Leu Leu Asp Glu Ser Ser Leu Phe Pro Thr Asn
    450                 455                 460

gag gag gtc gag ctc tcc tgg gcg atc ctg gac ccg atc ctc gag ttc 1740
Glu Glu Val Glu Leu Ser Trp Ala Ile Leu Asp Pro Ile Leu Glu Phe
465                 470                 475                 480

tgg gct gag aag ggc cag ccc gag gag tac gag tcc ggc acc tgg ggg 1788
Trp Ala Glu Lys Gly Gln Pro Glu Glu Tyr Glu Ser Gly Thr Trp Gly
                485                 490                 495

ccc gcc tcc gcc gac cgc atg ctg gaa cgg acc ggc cgc gcc tgg cgc 1836
Pro Ala Ser Ala Asp Arg Met Leu Glu Arg Thr Gly Arg Ala Trp Arg
            500                 505                 510

cgc ccc tga cctggaaggg acccgacttg aagatcgaac tgccggacac 1885
Arg Pro

cgacacccgg aacatcgccg ccagcctgct cgaggcccgg gagcacaact cccagaccac 1945

cgggcgggtg ctcaccctca tcgtcgtcgc cgcgctcgac gacgacgtcg accacatcat 2005

ctccgtcacc cgcgacgcct cccacgagca ccccgcccgt gtgctggtgc tgctcaccgg 2065

ccgggaggac gctgactcgc gtctcgacgc cgaggtgcgc gtcggcggtc acgccggcgc 2125

ctcggagatg gtcatcatgc 2145

<210> 32
<211> 514
<212> PRT
<213> Corynebacterium humireducens

<400> 32

Met Pro Leu Pro Val Thr Gly Thr Asp Ala Trp Ile Asn Pro Leu Arg
1               5                   10                  15

Asn Thr Val Asp Lys Arg Leu Pro Arg Ile Ala Gly Pro Ser Gly Met
            20                  25                  30

Val Ile Phe Gly Val Thr Gly Asp Leu Ala Arg Lys Lys Leu Leu Pro
        35                  40                  45

Ala Ile Tyr Asp Leu Ala Asn Arg Gly Leu Leu Pro Ala Gly Phe Thr
    50                  55                  60

Leu Ile Gly Phe Gly Arg Arg Pro Trp Ser Lys Pro Glu Phe Glu Glu
65                  70                  75                  80

Tyr Val Arg Glu Ala Val Glu Ser Gly Ala Arg Thr Glu Phe Arg Glu
                85                  90                  95

His Val Trp Glu Arg Leu Ala Glu Gly Met His Phe Val Glu Gly Asn
            100                 105                 110

Phe Asp Asp Glu Gly Phe Asp Arg Leu Ala Ala Thr Leu Ala Glu Met
        115                 120                 125

Asp Thr Ser Arg Gly Thr Ala Gly Asn Trp Ala Phe Tyr Leu Ser Val
    130                 135                 140

Pro Pro Asp Phe Phe Thr Ala Val Cys His Gln Leu Glu Arg Ser Gly
145                 150                 155                 160

Leu Ala Glu Ala Gly Glu Glu Ser Trp Arg Arg Val Ile Ile Glu Lys
                165                 170                 175

Pro Phe Gly His Asp Gln Asp Thr Ala Arg Glu Leu Asn Arg Val Val
            180                 185                 190

Asn Ala Val Phe Pro Glu Glu Ser Val Phe Arg Ile Asp His Tyr Leu
        195                 200                 205

Gly Lys Glu Thr Val Gln Asn Ile Leu Ala Leu Arg Phe Ala Asn Gln
    210                 215                 220

Leu Phe Asp Pro Ile Trp Asn Ala His Tyr Val Asp His Val Gln Ile
225                 230                 235                 240

Thr Met Ala Glu Asp Ile Gly Leu Gly Gly Arg Ala Gly Tyr Tyr Asp
                245                 250                 255

Gly Ile Gly Ala Ala Arg Asp Val Met Gln Asn His Leu Leu Gln Leu
            260                 265                 270

Leu Ala Leu Val Ala Met Glu Glu Pro Ile Ala Phe Asp Pro Ala Glu
        275                 280                 285

Leu Gln Ala Glu Lys Val Lys Val Leu Arg Ala Thr Asn Pro Val Phe
    290                 295                 300

Pro Leu Asn Lys Thr Thr Ala Arg Gly Gln Tyr Ala Ala Gly Trp Gln
305                 310                 315                 320

Gly Ser His Lys Val Arg Gly Leu Arg Glu Glu Glu Gly Phe Asp Pro
                325                 330                 335

Asp Ser Thr Thr Glu Thr Tyr Ala Ala Cys Thr Leu Glu Ile Asn Ser
            340                 345                 350

Arg Arg Trp Ala Gly Val Pro Phe Tyr Leu Arg Thr Gly Lys Arg Leu
        355                 360                 365

Gly Arg Arg Val Thr Glu Ile Ala Leu Val Phe Lys Glu Ala Pro His
    370                 375                 380

Gln Pro Phe Leu Glu Gly Gln Thr Ser Ser Leu Gly Gln Asn Ala Ile
385                 390                 395                 400

Val Ile Arg Val Gln Pro Asp Glu Gly Val Leu Met Arg Phe Gly Ser
                405                 410                 415

Lys Val Pro Gly Ser Ala Met Glu Val Arg Asp Val Asn Met Asp Phe
            420                 425                 430

Ser Tyr Ala Glu Ala Phe Thr Glu Ala Ser Pro Glu Ala Tyr Glu Arg
        435                 440                 445

Leu Ile Leu Asp Ala Leu Leu Asp Glu Ser Ser Leu Phe Pro Thr Asn
    450                 455                 460

Glu Glu Val Glu Leu Ser Trp Ala Ile Leu Asp Pro Ile Leu Glu Phe
465                 470                 475                 480

Trp Ala Glu Lys Gly Gln Pro Glu Glu Tyr Glu Ser Gly Thr Trp Gly
                485                 490                 495

Pro Ala Ser Ala Asp Arg Met Leu Glu Arg Thr Gly Arg Ala Trp Arg
            500                 505                 510

Arg Pro

<210>  33
<211>  1551
<212>  DNA
<213>  Corynebacterium humireducens

<220>
<221>  CDS
<222>  (301)..(1251)
<223>  gene opcA

<400>  33
cgcgatggag gtccgtgacg tcaacatgga cttctcctac gcggaggcct tcaccgaggc      60

gtccccggag gcctatgagc ggctcatcct cgatgcgctt ctcgacgagt cctcgctctt     120

ccccaccaac gaggaggtcg agctctcctg ggcgatcctg gacccgatcc tcgagttctg     180

ggctgagaag ggccagcccg aggagtacga gtccggcacc tgggggcccg cctccgccga     240

ccgcatgctg gaacggaccg gccgcgcctg gcgccgcccc tgacctggaa gggacccgac     300

ttg aag atc gaa ctg ccg gac acc gac acc cgg aac atc gcc gcc agc       348
Leu Lys Ile Glu Leu Pro Asp Thr Asp Thr Arg Asn Ile Ala Ala Ser
1               5                   10                  15

ctg ctc gag gcc cgg gag cac aac tcc cag acc acc ggg cgg gtg ctc       396
Leu Leu Glu Ala Arg Glu His Asn Ser Gln Thr Thr Gly Arg Val Leu
            20                  25                  30

acc ctc atc gtc gtc gcc gcg ctc gac gac gac gtc gac cac atc atc       444
Thr Leu Ile Val Val Ala Ala Leu Asp Asp Asp Val Asp His Ile Ile
        35                  40                  45

tcc gtc acc cgc gac gcc tcc cac gag cac ccc gcc cgt gtg ctg gtg       492
Ser Val Thr Arg Asp Ala Ser His Glu His Pro Ala Arg Val Leu Val
    50                  55                  60

ctg ctc acc ggc cgg gag gac gct gac tcg cgt ctc gac gcc gag gtg       540
Leu Leu Thr Gly Arg Glu Asp Ala Asp Ser Arg Leu Asp Ala Glu Val
65                  70                  75                  80

cgc gtc ggc ggt cac gcc ggc gcc tcg gag atg gtc atc atg cgc atc       588

Arg Val Gly Gly His Ala Gly Ala Ser Glu Met Val Ile Met Arg Ile
                85                  90                  95

acg ggc gag atg gtc aac cac ctc gcc gcg gtg gtc acc ccc ctg ctg      636
Thr Gly Glu Met Val Asn His Leu Ala Ala Val Val Thr Pro Leu Leu
            100                 105                 110

ctg ccg gac acc ccg gtc gtc gcc tgg tgg ccg acc acc gcg ccg gag      684
Leu Pro Asp Thr Pro Val Val Ala Trp Trp Pro Thr Thr Ala Pro Glu
        115                 120                 125

aac ccc tcc ggg cat ccg ctg ggc cag ctg gcg cag cgc cgc atc acc      732
Asn Pro Ser Gly His Pro Leu Gly Gln Leu Ala Gln Arg Arg Ile Thr
    130                 135                 140

aac gcg gac cac ccg gac agt gag tcg gtc ctc cgg ctc cgc cgt gcc      780
Asn Ala Asp His Pro Asp Ser Glu Ser Val Leu Arg Leu Arg Arg Ala
145                 150                 155                 160

acc tac gcc ccc ggt gac tcc gac atg gtg tgg tcg aag atc acc gcc      828
Thr Tyr Ala Pro Gly Asp Ser Asp Met Val Trp Ser Lys Ile Thr Ala
                165                 170                 175

tgg cgc ggt atc gtc gcc tcc tcc ctg gac cgt cac ccg cac gag gac      876
Trp Arg Gly Ile Val Ala Ser Ser Leu Asp Arg His Pro His Glu Asp
            180                 185                 190

gtg gag gcc gcc gtc ctc acc ggc ccg gcg gat gag ccg cag atc gac      924
Val Glu Ala Ala Val Leu Thr Gly Pro Ala Asp Glu Pro Gln Ile Asp
        195                 200                 205

atc gcc gcc ggc tgg ctc gcc gac cgc ctc ggt gtg ccg gtc cgc cgg      972
Ile Ala Ala Gly Trp Leu Ala Asp Arg Leu Gly Val Pro Val Arg Arg
    210                 215                 220

gag gtg tgt gaa ccg ggt aac gac gcc cgg ctc ttc ccc atc cgt cgt     1020
Glu Val Cys Glu Pro Gly Asn Asp Ala Arg Leu Phe Pro Ile Arg Arg
225                 230                 235                 240

ctc aag ttc gaa cgc gcc tcc ggc ccg gtc cgc gtc gag gtg ctc gac     1068
Leu Lys Phe Glu Arg Ala Ser Gly Pro Val Arg Val Glu Val Leu Asp
                245                 250                 255

cac cgg acc gtg ctc gtc gcc ctg cca ggg cgc ccg gag tcg ctc gtc     1116
His Arg Thr Val Leu Val Ala Leu Pro Gly Arg Pro Glu Ser Leu Val
            260                 265                 270

gcc atg aac gag cgt tcc gac gcc gac tgc ctc tcc gag gaa ctg cgt     1164
Ala Met Asn Glu Arg Ser Asp Ala Asp Cys Leu Ser Glu Glu Leu Arg
        275                 280                 285

cac ctc gac ccc gat ctc gcc tac gcc aac gct ctg cgc gga ctc acc     1212
His Leu Asp Pro Asp Leu Ala Tyr Ala Asn Ala Leu Arg Gly Leu Thr
    290                 295                 300

cgt gtg aaa gga atc aat ggt cac cct gcg tcg tcc tga caccctgtcc      1261
Arg Val Lys Gly Ile Asn Gly His Pro Ala Ser Ser
305                 310                 315

accctgctcg accaggccgc cacgtcgctc atcgacgtcg tcgctgccct ccagaccacc   1321

gggggcggtg tccacgccga cggtgtggcc cgcctggtgc tcaccggcgg cggcgccggc   1381

atcggcctgc tggagcgcgt ccgtgagctc gaggacgccg actcccgcat cgactggtcc 1441

cgcgtccacg tcttcttcgg cgacgagcgc aacgtgcccg tctcccacga ggactccaac 1501

gaggggcagg cgcgtaccgc cctgctcagc cacgtcgaca tcccggaggc 1551

<210> 34
<211> 316
<212> PRT
<213> Corynebacterium humireducens

<400> 34

Leu Lys Ile Glu Leu Pro Asp Thr Asp Thr Arg Asn Ile Ala Ala Ser
1               5                   10                  15

Leu Leu Glu Ala Arg Glu His Asn Ser Gln Thr Thr Gly Arg Val Leu
            20                  25                  30

Thr Leu Ile Val Val Ala Ala Leu Asp Asp Asp Val Asp His Ile Ile
        35                  40                  45

Ser Val Thr Arg Asp Ala Ser His Glu His Pro Ala Arg Val Leu Val
    50                  55                  60

Leu Leu Thr Gly Arg Glu Asp Ala Asp Ser Arg Leu Asp Ala Glu Val
65                  70                  75                  80

Arg Val Gly Gly His Ala Gly Ala Ser Glu Met Val Ile Met Arg Ile
                85                  90                  95

Thr Gly Glu Met Val Asn His Leu Ala Ala Val Val Thr Pro Leu Leu
            100                 105                 110

Leu Pro Asp Thr Pro Val Val Ala Trp Trp Pro Thr Thr Ala Pro Glu
        115                 120                 125

Asn Pro Ser Gly His Pro Leu Gly Gln Leu Ala Gln Arg Arg Ile Thr
    130                 135                 140

Asn Ala Asp His Pro Asp Ser Glu Ser Val Leu Arg Leu Arg Arg Ala
145                 150                 155                 160

Thr Tyr Ala Pro Gly Asp Ser Asp Met Val Trp Ser Lys Ile Thr Ala
                165                 170                 175

Trp Arg Gly Ile Val Ala Ser Ser Leu Asp Arg His Pro His Glu Asp
            180                 185                 190

Val Glu Ala Ala Val Leu Thr Gly Pro Ala Asp Glu Pro Gln Ile Asp

195                 200                 205

Ile Ala Ala Gly Trp Leu Ala Asp Arg Leu Gly Val Pro Val Arg Arg
    210                 215                 220

Glu Val Cys Glu Pro Gly Asn Asp Ala Arg Leu Phe Pro Ile Arg Arg
225                 230                 235                 240

Leu Lys Phe Glu Arg Ala Ser Gly Pro Val Arg Val Glu Val Leu Asp
                245                 250                 255

His Arg Thr Val Leu Val Ala Leu Pro Gly Arg Pro Glu Ser Leu Val
            260                 265                 270

Ala Met Asn Glu Arg Ser Asp Ala Asp Cys Leu Ser Glu Glu Leu Arg
        275                 280                 285

His Leu Asp Pro Asp Leu Ala Tyr Ala Asn Ala Leu Arg Gly Leu Thr
    290                 295                 300

Arg Val Lys Gly Ile Asn Gly His Pro Ala Ser Ser
305                 310                 315

<210>  35
<211>  1569
<212>  DNA
<213>  Corynebacterium humireducens

<220>
<221>  CDS
<222>  (301)..(1269)
<223>  gene ddh

<400>  35
ggcgccaccc tagcgcctga gccggtttct gacacccgcg gatcctgcgg taccatctgc      60

aggcaggcgg gtacatgtgc ctgccggtgc tggagtggcg caatcggtag cgcaacggtc     120

ttgtaaaccg taggttgcga gttcaagtct cgtctccagc tctgctctcc acgaggtggg     180

gtccgggtga accggccccg cctcgttccc ttttgtggga ctcatttcta ttgactgacc     240

ggtgatgggc cgtagaatgg gcggcagcaa gcaccgtccc ccacctcaag gagtcagcac     300

atg gcc aac atc agg gtc gcg atc gtc ggt tac ggc aac ctc ggc aag       348
Met Ala Asn Ile Arg Val Ala Ile Val Gly Tyr Gly Asn Leu Gly Lys
1               5                   10                  15

tcc gtc gag aag ctc atc ggc cag cag ccg gac atg gag ctc gcg ggc       396
Ser Val Glu Lys Leu Ile Gly Gln Gln Pro Asp Met Glu Leu Ala Gly
            20                  25                  30

gtg ttc tcg cgt cgc gac gca ctg gac acc ctc gac ccg gcc acc ccg       444
Val Phe Ser Arg Arg Asp Ala Leu Asp Thr Leu Asp Pro Ala Thr Pro

35                  40                  45

gtc ctg ccc gtc gcg gag ttc ggt gag cac acc gac aag ttc gac gtc      492
Val Leu Pro Val Ala Glu Phe Gly Glu His Thr Asp Lys Phe Asp Val
    50                  55                  60

gcc gtc ctc tgc ctc ggc tcc gcc acc gac atc ccg gag cag gcc ccc      540
Ala Val Leu Cys Leu Gly Ser Ala Thr Asp Ile Pro Glu Gln Ala Pro
65                  70                  75                  80

gag ttc atc cgc cac gcc aac acg gtc gac acc tac gac aac cac cgc      588
Glu Phe Ile Arg His Ala Asn Thr Val Asp Thr Tyr Asp Asn His Arg
                85                  90                  95

gac atc ccg cgc cac cgc gcc gag atg gac agg gtg gcc agg gag aac      636
Asp Ile Pro Arg His Arg Ala Glu Met Asp Arg Val Ala Arg Glu Asn
            100                 105                 110

ggc acc gtc gcc atc gtc tcc acc ggc tgg gac ccg ggc atg ttc tcc      684
Gly Thr Val Ala Ile Val Ser Thr Gly Trp Asp Pro Gly Met Phe Ser
        115                 120                 125

ctc aac cgc acc atc ggc cag gcg ctc ctg ccg ggc ggc cag cag cag      732
Leu Asn Arg Thr Ile Gly Gln Ala Leu Leu Pro Gly Gly Gln Gln Gln
    130                 135                 140

acc ttc tgg ggt ccg ggc ctc tcg cag ggc cac tct gac gcg gtg cgc      780
Thr Phe Trp Gly Pro Gly Leu Ser Gln Gly His Ser Asp Ala Val Arg
145                 150                 155                 160

cgc gtc gcg ggc gtg aag aag ggc gtg cag tac acc atc ccc tcc ccg      828
Arg Val Ala Gly Val Lys Lys Gly Val Gln Tyr Thr Ile Pro Ser Pro
                165                 170                 175

gag gcc ctc gag gcg gcc cgc aag ggt gag tcg ggt ctc agc ggc aag      876
Glu Ala Leu Glu Ala Ala Arg Lys Gly Glu Ser Gly Leu Ser Gly Lys
            180                 185                 190

cag gcc cac ctg cgc cgc tgc ttc atc gtg gcg gac gag gcc gac cac      924
Gln Ala His Leu Arg Arg Cys Phe Ile Val Ala Asp Glu Ala Asp His
        195                 200                 205

gag cgc atc gag aac gag atc cgc acc atg ccc gac tac ttc gtc ggc      972
Glu Arg Ile Glu Asn Glu Ile Arg Thr Met Pro Asp Tyr Phe Val Gly
    210                 215                 220

tac gag gtg gag gtc aac ttc atc gac gag gcc acc ttc gac gcc gag     1020
Tyr Glu Val Glu Val Asn Phe Ile Asp Glu Ala Thr Phe Asp Ala Glu
225                 230                 235                 240

cac acc ggc atg ccg cac ggc ggg cac gtc atc acg gcg ggt gac acc     1068
His Thr Gly Met Pro His Gly Gly His Val Ile Thr Ala Gly Asp Thr
                245                 250                 255

ggc ggc tac gtc cag tcc atc gag tac aac ctc gac ctg gac cgg aac     1116
Gly Gly Tyr Val Gln Ser Ile Glu Tyr Asn Leu Asp Leu Asp Arg Asn
            260                 265                 270

ccg gac ttc acc ggt gcc gtc gcc ctg gca tac gcc cgt gcg gcc cac     1164
Pro Asp Phe Thr Gly Ala Val Ala Leu Ala Tyr Ala Arg Ala Ala His
        275                 280                 285

cgt ctg cgt gag gcg ggc cag gcc ggc gcc tac acc gtc ctc gag gtc     1212

Arg Leu Arg Glu Ala Gly Gln Ala Gly Ala Tyr Thr Val Leu Glu Val
    290                 295                 300

gcc ccg tac ctg atc tcc ccg acg ccg ctc gag gag ctc atc gcc cgc      1260
Ala Pro Tyr Leu Ile Ser Pro Thr Pro Leu Glu Glu Leu Ile Ala Arg
305                 310                 315                 320

gac gtg tag ggtccaccac cgggatcagc aggagggcac cgaccagcag              1309
Asp Val

gaccaggccg atgccgagga tcccgaagcg gtcggtgccg cccagcagga cgaacatccc    1369

gaaggcgagc ggcgagagcc acgacacggc gcgtccggtg gtgacgtaca ggccgaagag    1429

ctggccctcc cgcccgtcgg gggcgacgcg ggagaggaac gagcgggccg ccgactgggc    1489

ggggccgacg aacaggcaca ggatgaggcc gaagatccag aacatctgcg ggccctccac    1549

gaagtagagg acgcccgcgg                                                1569

<210>  36
<211>  322
<212>  PRT
<213>  Corynebacterium humireducens

<400>  36

Met Ala Asn Ile Arg Val Ala Ile Val Gly Tyr Gly Asn Leu Gly Lys
1               5                   10                  15

Ser Val Glu Lys Leu Ile Gly Gln Gln Pro Asp Met Glu Leu Ala Gly
            20                  25                  30

Val Phe Ser Arg Arg Asp Ala Leu Asp Thr Leu Asp Pro Ala Thr Pro
        35                  40                  45

Val Leu Pro Val Ala Glu Phe Gly Glu His Thr Asp Lys Phe Asp Val
    50                  55                  60

Ala Val Leu Cys Leu Gly Ser Ala Thr Asp Ile Pro Glu Gln Ala Pro
65                  70                  75                  80

Glu Phe Ile Arg His Ala Asn Thr Val Asp Thr Tyr Asp Asn His Arg
                85                  90                  95

Asp Ile Pro Arg His Arg Ala Glu Met Asp Arg Val Ala Arg Glu Asn
            100                 105                 110

Gly Thr Val Ala Ile Val Ser Thr Gly Trp Asp Pro Gly Met Phe Ser
        115                 120                 125

Leu Asn Arg Thr Ile Gly Gln Ala Leu Leu Pro Gly Gly Gln Gln Gln
    130                 135                 140

Thr Phe Trp Gly Pro Gly Leu Ser Gln Gly His Ser Asp Ala Val Arg
145                 150                 155                 160

Arg Val Ala Gly Val Lys Lys Gly Val Gln Tyr Thr Ile Pro Ser Pro
                165                 170                 175

Glu Ala Leu Glu Ala Ala Arg Lys Gly Glu Ser Gly Leu Ser Gly Lys
            180                 185                 190

Gln Ala His Leu Arg Arg Cys Phe Ile Val Ala Asp Glu Ala Asp His
        195                 200                 205

Glu Arg Ile Glu Asn Glu Ile Arg Thr Met Pro Asp Tyr Phe Val Gly
    210                 215                 220

Tyr Glu Val Glu Val Asn Phe Ile Asp Glu Ala Thr Phe Asp Ala Glu
225                 230                 235                 240

His Thr Gly Met Pro His Gly Gly His Val Ile Thr Ala Gly Asp Thr
                245                 250                 255

Gly Gly Tyr Val Gln Ser Ile Glu Tyr Asn Leu Asp Leu Asp Arg Asn
            260                 265                 270

Pro Asp Phe Thr Gly Ala Val Ala Leu Ala Tyr Ala Arg Ala Ala His
        275                 280                 285

Arg Leu Arg Glu Ala Gly Gln Ala Gly Ala Tyr Thr Val Leu Glu Val
    290                 295                 300

Ala Pro Tyr Leu Ile Ser Pro Thr Pro Leu Glu Glu Leu Ile Ala Arg
305                 310                 315                 320

Asp Val

## Beschreibung

Die vorliegende Erfindung betrifft ein Verfahren zur Produktion von L-Lysin, in welchem ein alkaliphiles Bakterium, vorzugsweise ein Stamm der Spezies Corynebacterium humireducens, und/oder eine feedback-resistente Aspartat-Kinase aus C. humireducens eingesetzt wird.

Verfahren zur Produktion von L-Lysin, in welchen Bakterien der Gattung Corynebacterium eingesetzt werden, sind dem Fachmann bekannt.

Obwohl zahlreiche Corynebacterium-Arten bekannt sind, werden in diesen Verfahren üblicherweise Bakterien der Art Corynebacterium glutamicum eingesetzt, da sich diese Art als besonders vorteilhaft für die Produktion von L-Lysin herausgestellt hat.

Aufgabe der vorliegenden Erfindung war es, einen neuen Stamm zur Verfügung zu stellen, der als alternativer Produktionsstamm zu C. glutamicum für die Herstellung von L-Lysin in Frage kommt, entweder weil er unmittelbar eine signifikante Überproduktion von L-Lysin aufweist oder aber zumindest als erfolgversprechender Ausgangsstamm für die Entwicklung eines L-Lysin-Produktionsstamms in Betracht kommt.

Um als Ausgangsstamm für die Entwicklung eines neuen L-Lysin-Produktionsstamms in Frage zu kommen, ist bereits eine relativ geringe L-Lysin-Überproduktion ausreichend. Denn durch Überexpression oder Abschwächung bereits bekannter, für die L-Lysin-Produktion förderlicher bzw. schädlicher Gene sowie gegebenenfalls durch ungerichtete Mutagenese kann ausgehend von einem solchen Ausgangsstamm die L-Lysin-Ausbeute entsprechend erhöht werden.

Erfindungsgemäß wurde nun überraschenderweise gefunden, dass ein alkaliphiles Bakterium, nämlich ein Corynebacterium der Art C. humireducens, einen geeigneten L-Lysin-Produktionsstamm darstellt.

Denn durch Kultivierung eines Stammes der Art C. humireducens in einem Medium, das S-2-Aminoethyl-L-Cystein (AEC) und gegebenenfalls Threonin enthält, konnte ein Stamm erhalten werden, der eine signifikante Überproduktion an L-Lysin aufweist.

Ein erster Gegenstand der vorliegenden Erfindung ist daher ein Verfahren zur Herstellung eines alkaliphilen Bakteriums, insbesondere eines Stammes der Art Corynebacterium humireducens, das/der L-Lysin überproduziert, dadurch gekennzeichnet, dass ein alkaliphiles Bakterium, vorzugsweise ein Corynebacterium humireducens- Stamm, in einem Medium kultiviert wird, das S-2-Aminoethyl-L-Cystein (AEC) und gegebenenfalls L-Threonin enthält.

Die Selektion AEC-resistenter Stämme erfolgt dabei vorzugsweise in einem Medium, das AEC in einer Menge von 5 bis 100 mM, vorzugsweise 10 bis 50 mM, insbesondere 20 bis 30 mM, enthält.

Neben AEC ist in einer erfindungsgemäß bevorzugten Ausführungsform als weitere Selektionssubstanz L-Threonin in dem Selektionsmedium enthalten. Das L-Threonin ist in dem Selektionsmedium vorzugsweise in einer Menge von 2 bis 50 mM, besonders bevorzugt 5 bis 30 mM, insbesondere 8 bis 15 mM, enthalten.

Bei dem zur Selektion eingesetzten Medium handelt es sich hierbei vorzugsweise um ein Minimalmedium. Das Minimalmedium enthält vorzugsweise weniger als 5 Gew.-%, insbesondere weniger als 2 Gew.-% an Kohlenstoffquellen, wobei als Kohlenstoffquelle vorzugsweise Glucose enthalten ist. Das Minimalmedium enthält weiterhin vorzugsweise weniger als 10 Gew.-%, insbesondere weniger als 5 Gew.-%, an Salzen. Das Selektionsverfahren wird vorzugsweise auf Agar-Platten durchgeführt, die das Minimalmedium enthalten.

Weiterer Gegenstand der vorliegenden Erfindung ist daher auch ein L-Lysin überproduzierendes alkaliphiles Bakterium, insbesondere ein L-Lysin überproduzierendes alkaliphiles Corynebacterium, vor allem ein L-Lysin überproduzierendes Corynebacterium der Art Corynebacterium humireducens, erhältlich nach einem erfindungsgemäßen Verfahren.

Das erfindungsgemäße alkaliphile Bakterium ist weiterhin vorzugsweise halotolerant und/oder Huminsäure reduzierend.

Unter einem "alkaliphilen Bakterium" ist erfindungsgemäß ein Bakterium zu verstehen, das dazu in der Lage ist, bei einem pH-Wert von 8,5 bis 11 zu wachsen. Vorzugsweise ist darunter ein Bakterium zu verstehen, das auch dazu in der Lage, bei einem pH-Wert von 9 bis 10,5 zu wachsen.

Unter einem "halotoleranten Bakterium" ist erfindungsgemäß ein Bakterium zu verstehen, das dazu in der Lage ist, bei Wasseraktivitäten von 0,6 bis 0,98 zu wachsen. Vorzugsweise ist darunter ein Bakterium zu verstehen, das auch dazu in der Lage ist, bei Wasseraktivitäten von 0,75 bis 0,9 zu wachsen.

Das erfindungsgemäße L-Lysin überproduzierende alkaliphile Bakterium, insbesondere der erfindungsgemäße C. humireducens-Stamm, ist hierbei vorzugsweise resistent gegenüber AEC, besonders bevorzugt gegenüber AEC in einer Konzentration von 5 bis 100 mM, vorzugsweise 10 bis 50 mM, insbesondere 20 bis 30 mM.

In einer weiteren erfindungsgemäß bevorzugten Ausführungsform ist das erfindungsgemäße L-Lysin überproduzierende alkaliphile Bakterium, insbesondere der erfindungsgemäße C. humireducens-Stamm, resistent gegenüber einer Kombination aus AEC und L-Threonin, wobei die AEC-Konzentration vorzugsweise von 5 bis 100 mM, besonders bevorzugt 10 bis 50 mM, insbesondere 20 bis 30 mM, beträgt und die L-Threonin-Konzentration vorzugsweise 2 bis 50 mM, besonders bevorzugt 5 bis 30 mM, insbesondere 8 bis 15 mM, beträgt.

Das erfindungsgemäße alkaliphile Bakterium, insbesondere der erfindungsgemäße Corynebacterium humireducens-Stamm, produziert L-Lysin vorzugsweise in einer Menge von mindestens 500 mg/l, insbesondere mindestens 1 g/l, wobei das produzierte L-Lysin vorzugsweise im Medium akkumuliert.

Der Ausgangsstamm C. humireducens wird zum ersten Mal beschrieben durch Wu et al. (International Journal of Systematic and Evolutionary Microbiology (2011), 61, 882-887). Er wurde bei der DSMZ hinterlegt unter der Hinterlegungsnummer DSM 45392, seine 16S rRNA ist wurde bei EMBL hinterlegt und hat die Zugangsnummer GQ421281. Bei dem Ausgangsstamm handelt es sich um ein halotolerantes, alkaliphiles, Huminsäure reduzierendes Bakterium.

Weitere Informationen über C. humireducens sind folgenden Publikationen zu entnehmen: Wu et al. (Microb. Biotechnol. (2013), 6(2), 141-149), Lin et al. (Bioresour. Technol. (2013), 136, 302-308).

Die Selektion in AEC-Medium bzw. auf einem Gemisch aus AEC und Threonin zum Erhalt eines L-Lysin überproduzierenden Stamms wurde zuvor bereits für C. glutamicum beschrieben. Für C. glutamicum konnte nachgewiesen werden, dass die Überproduktion durch Selektion auf AEC dadurch erzielt wird, dass die Aspartat-Kinase (lysC) in eine feedback-resistente Variante überführt wird.

Erfindungsgemäß wurde nun überraschenderweise gefunden, dass bei Durchführung der Selektion in AEC-Medium feedback-resistente Varianten von C. humireducens aufgefunden wurden, die ein unverändertes lysC aufweisen und somit offensichtlich über keine feedback-resistente Variante von lysC verfügen. Bei Durchführung der Selektion mit einer Kombination aus AEC und Threonin konnten hingegen überraschenderweise mutierte lysC-Gene aufgefunden werden, so dass davon auszugehen ist, dass diese Gene für feedback-resistente Varianten des LysC kodieren, die von der nativen Aspartat-Kinase aus C. humireducens abgeleitet sind.

Unter einer "feedback-resistenten" bzw. "desensibilisierten" Aspartat-Kinase versteht man Aspartat-Kinasen (LysC), die im Vergleich zum Wildtyp weniger stark, vorzugsweise mindestens 15 oder 30 %, besonders bevorzugt mindestens 50 % weniger stark, durch AEC und/oder Lysin und/oder Mischungen aus Lysin und Threonin und/oder Mischungen aus AEC und Threonin, vorzugsweise durch Mischungen aus AEC und Threonin, gehemmt werden.

Folgende feedback-resistente Varianten der Aspartat-Kinase aus C. glutamicum sind dem Fachmann bekannt: A279T (US5688671), A279V (JP6-261766), L297Q (DE102006026328), S301 F (US6844176), S301Y (Kalinowski et al., Molecular and General Genetics 224, 317-324 (1990)), T3081 (JP6-261766), T3111 (WO00/63388), R320G und G345D (Jetten et al., Applied Microbiology and Biotechnology 43, 76-82 (1995)), T380I (WO01/49854), S381 F (EP0435132).

Feedback-resistente Varianten der Aspartat-Kinase aus C. humireducens wurden hingegen im Stand der Technik bisher noch nicht beschrieben.

Ein weiterer Gegenstand der vorliegenden Erfindung sind daher Aspartat-Kinasen (LysC), die eine Sequenzidentität von mindestens 92 %, vorzugsweise mindestens 94, 96 oder 98 %, besonders bevorzugt von mindestens 99 % oder 100 %, zu der Peptidsequenz gemäß SEQ ID NO: 12 aufweisen.

Weiterer Gegenstand der vorliegenden Erfindung sind daher auch feedback-resistente Varianten der Aspartat-Kinase LysC, die ausgehend von einem Polypeptid mit der Sequenz gemäß SEQ ID NO: 12 erhalten wurden, wobei diese vorzugsweise eine Sequenzidentität von mindestens 92 %, vorzugsweise mindestens 94, 96 oder 98 %, vor allem von mindestens 99 %, zu der Peptidsequenz gemäß SEQ ID NO: 12 aufweisen.

Weiterer Gegenstand der vorliegenden Erfindung sind daher auch feedback-resistente Varianten der Aspartat-Kinase LysC, ausgewählt aus der Gruppe:
a) Polypeptide mit einer Sequenzidentität von mindestens 70 %, vorzugsweise mindestens 80 oder 85 %, insbesondere mindestens 90, 92, 94 oder 96 %, vor allem mindestens 98 oder 99 %, zu der Polypeptidsequenz gemäß SEQ ID NO: 12, dadurch gekennzeichnet, dass sie mindestens eine der folgenden Mutationen aufweisen: D274Y, A279E, G359D;
b) Polypeptide mit einer Sequenzidentität von mindestens 92 %, vorzugsweise mindestens 94, 96 oder 98 %, zu der Peptidsequenz gemäß SEQ ID NO: 12, dadurch gekennzeichnet, dass sie mindestens eine der folgenden Mutationen aufweisen: S301Y, T308I, T3111.

Besonders bevorzugt handelt es sich bei den Varianten um Polypeptide mit einer Sequenz gemäß SEQ ID NO: 12 mit der Maßgabe, dass mindestens eine, vorzugsweise genau eine, der folgenden Mutationen vorliegt: D274Y, A279E, S301Y, T308I, T311I, G359D.

Weiterer Gegenstand der vorliegenden Erfindung sind auch Polynukleotide, die für erfindungsgemäße Polypeptide kodieren. Es handelt sich hierbei vorzugsweise um Polynukleotide, die eine Sequenzidentität von mindestens 70 %, vorzugsweise mindestens 80 oder 85 %, insbesondere mindestens 90 oder 95 %, zu der Sequenz von Position 301 bis 1566 gemäß SEQ ID NO: 11 aufweisen.

Weiterer Gegenstand der vorliegenden Erfindung sind entsprechend auch Vektoren, insbesondere Klonierungs- und Expressionsvektoren, die erfindungsgemäße Polynukleotide enthalten. Diese Vektoren können entsprechend in Mikroorganismen, insbesondere in coryneforme Bakterien, vor allem der Gattung Corynebacterium, oder Enterobacteriaceae, vor allem der Gattung Escherichia, eingebaut werden.

Erfindungsgemäße Polynukleotide können weiterhin zwecks Expression der kodierten Gene auch in das Genom von Mikroorganismen, insbesondere in das Genom von coryneformen Bakterien, insbesondere solchen der Gattung Corynebacterium, oder in das Genom von Enterobacteriaceaae, insbesondere solchen der Gattung Escherichia, eingebaut werden.

Weiterer Gegenstand der vorliegenden Erfindung sind entsprechend auch rekombinante Mikroorganismen, vorzugsweise Bakterien, insbesondere coryneforme Bakterien, vor allem solche der Gattung Corynebacterium, sowie Enterobacteriaceae, vor allem solche der Gattung Escherichia, die erfindungsgemäße Polypeptide und/oder erfindungsgemäße Polynukleotide und/oder erfindungsgemäße Vektoren enthalten.

Bevorzugt sind hierbei rekombinante Mikroorganismen, vorzugsweise Bakterien, insbesondere coryneforme Bakterien, vor allem solche der Gattung Corynebacterium, besonders bevorzugt der Art C. humireducens oder der Art C. glutamicum, die erfindungsgemäße feedback-resistente Aspartat-Kinasen und/oder für diese kodierende Polynukleotide und/oder solche Polynukleotide umfassende Vektoren enthalten.

Unter einem "rekombinanten Mikroorganismus" bzw. "rekombinanten Bakterium" ist erfindungsgemäß ein Mikroorganismus bzw. Bakterium zu verstehen, der/das mindestens einer gentechnischen Maßnahme unterzogen wurde. Bei der gentechnischen Maßnahme kann es sich hierbei insbesondere um eine zielgerichtete oder ungerichtete Mutation, den Einbau eines wirtsfremden Gens und/oder die Überexpression oder Abschwächung eines wirtseigenen oder wirtsfremden Gens handeln. Vorzugsweise zeichnet sich ein erfindungsgemäßer rekombinanter Mikroorganismus bzw. ein erfindungsgemäßes rekombinantes Bakterium durch die Überexpression oder Abschwächung mindestens eines Gens aus. In einer besonders bevorzugten Ausführungsform zeichnet sich ein erfindungsgemäßer Mikroorganismus bzw. ein erfindungsgemäßes Bakterium hierbei durch die Überexpression der erfindungsgemäßen, vorzugsweise feedback-resistenten, Aspartat-Kinase bzw. eines für diese kodierenden Polynukleotids aus.

Innerhalb der Gattung Corynebacterium werden erfindungsgemäß Stämme bevorzugt, die auf folgenden Arten beruhen: Corynebacterium efficiens, wie zum Beispiel der Typstamm DSM44549, Corynebacterium glutamicum, wie zum Beispiel der Typstamm ATCC13032 oder der Stamm R, Corynebacterium ammoniagenes, wie zum Beispiel der Stamm ATCC6871, Corynebacterium humireducens, wie zum Beispiel der Stamm DSM 45392, sowie Corynebacterium pekinese, wie zum Beispiel der Stamm CGMCC No. 5361.

Besonders bevorzugt sind die Arten Corynebacterium glutamicum und Corynebacterium humireducens. Sofern im Rahmen dieser Anmeldung vom Stamm Corynebacterium humireducens die Rede ist, handelt es sich vorzugsweise um den Stamm DSM 45392 oder um einen davon abgeleiteten Stamm.

Einige Vertreter der Art Corynebacterium glutamicum sind im Stand der Technik auch unter anderen Bezeichnungen bekannt. Hierzu gehören beispielsweise: Corynebacterium acetoacidophilum ATCC13870, Corynebacterium lilium DSM20137, Corynebacterium melassecola ATCC17965, Brevibacterium flavum ATCC14067, Brevibacterium lactofermentum ATCC13869 und Brevibacterium divaricatum ATCC14020. Der Begriff "Micrococcus glutamicus" für Corynebacterium glutamicum war ebenfalls gebräuchlich. Einige Vertreter der Art Corynebacterium efficiens wurden im Stand der Technik auch als Corynebacterium thermoaminogenes bezeichnet, wie zum Beispiel der Stamm FERM BP-1539.

Angaben zur taxonomischen Einordnung von Stämmen der Gruppe der coryneformen Bakterien findet man unter anderem bei Seiler (Journal of General Microbiology 129, 1433-1477 (1983)), Kinoshita (1985, Glutamic Acid Bacteria, p 115-142. In: Demain and Solomon (ed), Biology of Industrial Microorganisms. The Benjamin/Cummins Publishing Co., London, UK), Kämpfer und Kroppenstedt (Canadian Journal of Microbiology 42, 989-1005 (1996)), Liebl et al (International Journal of Systematic Bacteriology 41, 255-260 (1991)), Fudou et al (International Journal of Systematic and Evolutionary Microbiology 52, 1127-1131 (2002)) und in der US-A-5,250,434.

Stämme mit der Bezeichnung "ATCC" können von der American Type Culture Collection (Manassas, VA, USA) bezogen werden. Stämme mit der Bezeichnung "DSM" können von der Deutschen Sammlung von Mikroorganismen und Zellkulturen (DSMZ, Braunschweig, Deutschland) bezogen werden. Stämme mit der Bezeichnung "NRRL" können von der Agricultural Research Service Patent Culture Collection (ARS, Peoria, Illinois, US) bezogen werden. Stämme mit der Bezeichnung "FERM" können vom National Institute of Advanced Industrial Science and Technology (AIST Tsukuba Central 6, 1-1-1 Higashi, Tsukuba Ibaraki, Japan) bezogen werden.

Weiterer Gegenstand der vorliegenden Erfindung ist ebenso ein Verfahren zur Überproduktion von L-Lysin, in welchem ein erfindungsgemäßes L-Lysin überproduzierendes alkaliphiles Bakterium, insbesondere ein erfindungsgemäßes L-Lysin überproduzierendes alkaliphiles Corynebacterium, vor allem ein erfindungsgemäßer L-Lysin überproduzierender Corynebacterium humireducens-Stamm, eingesetzt wird.

Weiterer Gegenstand der vorliegenden Erfindung sind ebenso Verfahren zur Produktion bzw. Überproduktion von L-Lysin, in welchen eine erfindungsgemäße, vorzugsweise feedback-resistente, Aspartat-Kinase und/oder ein erfindungsgemäßes für eine Aspartat-Kinase kodierendes Polynukleotid und/oder ein erfindungsgemäßer Vektor eingesetzt wird. Der Einsatz erfolgt hierbei vorzugsweise in rekombinanten Corynebakterien, vorzugsweise in C. glutamicum oder C. humireducens. Die erfindungsgemäße, vorzugsweise feedback-resistente, Aspartat-Kinase und/oder das erfindungsgemäße Polynukleotid werden in erfindungsgemäßen Verfahren vorzugsweise überexprimiert.

Weiterer Gegenstand der vorliegenden Erfindung sind ebenso Verfahren zur Produktion bzw. Überproduktion von L-Lysin, in welchen ein erfindungsgemäßer rekombinanter Mikroorganismus bzw. ein erfindungsgemäßes rekombinantes Bakterium eingesetzt wird.

Unter "Überproduzieren" bzw. "Überproduktion" ist erfindungsgemäß in Bezug auf die L-Aminosäure zu verstehen, dass die Mikroorganismen die L-Aminosäure über ihren eigenen Bedarf hinaus produzieren und entweder in der Zelle anreichern oder in das sie umgebende Nährmedium ausscheiden und dort akkumulieren. Vorzugsweise sind die Mikroorganismen hierbei dazu in der Lage ≥ (mindestens) 0,25 g/l, ≥ 0,5 g/l, ≥ 1,0 g/l, ≥ 1,5 g/l, ≥ 2,0 g/l, ≥ 4 g/l oder ≥ 10 g/l der betreffenden L-Aminosäure in ≤ (maximal) 120 Stunden, ≤ 96 Stunden, ≤ 48 Stunden, ≤ 36 Stunden, ≤ 24 Stunden oder ≤ 12 Stunden in der Zelle oder im Nährmedium anzureichern bzw. zu akkumulieren.

Erfindungsgemäße rekombinante Mikroorganismen, in die erfindungsgemäße Polynukleotide und/oder erfindungsgemäße Vektoren eingebracht wurden, besitzen in einer bevorzugten Ausführungsform bereits vor dem Einbau der erfindungsgemäßen Polynukleotide und/oder Vektoren die Fähigkeit eine L-Aminosäure überzuproduzieren. Bei den Ausgangsstämmen handelt es sich bevorzugt um Stämme, die durch Mutagenese und Selektion, durch rekombinante DNA-Techniken oder durch eine Kombination beider Methoden hergestellt wurden.

Es ist offensichtlich und bedarf keiner weiteren Erklärungen, dass man zu einem erfindungsgemäßen rekombinanten Mikroorganismus auch dadurch gelangen kann, indem man einen Wildstamm, in welchem ein erfindungsgemäßes Polynukleotid und/oder ein erfindungsgemäßer Vektor enthalten ist oder eingebaut wurde, anschließend durch geeignete weitere genetische Maßnahmen dazu veranlasst, die L-Aminosäure zu produzieren bzw. die L-Aminosäure-Produktion zu erhöhen.

Bekannte Vertreter L-Lysin produzierender bzw. ausscheidender Stämme coryneformer Bakterien sind beispielsweise Corynebacterium glutamicum DM58-1/pDM6 (DSM4697, beschrieben in EP 0 358 940), Corynebacterium glutamicum MH20-22B (DSM16835, beschrieben in Menkel et al. (Applied and Environmental Microbiology 55(3), 684-688 (1989))), Corynebacterium glutamicum AHP-3 (Ferm BP-7382, beschrieben in EP 1 108 790), Corynebacterium glutamicum NRRL B-11474 (beschrieben in US 4,275,157), Corynebacterium glutamicum DSM13994 (beschrieben in US 6,783,967), Corynebacterium glutamicum DSM16834 (beschrieben in WO 06/063660), Corynebacterium glutamicum DSM17119 (beschrieben in WO 06/100211), Corynebacterium glutamicum DSM17223 (beschrieben in WO 06/125714), Corynebacterium glutamicum DSM16937 (beschrieben in WO 06/077004), und Corynebacterium thermoaminogenes AJ12521 (FERM BP-3304, beschrieben in US 5,250,423).

Weiterer Gegenstand der vorliegenden Erfindung sind auch weitere Polynukleotide aus C. humireducens sowie die durch diese Polynukleotide kodierten Polypeptide. Durch Überexpression der entsprechenden Polynukleotide bzw. Polypeptide kann die Produktion von L-Lysin positiv beeinflusst werden.

Gegenstand der vorliegenden Erfindung sind daher ebenso:
a) eine Dihydrodipicolinat-Synthase (DapA, EC 4.2.1.52) mit einer Sequenzidentität von mindestens 90, 95 oder 98 %, vorzugsweise 100 %, zu der Sequenz gemäß SEQ ID NO: 2 sowie für diese kodierende Polynukleotide,
b) eine Diaminopimelat-Dehydrogenase (Ddh, EC 1.4.1.16) mit einer Sequenzidentität von mindestens 90, 95 oder 98 %, vorzugsweise 100 %, zu der Sequenz gemäß SEQ ID NO: 36 sowie für diese kodierende Polynukleotide,
c) eine Aspartat-Semialdehyd-Dehydrogenase (Asd, EC 1.2.1.11) mit einer Sequenzidentität von mindestens 90, 95 oder 98 %, vorzugsweise 100 %, zu der Sequenz gemäß SEQ ID NO: 30 sowie für diese kodierende Polynukleotid,
d) eine Diaminopimelat-Decarboxylase (LysA, EC 4.1.1.20) mit einer Sequenzidentität von mindestens 90, 95 oder 98 %, vorzugsweise 100 %, zu der Sequenz gemäß SEQ ID NO: 4 sowie für diese kodierende Polynukleotide,
e) eine Aspartat-Aminotransferase (AaT, EC 2.6.1.1) mit einer Sequenzidentität von mindestens 90, 95 oder 98 %, vorzugsweise 100 %, zu der Sequenz gemäß SEQ ID NO: 6 sowie für diese kodierende Polynukleotide,
f) ein L-Lysin-Exporter (LysE, Lysin-Efflux-Permease) mit einer Sequenzidentität von mindestens 90, 95 oder 98 %, vorzugsweise 100 %, zu der Sequenz gemäß SEQ ID NO: 8 sowie für diesen kodierende Polynukleotide,
g) eine Pyruvat-Carboxylase (Pyc, EC 6.4.1.1) mit einer Sequenzidentität von mindestens 90, 95 oder 98 %, vorzugsweise 100 %, zu der Sequenz gemäß SEQ ID NO: 10 sowie für diese kodierende Polynukleotide,
h) eine Dihydropicolinat-Reduktase (DapB, EC 1.3.1.26) mit einer Sequenzidentität von mindestens 90, 95 oder 98 %, vorzugsweise 100 %, zu der Sequenz gemäß SEQ ID NO: 14 sowie für diese kodierende Polynukleotide,
i) eine Glucose-6-phosphat-Dehydrogenase (EC 1.1.1.49) mit einer Sequenzidentität von mindestens 90, 95 oder 98 %, vorzugsweise 100 %, zu der Sequenz gemäß SEQ ID NO: 16 sowie für diese kodierende Polynukleotide,
j) die Zwf-Untereinheit einer Glucose-6-phosphat-Dehydrogenase (Zwf, EC 1.1.1.49) mit einer Sequenzidentität von mindestens 90, 95 oder 98 %, vorzugsweise 100 %, zu der Sequenz gemäß SEQ ID NO: 32 sowie für diese kodierende Polynukleotide,
k) die OpcA-Untereinheit einer Glucose-6-phosphat-Dehydrogenase (OpcA, EC 1.1.1.49) mit einer Sequenzidentität von mindestens 90, 95 oder 98 %, vorzugsweise 100 %, zu der Sequenz gemäß SEQ ID NO: 34 sowie für diese kodierende Polynukleotide,
l) eine Phosphogluconsäure-Dehydrogenase (Gnd, EC 1.1.1.44) mit einer Sequenzidentität von mindestens 90, 95 oder 98 %, vorzugsweise 100 %, zu der Sequenz gemäß SEQ ID NO: 18 sowie für diese kodierende Polynukleotide,

Weiterer Gegenstand der vorliegenden Erfindung sind ebenso Vektoren, die die zuvor genannten Polynukleotide enthalten, sowie rekombinanten Mikroorganismen, die die zuvor genannten Enzyme und/oder Polynukleotide und/oder Vektoren enthalten. Das betreffende Polypeptid und/oder Polynukleotid liegt hierbei in einer bevorzugten Ausführungsform in dem Mikroorganismus in überexprimierter Form vor. Bei den rekombinanten Mikroorganismen handelt es sich hierbei vorzugsweise um coryneforme Bakterien, vor allem um Corynebakterien, insbesondere solchen der Art C. humireducens oder C. glutamicum.

Weiterer Gegenstand der vorliegenden Erfindung ist daher auch ein Verfahren zur Überproduktion von L-Lysin, in welchem mindestens eines, vorzugsweise mindestens zwei, drei oder vier, der genannten Polynukleotide in überexprimierter Form vorliegen, wobei das Verfahren vorzugsweise in Corynebakterien, insbesondere solche der Art. C. humireducens oder C. glutamicum, durchgeführt wird.

Weiterer Gegenstand der vorliegenden Erfindung sind auch weitere Polynukleotide aus C. humireducens sowie die durch diese Polynukleotide kodierten Polypeptide. Durch Ausschaltung oder Abschwächung der entsprechenden Polynukleotide bzw. Polypeptide kann die Produktion von L-Lysin positiv beeinflusst werden.

Gegenstand der vorliegenden Erfindung sind daher ebenso:
a) eine Malat-Chinon-Oxidoreduktase (Mqo, EC 1.1.99.16) mit einer Sequenzidentität von mindestens 90, 95 oder 98 %, vorzugsweise 100 %, zu der Sequenz gemäß SEQ ID NO: 20 sowie für diese kodierende Polynukleotide,
b) die E1p-Untereinheit eines Pyruvat-Dehydrogenase-Komplexes (AceE, EC 1.2.4.1) mit einer Sequenzidentität von mindestens 90, 95 oder 98 %, vorzugsweise 100 %, zu der Sequenz gemäß SEQ ID NO: 22 sowie für diese kodierende Polynukleotide,
c) eine Citrat-Synthase (GltA, EC 4.1.3.7) mit einer Sequenzidentität von mindestens 90, 95 oder 98 %, vorzugsweise 100 %, zu der Sequenz gemäß SEQ ID NO: 24 sowie für diese kodierende Polynukleotide,
d) eine Malat-Dehydrogenase (Mdh, EC 1.1.1.37) mit einer Sequenzidentität von mindestens 90, 95 oder 98 %, vorzugsweise 100 %, zu der Sequenz gemäß SEQ ID NO: 26 sowie für diese kodierende Polynukleotide,
e) eine UDP-N-acetylmuramoylalanyl-D-glutamat-2,6-diaminopimelat-Ligase, 6-Diaminopimelat-Ligase (MurE, EC 6.3.2.13) mit einer Sequenzidentität von mindestens 90, 95 oder 98 %, vorzugsweise 100 %, zu der Sequenz gemäß SEQ ID NO: 28 sowie für diese kodierende Polynukleotide.

Weiterer Gegenstand der vorliegenden Erfindung sind ebenso Vektoren, die die zuvor genannten Polynukleotide enthalten, sowie rekombinanten Mikroorganismen, die die zuvor genannten Enzyme und/oder Polynukleotide und/oder Vektoren enthalten. Das betreffende Polypeptid und/oder Polynukleotid liegt hierbei in einer bevorzugten Ausführungsform in dem Mikroorganismus in ausgeschalteter oder abgeschwächter Form vor. Bei den rekombinanten Mikroorganismen handelt es sich hierbei vorzugsweise um coryneforme Bakterien, vor allem um Corynebakterien, insbesondere solche der Art C. humireducens oder C. glutamicum, vor allem der Art C. humireducens.

Weiterer Gegenstand der vorliegenden Erfindung ist daher auch ein Verfahren zur Überproduktion von L-Lysin, in welchem mindestens eines, vorzugsweise mindestens zwei, drei oder vier, der genannten Polynukleotide in ausgeschalteter oder abgeschwächter Form vorliegen, wobei das Verfahren vorzugsweise in Corynebakterien, insbesondere solchen der Art. C. humireducens oder C. glutamicum, durchgeführt wird. In einer bevorzugten Ausführungsform liegt hierbei gleichzeitig mindestens eines, vorzugsweise mindestens zwei, drei oder vier, der in der zuvor aufgeführten Liste genannten Polynukleotide in überexprimierter Form vor.

In einer bevorzugten Ausführungsform weisen erfindungsgemäße Mikroorganismen oder Bakterien, insbesondere erfindungsgemäße Corynebakterien, vor allem erfindungsgemäße Corynebakterien der Art C. humireducens oder C. glutamicum, insbesondere erfindungsgemäße L-Lysin-Überproduktionsstämme, neben der Überexpression eines erfindungsgemäßen lysC-Gens, vorzugsweise lysC^{FBR}-Gens, entsprechend mindestens eines, vorzugsweise mindestens 2 oder 3, besonders bevorzugt mindestens 4 oder 5, der folgenden Merkmale auf:
a) ein überexprimiertes Polynukleotid (dapA-Gen), das für eine Dihydrodipicolinat-Synthase (DapA, EC 4.2.1.52), vorzugsweise für eine Dihydrodipicolinat-Synthase mit einer Sequenzidentität von mindestens 90, 95 oder 98 %, vorzugsweise 100 %, zu der Sequenz gemäß SEQ ID NO: 2, kodiert,
b) ein überexprimiertes Polynukleotid (ddh), das für eine Diaminopimelat-Dehydrogenase (Ddh, EC 1.4.1.16), vorzugsweise für eine Diaminopimelat-Dehydrogenase mit einer Sequenzidentität von mindestens 90, 95 oder 98 %, vorzugsweise 100 %, zu der Sequenz gemäß SEQ ID NO: 36 kodiert,
c) ein überexprimiertes Polynukleotid (asd-Gen), das für eine Aspartat-Semialdehyd-Dehydrogenase (Asd, EC 1.2.1.11), vorzugsweise für eine eine Aspartat-Semialdehyd-Dehydrogenase mit einer Sequenzidentität von mindestens 90, 95 oder 98 %, vorzugsweise 100 %, zu der Sequenz gemäß SEQ ID NO: 30, kodiert,
d) ein überexprimiertes Polynukleotid (lysA-Gen), das für eine Diaminopimelat-Decarboxylase (LysA, EC 4.1.1.20), vorzugsweise für eine Diaminopimelat-Decarboxylase mit einer Sequenzidentität von mindestens 90, 95 oder 98 %, vorzugsweise 100 %, zu der Sequenz gemäß SEQ ID NO: 4, kodiert,
e) ein überexprimiertes Polynukleotid (aat-Gen), das für eine Aspartat-Aminotransferase (AaT, EC 2.6.1.1), vorzugsweise für eine Aspartat-Aminotransferase mit einer Sequenzidentität von mindestens 90, 95 oder 98 %, vorzugsweise 100 %, zu der Sequenz gemäß SEQ ID NO: 6, kodiert,
f) ein überexprimiertes Polynukleotid (lysE-Gen), das für einen L-Lysin-Exporter (LysE, Lysin-Efflux-Permease), vorzugsweise für einen L-Lysin-Exporter mit einer Sequenzidentität von mindestens 90, 95 oder 98 %, vorzugsweise 100 %, zu der Sequenz gemäß SEQ ID NO: 8, kodiert,
g) ein überexprimiertes Polynukleotid (pyc-Gen), das für eine Pyruvat-Carboxylase (Pyc, EC 6.4.1.1), vorzugsweise für eine Pyruvat-Carboxylase mit einer Sequenzidentität von mindestens 90, 95 oder 98 %, vorzugsweise 100 %, zu der Sequenz gemäß SEQ ID NO: 10, kodiert,
h) ein überexprimiertes Polynukleotid (dapF-Gen), das für eine Diaminopimelat-Epimerase (DapF, EC 5.1.1.7) kodiert,
i) ein überexprimiertes Polynukleotid (dapB-Gen), das für eine Dihydropicolinat-Reduktase (DapB, EC 1.3.1.26), vorzugsweise für eine Dihydropicolinat-Reduktase mit einer Sequenzidentität von mindestens 90, 95 oder 98 %, vorzugsweise 100 %, zu der Sequenz gemäß SEQ ID NO: 14, kodiert,
j) ein überexprimiertes Polynukleotid, das für eine Glucose-6-phosphat-Dehydrogenase (EC 1.1.1.49), vorzugsweise für eine Glucose-6-phosphat-Dehydrogenase mit einer Sequenzidentität von mindestens 90, 95 oder 98 %, vorzugsweise 100 %, zu der Sequenz gemäß SEQ ID NO: 16, kodiert,
k) ein überexprimiertes Polynukleotid (zwf-Gen), das für die Zwf-Untereinheit einer Glucose-6-phosphat-Dehydrogenase (Zwf, EC 1.1.1.49), vorzugsweise für eine Zwf-Untereinheit mit einer Sequenzidentität von mindestens 90, 95 oder 98 %, vorzugsweise 100 %, zu der Sequenz gemäß SEQ ID NO: 32, kodiert,
l) ein überexprimiertes Polynukleotid (opcA-Gen), das für die OpcA-Untereinheit einer Glucose-6-phosphat-Dehydrogenase (OpcA, EC 1.1.1.49), vorzugsweise für eine OpcA-Untereinheit mit einer Sequenzidentität von mindestens 90, 95 oder 98 %, vorzugsweise 100 %, zu der Sequenz gemäß SEQ ID NO: 34, kodiert,
m) ein überexprimiertes Polynukleotid (gnd-Gen), das für eine Phosphogluconsäure-Dehydrogenase (Gnd, EC 1.1.1.44), vorzugsweise für eine Phosphogluconsäure-Dehydrogenase mit einer Sequenzidentität von mindestens 90, 95 oder 98 %, vorzugsweise 100 %, zu der Sequenz gemäß SEQ ID NO: 18, kodiert,
n) ein ausgeschaltetes oder abgeschwächtes Polynukleotid (mqo), das für eine Malat-Chinon-Oxidoreduktase (Mqo, EC 1.1.99.16), vorzugsweise für eine Malat-Chinon-Oxidoreduktase mit einer Sequenzidentität von mindestens 90, 95 oder 98 %, vorzugsweise 100 %, zu der Sequenz gemäß SEQ ID NO: 20, kodiert,
o) ein ausgeschaltetes oder abgeschwächtes Polynukleotid, das für die E1 p-Untereinheit eines Pyruvat-Dehydrogenase-Komplexes (AceE, EC 1.2.4.1), vorzugsweise für eine E1p-Untereinheit mit einer Sequenzidentität von mindestens 90, 95 oder 98 %, vorzugsweise 100 %, zu der Sequenz gemäß SEQ ID NO: 22, kodiert,
p) ein ausgeschaltetes oder abgeschwächtes Polynukleotid (gltA), das für eine Citrat-Synthase (GltA, EC 4.1.3.7), vorzugsweise für eine Citrat-Synthase mit einer Sequenzidentität von mindestens 90, 95 oder 98 %, vorzugsweise 100 %, zu der Sequenz gemäß SEQ ID NO: 24, kodiert,
q) ein ausgeschaltetes oder abgeschwächtes Polynukleotid (mdh), das für eine Malat-Dehydrogenase (Mdh, EC 1.1.1.37), vorzugsweise für eine Malat-Dehydrogenase mit einer Sequenzidentität von mindestens 90, 95 oder 98 %, vorzugsweise 100 %, zu der Sequenz gemäß SEQ ID NO: 26, kodiert,
r) ein ausgeschaltetes oder abgeschwächtes Polynukleotid (murE), das für eine UDP-N-acetylmuramoylalanyl-D-glutamat-2,6-diaminopimelat-Ligase, 6-Diaminopimelat-Ligase (MurE, EC 6.3.2.13), vorzugsweise für ein Enzym mit einer Sequenzidentität von mindestens 90, 95 oder 98 %, vorzugsweise 100 %, zu der Sequenz gemäß SEQ ID NO: 28, kodiert.

Weiterer Gegenstand der vorliegenden Erfindung ist entsprechend auch ein Verfahren zur Überproduktion von L-Lysin, in welchem ein derartiger Mikroorganismus bzw. ein derartiges Bakterium eingesetzt wird.

Die zuvor genannten, in erfindungsgemäßen Verfahren eingesetzten bzw. einzusetzenden Polynukleotide und Polypeptide stammen vorzugsweise aus Corynebakterien, insbesondere aus C. glutamicum oder C. humireducens, besonders bevorzugt aus C. humireducens.

Unter "Überexpression" ist erfindungsgemäß allgemein eine Erhöhung der intrazellulären Konzentration oder Aktivität einer Ribonukleinsäure, eines Proteins (Polypeptids) oder eines Enzyms, die durch eine entsprechende DNA kodiert werden, in einem Mikroorganismus im Vergleich zum Ausgangsstamm (Elternstamm) oder Wildtypstamm zu verstehen. Unter einem Ausgangsstamm (Elternstamm) versteht man den Stamm, an dem die zur Überexpression führende Maßnahme durchgeführt wurde.

Die Erhöhung der Konzentration oder Aktivität lässt sich beispielsweise dadurch erzielen, dass man die Kopienzahl der entsprechenden kodierenden Polynukleotide chromosomal oder extrachromosomal um mindestens eine Kopie erhöht.

Eine weit verbreitete Methode zur Erhöhung der Kopienzahl besteht darin, dass man das entsprechende kodierende Polynukleotid in einen Vektor, bevorzugt ein Plasmid, einbaut, der von einem Mikroorganismuns, insbesondere einem coryneformen Bakterium, repliziert wird. Weiterhin kann man als Vektoren Transposons, Insertionselemente (IS-Elemente) oder Phagen einsetzen. Im Stand der Technik ist eine Fülle geeigneter Vektoren beschrieben.

Eine andere verbreitete Methode zur Erzielung einer Überexpression ist das Verfahren der chromosomalen Genamplifikation. Bei dieser Methode wird mindestens eine zusätzliche Kopie des interessierenden Polynukleotids in das Chromosom eines coryneformen Bakteriums eingefügt. Derartige Amplifikationsverfahren sind beispielsweise in der WO 03/014330 oder WO 03/040373 beschrieben.

Eine weitere Methode zur Erzielung einer Überexpression besteht darin, das entsprechende Gen beziehungsweise Allel in funktioneller Weise (operably linked) mit einem Promotor beziehungsweise einer Expressionskassette zu verknüpfen. Geeignete Promotoren für Corynebacterium glutamicum sind beispielsweise in der Fig. 1 des Übersichtsartikel von Patek et al. (Journal of Biotechnology 104(1-3), 311-323 (2003)) und in zusammenfassenden Darstellungen wie dem "Handbook of Corynebacterium glutamicum" (Eds.: Lothar Eggeling und Michael Bott, CRC Press, Boca Raton, US (2005)) oder dem Buch "Corynebacteria, Genomics and Molecular Biology" (Ed.: Andreas Burkovski, Caister Academic Press, Norfolk, UK (2008)) beschrieben. In gleicher Weise können die von Vasicova et al (Journal of Bacteriology 181, 6188-6191 (1999)) beschriebenen Varianten des dapA-Promotors, beispielsweise der Promotor A25 eingesetzt werden. Weiterhin kann der gap-Promotor von Corynebacterium glutamicum (EP 06007373) verwendet werden. Schließlich können die hinlänglich bekannten von Amann et al. (Gene 69(2), 301-315 (1988)) und Amann und Brosius (Gene 40(2-3), 183-190 (1985)) beschriebenen Promotoren T3, T7, SP6, M13, lac, tac und trc verwendet werden. Ein derartiger Promotor kann beispielsweise stromaufwärts des betreffenden Gens, typischerweise im Abstand von ungefähr 1 - 500 Nukleobasen vom Startkodon, eingefügt werden.

Durch die Maßnahme der Überexpression wird die Aktivität oder Konzentration des entsprechenden Polypeptids vorzugsweise um mindestens 10%, 25%, 50%, 75%, 100%, 150%, 200%, 300%, 400% oder 500%, maximal bis vorzugsweise 1000% oder 2000%, bezogen auf die Aktivität oder Konzentration des Polypeptids im Stamm vor der zur Überexpression führenden Maßnahme, erhöht.

Die Konzentration eines Proteins kann über 1- und 2-dimensionale Proteingelauftrennung und anschließende optische Identifizierung der Proteinkonzentration mit entsprechender Auswertesoftware im Gel bestimmt werden. Eine gebräuchliche Methode zur Präparation der Proteingele bei coryneformen Bakterien und zur Identifizierung der Proteine ist die von Hermann et al. (Electrophoresis, 22:1712-23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit entsprechender Software zur Konzentrationsbestimmung (Lohaus und Meyer (1998) Biospektrum 5:32-39; Lottspeich, Angewandte Chemie 38: 2630-2647 (1999)) bestimmt werden. Die Aktivität kann mit Hilfe eines geeigneten Enzymtest bestimmt werden.

Der Begriff "Abschwächung" bezeichnet erfindungsgemäß die Verringerung der intrazellulären Konzentration oder Aktivität einer Ribonukleinsäure, eines Proteins (Polypeptids) oder eines Enzyms, die durch eine entsprechende DNA kodiert werden, in einem Mikroorganismus, im Vergleich zum Ausgangsstamm (Elternstamm) oder Wildtypstamm. Als Ausgangsstamm (Elternstamm) wird der Stamm bezeichnet, an dem die Maßnahme der Abschwächung durchgeführt wurde.

Die Abschwächung kann erzielt werden, indem die Expression eines Polypeptids, beispielsweise durch Verwendung eines schwachen Promotors, verringert wird oder indem ein Allel verwendet wird, das für ein Polypeptid mit einer niedrigeren Aktivität kodiert und gegebenenfalls diese Maßnahmen kombiniert. Die Abschwächung kann auch dadurch erreicht werden, indem die Expression des Polypeptids vollständig unterbunden wird, beispielsweise dadurch, dass das kodierende Gen ausgeschaltet wird.

Durch die Maßnahme der Abschwächung wird die Aktivität oder Konzentration des entsprechenden Polypeptids vorzugsweise um mindestens 10%, 25%, 50% oder 75%, maximal um 100%, bezogen auf die Aktivität oder Konzentration des Polypeptids im Stamm vor der zur Abschwächung führenden Maßnahme, reduziert. In einer bevorzugten Ausführungsform besteht die Abschwächung in der vollständigen Ausschaltung der Expression des betreffenden Polypeptids.

Hinsichtlich weiterer bevorzugter Eigenschaften des erfindungsgemäßen L-Lysin überproduzierenden C. humireducens-Stamms wird auf die oben zitierte Veröffentlichung von Wu et al. (2011) sowie die weiteren oben genannten Veröffentlichungen verwiesen.

Erfindungsgemäße Mikroorganismen, insbesondere Bakterien der Gattung Corynebacterium, können kontinuierlich - wie beispielsweise in der WO 05/021772 beschrieben- oder diskontinuierlich im Batch-Verfahren (Satzkultivierung bzw. Satzverfahren) oder im Fed-Batch- (Zulaufverfahren) oder Repeated-Fed-Batch Verfahren (repetitives Zulaufverfahren) zum Zwecke der Produktion von L-Lysin kultiviert werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium beziehungsweise Fermentationsmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium und Fermentationsmedium beziehungsweise Medium sind gegenseitig austauschbar.

Als Kohlenstoffquelle können Zucker und Kohlenhydrate wie z.B. Glucose, Saccharose, Lactose, Fructose, Maltose, Melasse, Saccharose-haltige Lösungen aus der Zuckerrüben- oder Zuckerrohrherstellung, Stärke, Stärkehydrolysat und Cellulose, Öle und Fette, wie zum Beispiel Sojaöl, Sonnenblumenöl, Erdnussöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure oder Milchsäure verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Das Kulturmedium muss weiterhin Salze beispielsweise in Form von Chloriden oder Sulfaten von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine beispielsweise Thiamin, Biotin oder Pantothensäure zusätzlich zu den oben genannten Stoffen eingesetzt werden.

Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak beziehungsweise Ammoniakwasser oder saure Verbindungen wie Phosphorsäure oder Schwefelsäure in geeigneter Weise eingesetzt. Der pH wird im Allgemeinen auf einen Wert von 6,0 bis 9,0 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete, selektiv wirkende Stoffe, wie zum Beispiel Antibiotika hinzugefügt werden. Um aerobe Bedingungen aufrechtzuerhalten, werden Sauerstoff oder Sauerstoff-haltige Gasmischungen, wie zum Beispiel Luft in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Druck von 0,03 bis 0,2 MPa, gefahren. Die Temperatur der Kultur liegt normalerweise bei 20°C bis 45°C und vorzugsweise bei 25°C bis 40°C. Bei batch-Verfahren wird die Kultivierung solange fortgesetzt, bis sich ein Maximum der gewünschten L-Aminosäure, gebildet hat. Dieses Ziel wird normalerweise innerhalb von 10 Stunden bis 160 Stunden erreicht. Bei kontinuierlichen Verfahren sind längere Kultivierungszeiten möglich. Durch die Tätigkeit der Bakterien kommt es zu einer Anreicherung (Akkumulation) der L-Aminosäure im Fermentationsmedium und/oder in den Bakterienzellen.

Beispiele für geeignete Fermentationsmedien finden sich unter anderem in den Patentschriften 5,770,409, US 5,840,551 und US 5,990,350 oder US 5,275,940.

Die Analyse von L-Aminosäuren zur Bestimmung der Konzentration zu einem oder mehreren Zeitpunkt(en) im Verlauf der Fermentation kann durch Trennung der L-Aminosäuren mittels Ionenaustauschchromatographie vorzugsweise Kationenaustauschchromatographie mit anschließender Nachsäulenderivatisierung unter Verwendung von Ninhydrin erfolgen, so wie bei Spackman et al. (Analytical Chemistry 30: 1190-1206 (1958)) beschrieben. Anstelle von Ninhydrin kann auch ortho-Phtadialdehyd zur Nachsäulenderivatisierung eingesetzt werden. Einen Übersichtsartikel zur Ionenaustauschchromatographie findet man bei Pickering (LC·GC (Magazine of Chromatographic Science) 7(6), 484-487 (1989)).

Es ist ebenfalls möglich eine Vorsäulenderivatisierung beispielsweise unter Verwendung von ortho-Phtadialdehyd oder Phenylisothiocyanat vorzunehmen und die entstandenen Aminosäurederivate durch Reversed-Phase-Chromatographie (RP) vorzugsweise in Form der Hochleistungsflüssigkeits-chromatographie (HPLC) aufzutrennen. Eine derartige Methode ist beispielsweise bei Lindroth et al. (Analytical Chemistry 51: 1167-1174 (1979)) beschrieben.

Die Detektion erfolgt photometrisch (Absorption, Fluoreszenz).

Eine zusammenfassende Darstellung zur Aminosäureanalyse findet man unter anderem im Lehrbuch "Bioanalytik" von Lottspeich und Zorbas (Spektrum Akademischer Verlag, Heidelberg, Deutschland 1998).

Gegenstand der Erfindung ist dementsprechend auch ein Verfahren zur Herstellung von L-Lysin, dadurch gekennzeichnet, dass man folgende Schritte durchführt:
a) Fermentation erfindungsgemäßer Mikroorganismen, insbesondere coryneformer Bakterien, bevorzugt der Gattung Corynebacterium, besonders bevorzugt der Art Corynebacterium glutamicum oder Corynebacterium humireducens, in einem geeignetem Nährmedium, und
b) Akkumulation des L-Lysins in dem Nährmedium und/oder in den Zellen der genannten Bakterien.

Anschließend erfolgt die Bereitstellung bzw. Herstellung oder Gewinnung eines L-Lysin haltigen Produktes in flüssiger oder fester Form.

Durch die Maßnahmen der Fermentation erhält man eine Fermentationsbrühe, welche L-Lysin enthält.

Unter einer Fermentationsbrühe versteht man ein Fermentationsmedium bzw. Nährmedium, in dem ein Mikroorganismus für eine gewisse Zeit und bei einer gewissen Temperatur kultiviert wurde. Das Fermentationsmedium beziehungsweise die während der Fermentation eingesetzten Medien enthält/enthalten sämtliche Substanzen beziehungsweise Komponenten, die eine Vermehrung des Mikroorganismus und eine Bildung der gewünschten L-Aminosäure sicherstellen.

Bei Abschluss der Fermentation enthält die entstandene Fermentationsbrühe dementsprechend
a) die infolge der Vermehrung der Zellen des Mikroorganismus entstandene Biomasse (Zellmasse) des Mikroorganismus,
b) das im Laufe der Fermentation gebildete L-Lysin,
c) die im Laufe der Fermentation gebildeten organischen Nebenprodukte, und
d) die durch die Fermentation nicht verbrauchten Bestandteile des eingesetzten Fermentationsmediums beziehungsweise der Einsatzstoffe wie beispielsweise Vitamine wie Biotin oder Salze wie Magnesiumsulfat.

Zu den organischen Nebenprodukten gehören Stoffe, die von den bei der Fermentation eingesetzten Mikroorganismen neben dem L-Lysin erzeugt und gegebenenfalls ausgeschieden werden. Hierzu gehören auch Zucker wie zum Beispiel Trehalose.

Die Fermentationsbrühe wird dem Kulturgefäß beziehungsweise dem Fermentationsbehälter entnommen, gegebenenfalls gesammelt, und dazu verwendet, ein L-Lysin haltiges Produkt in flüssiger oder fester Form bereitzustellen. Hierfür wird auch der Ausdruck "Gewinnen des L-Lysin haltigen Produktes" verwendet. Im einfachsten Fall stellt die L-Lysin-haltige Fermentationsbrühe selbst das gewonnene Produkt dar.

Durch eine oder mehrere der Maßnahmen ausgewählt aus der Gruppe
a) teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%) Entfernung des Wassers,
b) teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%) Entfernung der Biomasse, wobei diese gegebenenfalls vor der Entfernung inaktiviert wird,
c) teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%, ≥ 99,3%, ≥ 99,7%) Entfernung der im Laufe der Fermentation gebildeten organischen Nebenprodukte, und
d) teilweise (> 0%) bis vollständige (100%) oder nahezu vollständige (≥ 80%, ≥ 90%, ≥ 95%, ≥ 96%, ≥ 97%, ≥ 98%, ≥ 99%, ≥ 99,3%, ≥ 99,7%) Entfernung der durch die Fermentation nicht verbrauchten Bestandteile des eingesetzten Fermentationsmediums beziehungsweise der Einsatzstoffe,

aus der Fermentationsbrühe erzielt man eine Konzentrierung bzw. Reinigung des L-Lysins. Auf diese Weise werden Produkte isoliert, die einen gewünschten Gehalt an L-Lysin aufweisen.

Die teilweise (> 0% bis < 80%) bis vollständige (100%) oder nahezu vollständige (≥ 80% bis < 100%) Entfernung des Wassers (Maßnahme a)) wird auch als Trocknung bezeichnet.

Durch vollständige oder nahezu vollständige Entfernung des Wassers, der Biomasse, der organischen Nebenprodukte und der nicht verbrauchten Bestandteile des eingesetzten Fermentationsmediums gelangt man zu reinen ((≥ 80 Gew.-%, ≥ 90 Gew.-%) oder hochreinen (≥ 95 Gew.-%, ≥ 97 Gew.-%, ≥ 99% Gew.-%) Produktformen des L-Lysins. Für die Maßnahmen gemäß a), b), c) oder d) sind im Stand der Technik eine Fülle von technischen Anleitungen verfügbar.

Im Falle der Aminosäure L-Lysin sind im Stand der Technik im Wesentlichen vier verschiedene Produktformen bekannt.

Eine Gruppe L-Lysin-haltiger Produkte umfasst konzentrierte, wässrige, alkalische Lösungen von aufgereinigtem L-Lysin (EP-B-0534865). Eine weitere Gruppe, wie beispielsweise in der US 6,340,486 und US 6,465,025 beschrieben, umfasst wässrige, saure, Biomasse-haltige Konzentrate von L-Lysin-haltigen Fermentationsbrühen. Die bekannteste Gruppe fester Produkte umfasst pulverförmige oder kristalline Formen von aufgereinigtem beziehungsweise reinem L-Lysin, das typischerweise in Form eines Salzes wie zum Beispiel L-Lysin-Monohydrochlorid vorliegt. Eine weitere Gruppe fester Produktformen ist beispielsweise in der EP-B-0533039 beschrieben. Die dort beschriebene Produktform enthält neben L-Lysin den größten Teil der während der fermentativen Herstellung verwendeten und nicht verbrauchten Einsatzstoffe und gegebenfalls die Biomasse des eingesetzten Mikroorganismus mit einem Anteil von >0% - 100%.

Entsprechend der verschiedenen Produktformen kennt man verschiedenste Verfahren, bei denen aus der Fermentationsbrühe das L-Lysin-haltige Produkt oder das gereinigte L-Lysin hergestellt wird.

Zur Herstellung von festem, reinen L-Lysin werden im wesentlichen Methoden der Ionenaustauschchromatographie gegebenfalls unter Verwendung von Aktivkohle und Methoden der Kristallisierung angewendet. Auf diese Weise erhält man die entsprechende Base oder ein entsprechendes Salz wie beispielsweise das Monohydrochlorid (Lys-HCl) oder das Lysinsulfat (Lys₂-H₂SO₄).

In der EP-B-0534865 ein Verfahren zur Herstellung wässriger, basischer L-Lysin-haltiger Lösungen aus Fermentationsbrühen beschrieben. Bei dem dort beschriebenen Verfahren wird die Biomasse aus der Fermentationsbrühe abgetrennt und verworfen. Mittels einer Base wie beispielsweise Natrium-, Kalium- oder Ammoniumhydroxid wird ein pH-Wert zwischen 9 bis 11 eingestellt. Die mineralischen Bestandteile (anorganischen Salze) werden nach Konzentrierung und Abkühlung durch Kristallisation aus der Brühe abgetrennt und entweder als Dünger verwendet oder verworfen.

Bei Verfahren zur Herstellung von Lysin unter Verwendung der erfindungsgemäßen Bakterien werden solche Verfahren bevorzugt, bei denen Produkte erhalten werden, die Bestandteile der Fermentationsbrühe enthalten. Diese werden insbesondere als Tierfuttermitteladditive verwendet.

Je nach Anforderung kann die Biomasse ganz oder teilweise durch Separationsmethoden wie z.B. der Zentrifugation, der Filtration, dem Dekantieren oder einer Kombination hieraus aus der Fermentationsbrühe entfernt oder vollständig in ihr belassen werden. Gegebenenfalls wird die Biomasse beziehungsweise die Biomasse enthaltene Fermentationsbrühe während eines geeigneten Verfahrensschrittes inaktiviert beispielsweise durch thermische Behandlung (Erhitzen) oder durch Säurezugabe.

In einer Verfahrensweise wird die Biomasse vollständig oder nahezu vollständig entfernt, sodass keine (0 %) oder höchstens 30%, höchstens 20%, höchstens 10%, höchstens 5%, höchstens 1% oder höchstens 0,1% Biomasse im hergestellten Produkt verbleibt. In einer weiteren Verfahrensweise wird die Biomasse nicht oder nur in geringfügigen Anteilen entfernt, sodass sämtliche (100%) oder mehr als 70%, 80%, 90%, 95%, 99% oder 99,9% Biomasse im hergestellten Produkt verbleibt. In einem erfindungsgemäßen Verfahren wird dementsprechend die Biomasse in Anteilen ≥ 0% bis ≤ 100% entfernt.

Schließlich kann die nach der Fermentation erhaltene Fermentationsbrühe vor oder nach der vollständigen oder teilweisen Entfernung der Biomasse mit einer anorganischen Säure wie beispielsweise Salzsäure, Schwefelsäure oder Phosphorsäure oder organischen Säure wie beispielsweise Propionsäure auf einen sauren pH Wert eingestellt werden (GB 1,439,728 oder EP 1 331 220). Es ist gleichfalls möglich die Fermentationsbrühe mit der vollständig enthaltenen Biomasse anzusäuern. Schließlich kann die Brühe auch durch Zusatz von Natriumbisulfit (NaHSO₃, GB 1,439,728) oder einem anderen Salz beispielsweise Ammonium-, Alkali- oder Erdalkalisalz der schwefligen Säure stabilisiert werden.

Bei der Abtrennung der Biomasse werden gegebenenfalls in der Fermentationsbrühe enthaltene organische oder anorganische Feststoffe teilweise oder ganz entfernt. Die in der Fermentationsbrühe gelösten organischen Nebenprodukte und die gelösten nicht verbrauchten Bestandteile des Fermentationsmediums (Einsatzstoffe) bleiben mindestens teilweise (> 0%), bevorzugt zu mindestens 25%, besonders bevorzugt zu mindestens 50% und ganz besonders bevorzugt zu mindestens 75% im Produkt. Gegebenenfalls bleiben diese auch vollständig (100%) oder nahezu vollständig, das heißt > 95% oder > 98% oder größer 99%, im Produkt. Enthält ein Produkt in diesem Sinn mindestens einen Teil der Bestandteile der Fermentationsbrühe, so wird dies auch mit dem Begriff "Produkt auf Fermentationsbrühebasis" umschrieben.

Anschließend wird der Brühe mit bekannten Methoden wie z.B. mit Hilfe eines Rotationsverdampfers, Dünnschichtverdampfers, Fallfilmverdampfers, durch Umkehrosmose oder durch Nanofiltration Wasser entzogen beziehungsweise eingedickt oder konzentriert. Diese aufkonzentrierte Fermentationsbrühe kann anschließend durch Methoden der Gefriertrocknung, der Sprühtrocknung, der Sprühgranulation oder durch anderweitige Verfahren wie zum Beispiel in der zirkulierenden Wirbelschicht gemäß PCT/EP2004/006655 beschrieben, zu rieselfähigen Produkten insbesondere zu einem feinteiligen Pulver oder vorzugsweise grobkörnigem Granulat aufgearbeitet werden. Gegebenenfalls wird aus dem erhaltenen Granulat durch Sieben oder Staubabtrennung ein gewünschtes Produkt isoliert.

Es ist ebenfalls möglich, die Fermentationsbrühe direkt d. h. ohne vorherige Aufkonzentrierung durch Sprühtrocknung oder Sprühgranulation zu trocknen.

Unter "rieselfähig" versteht man Pulver, die aus einer Serie von Glasauslaufgefäßen mit verschieden großen Auslauföffnungen mindestens aus dem Gefäß mit der Öffnung 5 mm (Millimeter) ungehindert auslaufen (Klein: Seifen, Öle, Fette, Wachse 94, 12 (1968)).

Mit "feinteilig" ist ein Pulver mit überwiegendem Anteil (> 50 %) einer Korngröße von 20 bis 200 µm Durchmesser gemeint.

Mit "grobkörnig" ist ein Produkt mit einem überwiegendem Anteil (> 50 %) einer Korngröße von 200 bis 2000 µm Durchmesser gemeint.

Die Korngrößenbestimmung kann mit Methoden der Laserbeugungsspektrometrie durchgeführt werden. Die entsprechenden Methoden sind im Lehrbuch zur "Teilchengrößenmessung in der Laborpraxis" von R. H. Müller und R. Schuhmann, Wissenschaftliche Verlagsgesellschaft Stuttgart (1996) oder im Lehrbuch "Introduction to Particle Technology" von M. Rhodes, Verlag Wiley & Sons (1998) beschrieben.

Das rieselfähige, feinteilige Pulver kann wiederum durch geeignete Kompaktier- oder Granulier-Verfahren in ein grobkörniges, gut rieselfähiges, lagerfähiges und weitgehend staubfreies Produkt überführt werden.

Der Begriff "staubfrei" bedeutet, dass das Produkt lediglich geringe Anteile (< 5 %) an Korngrößen unter 100 µm Durchmesser enthält.

"Lagerfähig", im Sinne dieser Erfindung, bedeutet ein Produkt, das mindestens ein (1) Jahr oder länger, bevorzugt mindestens 1,5 Jahre oder länger, besonders bevorzugt zwei (2) Jahre oder länger in trockener und kühler Umgebung gelagert werden kann, ohne dass ein wesentlicher Verlust (maximal 5%) der jeweiligen Aminosäure auftritt.

Ein weiterer Gegenstand der Erfindung ist ein Verfahren, das in seinen Grundzügen in der DE 102006016158 beschrieben ist, und bei dem die unter Verwendung der erfindungsgemäßen Mikroorganismen erhaltene Fermentationsbrühe, aus der die Biomasse gegebenenfalls ganz oder teilweise abgetrennt wurde, weiterverarbeitet wird, indem man ein Verfahren durchführt das mindestens folgende Schritte umfasst:
a) den pH-Wert durch Zugabe von Schwefelsäure auf 4,0 bis 5,2, insbesondere 4,9 bis 5,1, absenkt, und ein molares Sulfat/L-Lysin-Verhältnis von 0,85 bis 1,2, bevorzugt 0,9 bis 1,0, besonders bevorzugt >0,9 bis <0,95, in der Brühe einstellt, gegebenenfalls durch Zugabe von einer weiteren oder mehreren Sulfat-haltigen Verbindung(n) und
b) das so erhaltene Gemisch durch Wasserentzug aufkonzentriert, und gegebenenfalls granuliert,
   wobei gegebenenfalls vor Schritt a) eine oder beide der folgenden Maßnahmen durchgeführt wird/werden:
c) Messung des molaren Verhältnisses von Sulfat/L-Lysin zur Ermittlung der benötigten Menge an Sulfat-haltigen Verbindung(n)
d) Zusatz einer Sulfat-haltigen Verbindung ausgewählt aus der Gruppe Ammoniumsulfat, Ammoniumhydrogensulfat und Schwefelsäure in entsprechenden Verhältnissen.

Gegebenenfalls wird weiterhin vor Schritt b) ein Salz der schwefligen Säure, bevorzugt Alkalihydrogensulfat, besonders bevorzugt Natriumhydrogensulfit in einer Konzentration von 0,01 bis 0,5 Gew.-%, bevorzugt 0,1 bis 0,3 Gew.%, besonders bevorzugt 0,1 bis 0,2 Gew.-% bezogen auf die Fermentationsbrühe hinzugesetzt.

Als bevorzugte Sulfat-haltigen Verbindungen im Sinne der oben genannten Verfahrensschritte sind insbesondere Ammoniumsulfat und/oder Ammoniumhydrogensulfat oder entsprechende Mischungen von Ammoniak und Schwefelsäure und Schwefelsäure selbst zu nennen.

Das molare Sulfat/L-Lysin-Verhältnis V wird nach der Formel: V = 2 x [SO₄²⁻] / [L-Lysin] berechnet. Diese Formel berücksichtigt die Tatsache, dass das SO₄²⁻Anion, bzw. die Schwefelsäure zweiwertig ist. Ein Verhältnis V = 1 bedeutet, dass ein stöchiometrisch zusammengesetztes Lys₂-H₂SO₄) vorliegt, während bei einem Verhältnis von V = 0,9 ein 10%iger Sulfatmangel und bei einem Verhältnis von V = 1,1 ein 10% Sulfatüberschuss gefunden wird.

Vorteilhaft bei der Granulation oder Kompaktierung ist der Einsatz von üblichen organischen oder anorganischen Hilfsstoffen, beziehungsweise Trägern wie Stärke, Gelatine, Cellulosederivaten oder ähnlichen Stoffen, wie sie üblicherweise in der Lebensmittel- oder Futterverarbeitung als Binde-, Gelier-, oder Verdickungsmittel Verwendung finden, oder von weiteren Stoffen wie zum Beispiel Kieselsäuren, Silikaten (EP0743016A) Stearaten.

Weiterhin ist es vorteilhaft die Oberfläche der erhaltenen Granulate mit Ölen zu behandeln so wie es in der WO 04/054381 beschrieben ist. Als Öle können Mineralöle, pflanzliche Öle oder Mischungen pflanzlicher Öle verwendet werden. Beispiele für derartige Öle sind Sojaöl, Olivenöl, Sojaöl/Lecithingemische. In gleicher Weise sind auch Silikonöle, Polyethylenglykole oder Hydroxyethylcellulose geeignet. Durch die Behandlung der Oberflächen mit den genannten Ölen erzielt man eine erhöhte Abriebfestigkeit des Produktes und einen Verringerung des Staubanteils. Der Gehalt an Öl im Produkt beträgt 0,02 bis 2,0 Gew.-%, bevorzugt 0,02 bis 1,0 Gew.-%, und ganz besonders bevorzugt 0,2 bis 1,0 Gew.-% bezogen auf die Gesamtmenge des Futtermitteladditivs.

Bevorzugt sind Produkte mit einem Anteil von ≥ 97 Gew.-% einer Korngröße von 100 bis 1800 µm oder einem Anteil von ≥ 95 Gew.-% einer Korngröße von 300 bis 1800 µm Durchmesser. Der Anteil an Staub d. h. Partikeln mit einer Korngröße < 100 µm liegt bevorzugt bei > 0 bis 1 Gew.- besonders bevorzugt bei maximal 0,5 Gew.-%.

Alternativ kann das Produkt aber auch auf einen in der Futtermittelverarbeitung bekannten und üblichen organischen oder anorganischen Trägerstoff wie zum Beispiel Kieselsäuren, Silikate, Schrote, Kleien, Mehle, Stärken, Zucker oder andere aufgezogen und/oder mit üblichen Verdickungs- oder Bindemitteln vermischt und stabilisiert werden. Anwendungsbeispiele und Verfahren hierzu sind in der Literatur (Die Mühle + Mischfuttertechnik 132 (1995) 49, Seite 817) beschrieben.

Schließlich kann das Produkt auch durch Beschichtungsverfahren ("Coating") mit Filmbildnern wie beispielsweise Metallcarbonate, Kieselsäuren, Silikate, Alginate, Stearate, Stärken, Gummis und Celluloseether, wie in der DE-C-4100920 beschrieben, in einen Zustand gebracht werden, in dem es stabil gegenüber der Verdauung durch Tiermägen insbesondere dem Magen von Wiederkäuern ist.

Zur Einstellung einer gewünschten L-Lysin-Konzentration im Produkt kann je nach Anforderung das L-Lysin während des Verfahrens in Form eines Konzentrates oder gegebenenfalls einer weitgehend reinen Substanz beziehungsweise dessen Salz in flüssiger oder fester Form hinzugefügt werden. Diese können einzeln oder als Mischungen zur erhaltenen oder aufkonzentrierten Fermentationsbrühe, oder auch während des Trocknungs- oder Granulationsprozesses hinzugefügt werden.

Ein weiterer Gegenstand der Erfindung ist ein Verfahren zur Herstellung eines festen Lysin-haltigen Produktes, wie es in seinen Grundzügen in der US 20050220933 beschrieben ist, und das die Aufarbeitung der unter Verwendung der erfindungsgemäßen Mikroorganismen erhaltenen Fermentationsbrühe in folgenden Schritten umfasst:
a) Filtration der Fermentationsbrühe, bevorzugt mit einem Membranfilter, so dass ein Biomasse-haltiger Schlamm und ein Filtrat erhalten wird,
b) Aufkonzentration des Filtrates, bevorzugt so, dass ein Feststoffgehalt von 48 bis 52 Gew.-% erhalten wird,
c) Granulation des in Schritt b) erhaltenen Konzentrates, bevorzugt bei einer Temperatur von 50°C bis 62°C, und
d) Beschichtung des in c) erhaltenen Granulates mit einem oder mehreren der Beschichtungsmittel (coating agent(s))

Zur Beschichtung in Schritt d) werden bevorzugt Beschichtungsmittel verwendet, welche ausgewählt werden aus der Gruppe, bestehend aus
d1) der in Schritt a) erhaltenen Biomasse,
d2) einer L-Lysin-haltigen Verbindung, bevorzugt ausgewählt aus der Gruppe L-Lysinhydrochlorid oder L-Lysinsulfat,
d3) einem im wesentlichen L-Lysin-freien Stoff mit L-Lysingehalt < 1 Gew.-%, bevorzugt < 0,5 Gew.-%, bevorzugt ausgewählt aus der Gruppe Stärke, Karageenan, Agar, Kieselsäuren, Silikate, Schrote, Kleien und Mehle, und
d4) einem wasserabstoßenden Stoff, bevorzugt ausgewählt aus der Gruppe Öle, Polyethylenglykole und flüssige Paraffine.

Durch die Massnahmen entsprechend den Schritten d1) bis d4), insbesondere d1) bis d3), wird der Gehalt an L-Lysin auf einen gewünschten Wert eingestellt.

Bei der Herstellung L-Lysin-haltiger Produkte wird das Verhältnis der Ionen bevorzugt so eingestellt, dass das molare Ionenverhältnis entsprechend nachstehender Formel

2x[SO₄²⁻]+[Cl-]-[NH₄⁺]-[Na⁺]-[K⁺]-2x[Mg²⁺]-2x[Ca²⁺]/[L-Lys]

0,68 bis 0,95, bevorzugt 0,68 bis 0,90, besonders bevorzugt 0,68 bis 0,86 ergibt, so wie von Kushiki et al. in der US 20030152633 beschrieben.

Im Falle des L-Lysins hat das auf diese Weise hergestellte feste Produkt auf Fermentationsbrühebasis einen Lysingehalt (als Lysinbase) von 10 Gew.-% bis 70 Gew.-% oder 20 Gew.-% bis 70 Gew.-%, bevorzugt 30 Gew.-% bis 70 Gew.-% und ganz besonders bevorzugt von 40 Gew.-% bis 70 Gew.-% bezogen auf die Trockenmasse des Produkts. Maximale Gehalte an Lysinbase von 71 Gew.-% , 72 Gew.-%, 73 Gew.-% sind ebenfalls möglich.

Der Wassergehalt des L-Lysin-haltigen, festen Produktes beträgt bis zu 5 Gew.-%, bevorzugt bis zu 4 Gew.-%, und besonders bevorzugt weniger als 3 Gew.-%.

### Ausführungsbeispiele

### Beispiel 1: AEC-Screening

Zehn Einzelklone des wildtypischen Stammes C. *humireducens* (DSM 45392) wurden in jeweils 10 ml BHI-Flüssigmedium (Brain-Heart-Infusion; Merck) (37 g/l H₂O) in Schüttelkolben über Nacht bei 37°C mit 200 rpm kultiviert. Anschließend wurden jeweils 100 µl der Übernachtkultur auf Minimalmedium-Agarplatten mit 25 mM S-2-Aminoethyl-L-Cystein (AEC) (MW=164 g/mol) ausplattiert und drei Tage lang bei 37°C inkubiert. Zur Herstellung des Minimalmediums wurden 5 g (NH₄)₂SO₄, 5 g Harnstoff, 2 g KH₂PO₄, 2 g K₂HPO₄, 10 g MOPS in 750 ml H₂O gelöst, der pH-Wert wurde mit 1 M KOH auf 7,6 eingestellt und autoklaviert. Die restlichen Bestandteile wurden getrennt angesetzt und steril filtriert. Dabei wurden 20 ml 50% (w/v) Glukose, 1 ml 1% (w/v) CaCl₂, 1 ml 1 M MgSO₄, 1 ml 0,02% Biotin und 1 ml Spurenelementlösung (1 g FeSO₄ x 7 H₂O, 1 g MnSO₄ 7 x H₂O, 0,1 g ZnSO₄ x 7 H₂O, 0,021 g CuSO₄ x 5 H₂O, 0,002 g NiCl₂ x 6 H₂O auf 100 ml H₂O) dem Medium hinzugefügt und anschließend mit sterilem H₂O auf 1000 ml aufgefüllt. Für die Kultivierung auf Festmediumplatten wurde dem Medium 15 g/l Agar-Agar (Merck) zugesetzt.

Sichtbare Einzelkolonien wurden erneut auf frische Minimalmedium-Agarplatten mit 25 mM AEC und 12,5 mM Threonin als fraktionierter Ausstrich ausplattiert und drei Tage bei 37°C inkubiert. Daraufhin wurden jeweils 10 ml BHI-Flüssigmedium (Brain-Heart-Infusion; Merck) (37 g/l H₂O) im Schüttelkolben mit einem Einzelklon inokuliert und über Nacht bei 37°C mit 200 rpm schüttelnd inkubiert, anschließend mit 10% Glycerin versetzt und bei -80°C als Rückstellmuster gelagert.

### Sequenzanalyse des lysC-Sequenz Bereiches

Zur Analyse der lysC-Sequenz Bereiche der isolierten Einzelklone, wurde der entsprechende Genbereich von *lysC* mittels Primern (lysC_for: 5'AGACGAAAGGCGGCCTACAC3' und lysC_rev: 5'TCCAGGATCGAGCGCATCAC3') und PCR-Technik amplifiziert. Die erhaltenen DNA-Sequenzen wurden mit Hilfe der Software Clone Manager analysiert. Durch die Analyse der lysC-Sequenz der isolierten AEC + Threonin resistenten C. *humireducens* Klone wurden folgende Punktmutationen identifiziert:

**Tab. 1: Identifizierte Veränderungen im lysC-Gen von AEC + Threonin resistenten C. humireducens Klonen und dadurch verursachte Aminosäureaustausche.**

| ***C. humireducens* Klone** | **Punktmutation** | **AS-Austausch** |
|---|---|---|
| | | |
| C. humireducens_AEC_Thr_r#1 | C → T | T308I |
| C. humireducens_AEC_Thr_r#2 | G → A | G359D |
| C. humireducens_AEC_Thr_r#3 | C → T | T311I |
| C. humireducens_AEC_Thr_r#4 | C → T | T311I |
| C. humireducens_AEC_Thr_r#5 | G → T | D274Y |
| C. humireducens_AEC_Thr_r#6 | C → T | T308I |
| C. humireducens_AEC_Thr_r#7 | C → A | S301Y |
| C. humireducens_AEC_Thr_r#9 | C → T | T308I |
| C. humireducens_AEC_Thr_r#10 | C → A | A279E |

### Beispiel 2: L-Lysin-Leistungstest

Der Typstamm *C*. *humireducens* und die isolierten Einzelklone aus dem AEC + Threonin Screening wurden in Schüttelkolben kultiviert und einem Leistungstest bezüglich ihrer Lysinsynthese im Schüttelkolbenmaßstab unterzogen. Hierzu wurde der *C*. *humireducens* Stamm und die isolierten AEC + Threonin resistenten *C*. *humireducens* Klone in 10 ml BHI-Flüssigmedium (Brain-Heart-Infusion; Merck) (37 g/l H₂O) als Vorkultur bei 37°C mit 200 rpm 24 h lang inkubiert. Anschließend wurden 10 ml Schüttelkolbenmedium auf eine OD₆₆₀ von 0,2 inokuliert und bei 37°C mit 200 rpm, 48 h lang kultiviert. Zur Herstellung dieses Mediums wurden 7,5 g Cornsteep Liquor (50%), 20 g Morpholinopropansulfonsäure (MOPS), 25 g (NH₄)₂SO₄, 0,1 g KH₂PO₄, 1 g MgSO₄*7H₂O, 0,01 g CaCl₂*2H₂O, 0,01 g FeSO₄*7H₂O, 0,005 g MnSO₄*H₂O in 750 ml H₂O gelöst, der pH-Wert wurde mit Ammoniakwasser auf 7,0 eingestellt und autoklaviert. Anschließend wurden 25 g trocken autoklaviertes CaCO₃ hinzugefügt. Die restlichen Bestandteile wurden getrennt angesetzt und steril filtriert. Dabei wurden 90 ml 50% (w/v) Glukose und 10 ml einer Lösung von 30 mg/l Thiamin und 20 mg/l Biotin dem Medium hinzugefügt und anschließend mit sterilem H₂O auf 1000 ml aufgefüllt.

Nach der Kultivierung wurde jeweils vom Überstand zwei paralleler Kulturen eine HPLC-Analyse zur Bestimmung der L-Lysingehalte mit einer Nachweisgrenze von ≥0,01 g/l durchgeführt. Die Lysin-Endtiter und Ausbeuten der Kultivierungen sind in der nachfolgenden Tabelle dargestellt.

**Tab. 2: Mittelwerte und Standardabweichung der Lysin-Endtiter von zwei parallelen Kulturen mit AEC + Threonin resistenten C. humireducens Klonen nach 48 h Kultivierung in Schüttelkolbenmedium bei 37°C, 200 rpm.**

| | Lysin (g/l) | |
|---|---|---|
| | Mittelwert | Stabw. |
| Typstamm C. humireducens | 0,13 | 0,01 |
| C. humireducens_AEC_Thr_r#1 | 1,33 | 0,08 |
| C. humireducens_AEC_Thr_r#2 | 1,33 | 0,08 |
| C. humireducens_AEC_Thr_r#3 | 1,25 | 0,01 |
| C. humireducens_AEC_Thr_r#4 | 1,29 | 0,01 |
| C. humireducens_AEC_Thr_r#5 | 1,24 | 0,09 |
| C. humireducens_AEC_Thr_r#6 | 0,85 | 0,04 |
| C. humireducens_AEC_Thr_r#7 | 1,12 | 0,00 |
| C. humireducens_AEC_Thr_r#9 | 1,18 | 0,02 |
| C. humireducens_AEC_Thr_r#10 | 1,34 | 0,03 |

## Patentansprüche

1. Alkaliphiles Bakterium, **dadurch gekennzeichnet, dass** es L-Lysin überproduziert.

2. Alkaliphiles Bakterium gemäß Anspruch 1, **dadurch gekennzeichnet, dass** es sich um ein alkaliphiles Corynebacterium, vorzugsweise um einen Corynebacterium humireducens-Stamm, handelt.

3. Alkaliphiles Bakterium nach Anspruch 1 oder 2, **dadurch gekennzeichnet, dass** es resistent gegenüber AEC in einer Konzentration von 5 bis 100 mM, vorzugsweise 10 bis 50 mM, insbesondere 20 bis 30 mM, ist.

4. Alkaliphiles Bakterium nach einem der vorhergehenden Ansprüche, **dadurch gekennzeichnet, dass** es resistent gegenüber L-Threonin in einer Konzentration von 2 bis 50 mM, vorzugsweise 5 bis 30 mM, insbesondere 8 bis 15 mM, ist.

5. Alkaliphiles Bakterium nach einem der vorhergehenden Ansprüche, **dadurch gekennzeichnet, dass** es ein feedback-resistentes lysC-Enzym enthält.

6. Alkaliphiles Bakterium gemäß vorhergehendem Anspruch, **dadurch gekennzeichnet, dass** das feedback-resistente lysC-Enzym eine Sequenzidentität von mindestens 92 %, vorzugsweise mindestens 95 %, zu der Polypeptidsequenz gemäß SEQ ID NO: 12 aufweist.

7. Alkaliphiles Bakterium gemäß Anspruch 6, **dadurch gekennzeichnet, dass** das feedback-resistente lysC-Enzym ausgewählt ist aus:
a) Polypeptiden mit einer Sequenzidentität von mindestens 80 oder 90 % zu der Polypeptidsequenz gemäß SEQ ID NO: 12, **dadurch gekennzeichnet, dass** sie mindestens eine der folgenden Mutationen aufweist: D274Y, A279E, G359D;
b) Polypeptiden mit einer Sequenzidentität von mindestens 92 oder 95 % zu der Polypeptidsequenz gemäß SEQ ID NO: 12, **dadurch gekennzeichnet, dass** sie mindestens eine der folgenden Mutationen aufweist: S301Y, T308I, T311I.

8. Aspartat-Kinase mit einer Sequenzidentität von mindesens 92 % zu der Polypeptidsequenz gemäß SEQ ID NO: 12.

9. Feedback-resistente Aspartat-Kinasen ausgewählt aus der Gruppe bestehend aus:
a) Polypeptiden mit einer Sequenzidentität von mindestens 80 oder 90 % zu der Polypeptidsequenz gemäß SEQ ID NO: 12, **dadurch gekennzeichnet, dass** sie mindestens eine der folgenden Mutationen aufweist: D274Y, A279E, G359D;
b) Polypeptiden mit einer Sequenzidentität von mindestens 92 oder 95 % zu der Polypeptidsequenz gemäß SEQ ID NO: 12, **dadurch gekennzeichnet, dass** sie mindestens eine der folgenden Mutationen aufweist: S301Y, T308I, T311I.

10. Feedback-resistente Aspartat-Kinasen gemäß Anspruch 9, **dadurch gekennzeichnet, dass** es sich hierbei um Polypeptide mit einer Sequenz gemäß SEQ ID NO: 12 handelt mit der Maßgabe, dass mindestens eine, vorzugsweise genau eine, der folgenden Mutationen vorliegt: D274Y, A279E, S301Y, T308I, T311I, G359D.

11. Polynukleotide ausgewählt aus der Gruppe bestehend aus
a) Polynukleotide, die für Aspartat-Kinasen nach einem der Ansprüche 8 bis 10 kodieren,
b) Polynukleotide, die eine Sequenzidentität von mindestens 70 %, vorzugsweise mindestens 80 oder 85 %, insbesondere mindestens 90 oder 95 %, zu der Sequenz von Position 301 bis 1566 gemäß SEQ ID NO: 11 aufweisen.

12. Mikroorganismus, **dadurch gekennzeichnet, dass** er eine Aspartat-Kinase gemäß Anspruch 9 oder 10 und/oder ein Polynukleotid gemäß Anspruch 11, vorzugsweise in überexprimierter Form, enthält.

13. Mikroorganismus nach einem der Ansprüche 1 bis 8 oder 12, **dadurch gekennzeichnet, dass** es mindestens eines, vorzugsweise mindestens zwei oder drei, der folgenden Merkmale aufweist:
a) ein überexprimiertes Polynukleotid (dapA-Gen), das für eine Dihydrodipicolinat-Synthase (DapA, EC 4.2.1.52), vorzugsweise für eine Dihydrodipicolinat-Synthase mit einer Sequenzidentität von mindestens 90, 95 oder 98 %, vorzugsweise 100 %, zu der Sequenz gemäß SEQ ID NO: 2, kodiert,
b) ein überexprimiertes Polynukleotid (ddh), das für eine Diaminopimelat-Dehydrogenase (Ddh, EC 1.4.1.16), vorzugsweise für eine Diaminopimelat-Dehydrogenase mit einer Sequenzidentität von mindestens 90, 95 oder 98 %, vorzugsweise 100 %, zu der Sequenz gemäß SEQ ID NO: 36 kodiert,
c) ein überexprimiertes Polynukleotid (asd-Gen), das für eine Aspartat-Semialdehyd-Dehydrogenase (Asd, EC 1.2.1.11), vorzugsweise für eine Aspartat-Semialdehyd-Dehydrogenase mit einer Sequenzidentität von mindestens 90, 95 oder 98 %, vorzugsweise 100 %, zu der Sequenz gemäß SEQ ID NO: 30, kodiert,
d) ein überexprimiertes Polynukleotid (lysA-Gen), das für eine Diaminopimelat-Decarboxylase (LysA, EC 4.1.1.20), vorzugsweise für eine Diaminopimelat-Decarboxylase mit einer Sequenzidentität von mindestens 90, 95 oder 98 %, vorzugsweise 100 %, zu der Sequenz gemäß SEQ ID NO: 4, kodiert,
e) ein überexprimiertes Polynukleotid (aat-Gen), das für eine Aspartat-Aminotransferase (AaT, EC 2.6.1.1), vorzugsweise für eine Aspartat-Aminotransferase mit einer Sequenzidentität von mindestens 90, 95 oder 98 %, vorzugsweise 100 %, zu der Sequenz gemäß SEQ ID NO: 6, kodiert,
f) ein überexprimiertes Polynukleotid (lysE-Gen), das für einen L-Lysin-Exporter (LysE, Lysin-Efflux-Permease), vorzugsweise für einen L-Lysin-Exporter mit einer Sequenzidentität von mindestens 90, 95 oder 98 %, vorzugsweise 100 %, zu der Sequenz gemäß SEQ ID NO: 8, kodiert,
g) ein überexprimiertes Polynukleotid (pyc-Gen), das für eine Pyruvat-Carboxylase (Pyc, EC 6.4.1.1), vorzugsweise für eine Pyruvat-Carboxylase mit einer Sequenzidentität von mindestens 90, 95 oder 98 %, vorzugsweise 100 %, zu der Sequenz gemäß SEQ ID NO: 10, kodiert,
h) ein überexprimiertes Polynukleotid (dapF-Gen), das für eine Diaminopimelat-Epimerase (DapF, EC 5.1.1.7) kodiert,
i) ein überexprimiertes Polynukleotid (dapB-Gen), das für eine Dihydropicolinat-Reduktase (DapB, EC 1.3.1.26), vorzugsweise für eine Dihydropicolinat-Reduktase mit einer Sequenzidentität von mindestens 90, 95 oder 98 %, vorzugsweise 100 %, zu der Sequenz gemäß SEQ ID NO: 14, kodiert,
j) ein überexprimiertes Polynukleotid, das für eine Glucose-6-phosphat-Dehydrogenase (EC 1.1.1.49), vorzugsweise für eine Glucose-6-phosphat-Dehydrogenase mit einer Sequenzidentität von mindestens 90, 95 oder 98 %, vorzugsweise 100 %, zu der Sequenz gemäß SEQ ID NO: 16, kodiert,
k) ein überexprimiertes Polynukleotid (zwf-Gen), das für die Zwf-Untereinheit einer Glucose-6-phosphat-Dehydrogenase (Zwf, EC 1.1.1.49), vorzugsweise für eine Zwf-Untereinheit mit einer Sequenzidentität von mindestens 90, 95 oder 98 %, vorzugsweise 100 %, zu der Sequenz gemäß SEQ ID NO: 32, kodiert,
l) ein überexprimiertes Polynukleotid (opcA-Gen), das für die OpcA-Untereinheit einer Glucose-6-phosphat-Dehydrogenase (OpcA, EC 1.1.1.49), vorzugsweise für eine OpcA-Untereinheit mit einer Sequenzidentität von mindestens 90, 95 oder 98 %, vorzugsweise 100 %, zu der Sequenz gemäß SEQ ID NO: 34, kodiert,
m) ein überexprimiertes Polynukleotid (gnd-Gen), das für eine Phosphogluconsäure-Dehydrogenase (Gnd, EC 1.1.1.44), vorzugsweise für eine Phosphogluconsäure-Dehydrogenase mit einer Sequenzidentität von mindestens 90, 95 oder 98 %, vorzugsweise 100 %, zu der Sequenz gemäß SEQ ID NO: 18, kodiert,
n) ein ausgeschaltetes oder abgeschwächtes Polynukleotid (mqo), das für eine Malat-Chinon-Oxidoreduktase (Mqo, EC 1.1.99.16), vorzugsweise für eine Malat-Chinon-Oxidoreduktase mit einer Sequenzidentität von mindestens 90, 95 oder 98 %, vorzugsweise 100 %, zu der Sequenz gemäß SEQ ID NO: 20, kodiert,
o) ein ausgeschaltetes oder abgeschwächtes Polynukleotid, das für die E1 p-Untereinheit eines Pyruvat-Dehydrogenase-Komplexes (AceE, EC 1.2.4.1), vorzugsweise für eine E1 p-Untereinheit mit einer Sequenzidentität von mindestens 90, 95 oder 98 %, vorzugsweise 100 %, zu der Sequenz gemäß SEQ ID NO: 22, kodiert,
p) ein ausgeschaltetes oder abgeschwächtes Polynukleotid (gltA), das für eine Citrat-Synthase (GltA, EC 4.1.3.7), vorzugsweise für eine Citrat-Synthase mit einer Sequenzidentität von mindestens 90, 95 oder 98 %, vorzugsweise 100 %, zu der Sequenz gemäß SEQ ID NO: 24, kodiert,
q) ein ausgeschaltetes oder abgeschwächtes Polynukleotid (mdh), das für eine Malat-Dehydrogenase (Mdh, EC 1.1.1.37), vorzugsweise für eine Malat-Dehydrogenase mit einer Sequenzidentität von mindestens 90, 95 oder 98 %, vorzugsweise 100 %, zu der Sequenz gemäß SEQ ID NO: 26, kodiert,
r) ein ausgeschaltetes oder abgeschwächtes Polynukleotid (murE), das für eine UDP-N-acetylmuramoylalanyl-D-glutamat-2,6-diaminopimelat-Ligase, 6-Diaminopimelat-Ligase (MurE, EC 6.3.2.13), vorzugsweise für ein Enzym mit einer Sequenzidentität von mindestens 90, 95 oder 98 %, vorzugsweise 100 %, zu der Sequenz gemäß SEQ ID NO: 28, kodiert.

14. Verfahren zur Überproduktion von L-Lysin in Mikroorganismen, **dadurch gekennzeichnet, dass** in dem Verfahren eine Aspartat-Kinase nach einem der Ansprüche 8 bis 10 und/oder ein Polynukleotid gemäß Anspruch 11 und/oder ein alkaliphiles Bakterium nach einem der Ansprüche 1 bis 7 und/oder ein Mikroorganismus nach Anspruch 12 oder 13 zum Einsatz kommt.

15. Verfahren nach Anspruch 14, **dadurch gekennzeichnet, dass** die Aspartat-Kinase und/oder das Polynukleotid in überexprimierter Form eingesetzt werden.
